(19) **Europäisches Patentamt / European Patent Office / Office européen des brevets**

(11) **EP 3 110 961 B1**

(12) # EUROPEAN PATENT SPECIFICATION

(45) Date of publication and mention of the grant of the patent:
**27.11.2019 Bulletin 2019/48**

(21) Application number: **15709430.1**

(22) Date of filing: **24.02.2015**

(51) Int Cl.:
*C12P 21/00* (2006.01)       *C12P 21/08* (2006.01)
*C12N 1/00* (2006.01)        *C12N 1/38* (2006.01)
*C12N 5/071* (2010.01)       *C07K 16/00* (2006.01)

(86) International application number:
**PCT/EP2015/053804**

(87) International publication number:
**WO 2015/128314 (03.09.2015 Gazette 2015/35)**

(54) **MODULATION OF CELL GROWTH AND GLYCOSYLATION IN RECOMBINANT GLYCOPROTEIN PRODUCTION**

MODULATION DES ZELLWACHSTUMS UND GLYKOLYSIERUNG IN DER REKOMBINANTEN GLYCOPROTEINHERSTELLUNG

MODULATION DE LA CROISSANCE CELLULAIRE ET DE LA GLYCOSYLATION DANS LA PRODUCTION DE GLYCOPROTÉINES RECOMBINANTES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.02.2014 EP 14157030**

(43) Date of publication of application:
**04.01.2017 Bulletin 2017/01**

(73) Proprietor: **F. Hoffmann-La Roche AG**
**4070 Basel (CH)**

(72) Inventors:
• **POPP, Oliver**
  **82377 Penzberg (DE)**
• **BEAUCAMP, Nicola**
  **80639 Muenchen (DE)**
• **DRABNER, Georg**
  **81669 Muenchen (DE)**
• **ESSLINGER, Stephanie**
  **81377 Muenchen (DE)**

(74) Representative: **Mewburn Ellis LLP**
**City Tower**
**40 Basinghall Street**
**London EC2V 5DE (GB)**

(56) References cited:
EP-A2- 1 096 017        WO-A1-99/61650
WO-A1-2008/008360       WO-A1-2008/109410
WO-A1-2011/019622       WO-A1-2011/095596
WO-A1-2013/114245       US-A1- 2007 161 084

• KIM S-G ET AL: "Coexpression of folding accessory proteins for production of active cyclodextrin glycosyltransferase of Bacillus macerans in recombinant Escherichia coli", PROTEIN EXPRESSION AND PURIFICATION, ACADEMIC PRESS, SAN DIEGO, CA, vol. 41, no. 2, 1 June 2005 (2005-06-01), pages 426-432, XP004875134, ISSN: 1046-5928, DOI: 10.1016/J.PEP.2005.01.017
• BELCARZ<A> A ET AL: "The novel non-glycosylated invertase from Candida utilis (the properties and the conditions of production and purification)", BIOCHIMICA ET BIOPHYSICA ACTA. PROTEIN STRUCTURE AND MOLECULAR ENZYMOLOGY, ELSEVIER, AMSTERDAM; NL, vol. 1594, no. 1, 31 January 2002 (2002-01-31), pages 40-53, XP004334740, ISSN: 0167-4838, DOI: 10.1016/S0167-4838(01)00279-5
• SIMMONS L C ET AL: "Expression of full-length immunoglobulins in Escherichia coli: rapid and efficient production of aglycosylated antibodies", JOURNAL OF IMMUNOLOGICAL METHODS, ELSEVIER SCIENCE PUBLISHERS B.V.,AMSTERDAM, NL, vol. 263, no. 1-2, 1 May 2002 (2002-05-01), pages 133-147, XP004354391, ISSN: 0022-1759, DOI: 10.1016/S0022-1759(02)00036-4

**(Cont. next page)**

• SAUER P W ET AL: "A high-yielding, generic fed-batch cell culture process for production of recombinant antibodies", BIOTECHNOLOGY AND BIOENGINEERING, WILEY, US, vol. 67, no. 5, 5 March 2000 (2000-03-05), pages 585-597, XP002690472, ISSN: 0006-3592, DOI: 10.1002/(SICI)1097-0290(20000305)67:5<585: :AID-BIT9>3.0.CO;2-H [retrieved on 2000-03-26]

**Description**

The present application relates to the role that trace elements play in cell growth and protein glycosylation in the production by fermentation of recombinant glycoproteins.

BACKGROUND

Recombinant glycoproteins are typically produced by a fermentative production process using eukaryotic expression systems. Post-translational glycosylation of proteins is essential to fulfil important physicochemical and biological properties and functions, such as protein solubility, stability, clearance, immunogenicity and immune effector functions. The glycosylation status of a glycoprotein is tightly regulated and even small differences in glycosylation can have significant effects. However, due to the large number of enzymes involved, particularly in N-glycosylation, the pathway is the most complex post-translational modification performed in eukaryotic cells. In glycoproteins, sugars are attached either to the amide nitrogen atom in the side chain of asparagine (an N-linkage) or to the oxygen atom in the side chain of serine or threonine (an O-linkage). Glycosylation starts with formation of the N-linkages in the endoplasmic reticulum, so-called "core glycosylation". After this the polypeptides are transported to the Golgi apparatus where the O-linked sugars are added and the N-linked sugar chains are modified in many different ways, for example by the removal of mannose residues, addition of N-acetylglucosamine, galactose and/or fucose and/or sialic acid residues.

Metals and their ions play essential roles in biology (Yannone et al., Current Opinion in Biotechnology 23.1 (2013) 89-95). It has been reported that, out of 39 essential biological elements, 26 are metals. Furthermore, there is significant variation in the active concentrations of these metals required to fulfill their biological function. The essential metals can be classified into macronutrients, like calcium, potassium or magnesium, and micronutrients like the trace metals iron, zinc, manganese or copper, and ultra-trace elements, like vanadium and tungsten. Although these essential metals are critical for biological homeostasis, they can be toxic in concentrations beyond those necessary for their biological function. Thus, tight regulation of systemic metal concentration is vital for biological homeostasis.

The transition metals copper, iron, zinc, and manganese are classified according to their biological active concentration as trace elements and are key players in cellular homeostasis. They are found in active sites of many enzymes as described in Fraga and Fraga (Mol. Aspects Med. 26.4-5 (2005) 235-44).

For many years now, the role of trace elements in cellular growth has been studied using animal feeding and cell culture experiments. That trace elements are needed for successful cell cultivation has been shown by Ham et al (Proc. Natl. Acad. Sci. USA 53 (1965) 288-93). There is also much interest in the role of trace elements in protein glycosylation, but the detailed enzymatic contributions of distinct trace elements and the interactions of metal cations in the glycosylation pathway remain relatively unknown.

In general, the activity of glycosylation enzymes depends on the availability of divalent trace elements (such as $Zn^{2+}$, $Mn^{2+}$, $Ca^{2+}$, $Mg^{2+}$), the pH milieu and the availability of nucleotide phosphates (UTP, GTP, CTP) and corresponding sugar derivatives (UDP-Gal, UDP-GlcNAc, GDP-Fuc, CMP-sialic acid).

Kaminska et al., (Glyconj. J. 15.8 (1998) 783-88) studied the role of divalent cations on the activity of glycoprotein 6-$\alpha$-L-fucosyltransferase and discovered that, whilst divalent cations such as $Mg^{2+}$ and $Ca^{2+}$ activated this enzyme, $Cu^{2+}$, $Zn^{2+}$ and $Ni^{2+}$ strongly inhibited the activity of this enzyme. Witsell et al., (J. Biol. Chem. 265.26 (1990) 15731-37) describe the role of particular divalent cations in activation of galactosyltransferase in native mammary Golgi vesicles. Crowell et al., (Biotechnol. Bioeng. 96.3 (2007) 538-49) describe that trace elements, especially manganese, interact with other culture factors, like amino acid availability, in late process phases with stressed cultures to regulate protein glycosylation. Gawlitzek et al., (Biotechnol. Bioeng. 103.6 (2009) 1164-75) observe that cell culture conditions, such as manganese and iron concentrations, specific productivity enhancer butyrate, thyroid hormones and culture pH can control N-glycosylation site-occupancy of recombinant glycoproteins expressed in CHO cells.

As outlined in US 2007/0161084 a number of enzymes involved in glycosylation utilise different cations as co-factors. In that application the inventors discovered that sialylation of glycoproteins, i.e. the addition of a terminal sialic acid residue to a carbohydrate chain on a glycoprotein, and in particular sialylation of erythropoietin, could be improved by growing the mammalian host cells expressing the glycoprotein in a medium containing a non-toxic amount of manganese. The manganese can be present in the initial growth medium or may be added after a rapid cell growth phase or may be added after one or two harvest cycles.

The role of manganese and its active concentration in cell growth is controversial. It has been reported that manganese is essential for normal cell growth and development, but simultaneously is toxic at high concentrations (Au et al., Neurotoxicology 29.4 (2008) 569-76). For example, manganese has been shown to bind to cell surface located integrins and activate the MAP kinases, ERK1 and 2, both important switches for cellular growth (Roth et al., Neurotoxicology 23.2 (2002) 147-57). On the other hand, Roth et al., also show that manganese is implicated in cell death and apoptosis of cells by turning on many apoptotic factors, which finally lead to cell death by disruption of mitochondrial function and inducing loss of ATP. The toxic effect of manganese is quite strong when iron is removed from the cell culture medium, since iron and manganese compete for the same cell membrane shuttle DMT1 (Roth et al.).

US 2008/0081356 describes a method for large scale production of glycoproteins with altered glycosylation patterns in commercial scale cell culture by using a medium containing a molar cumulative concentration of manganese between 10 and 600 nM and a molar cumulative glutamine concentration of less than about 8 mM.

WO 2012/149197 describes methods for modulating the galactosylation profile of recombinantly expressed proteins, and in particular antibodies, in CHO and NSO cell lines by supplementing the production media with manganese and/or galactose.

The importance of $Mn^{2+}$ as cofactor for $\beta$-1,4-galactosyl-transferase activity has been described previously by Ramakrishnan et al., (J. Mol. Biol. 357.5 (2006) 1619-33).

Zinc has been reported to be essential for cell proliferation and differentiation, especially for regulation of DNA synthesis and mitosis. Zinc is a structural constituent of a great number of proteins, including transcription factors and enzymes from cellular signalling pathways, such as second messenger metabolism, protein kinase and proteins phosphatase activities (Beyersmann et al., Biometals 14.3-4 (2001) 331-41). Accordingly, bioavailable zinc has been associated with cell proliferation.

Iron is a key player in cell growth regulation. It has been reported previously that supplementation of iron, or its transport protein transferrin, to a chemically defined cell culture medium is vital for biomass generation. For example, iron has been shown to promote cell growth of C6 glioma and L1210 leukemic cancer cell lines in serum-free medium (Basset et al., Carcinogenesis 6.3 (1985) 355-59). The fundamental role of iron in cell growth is further demonstrated by chelators that can cross the plasma membrane, limiting its bioavailability by binding the metal inside the cell. Agents such as desferrioxamine and desferrithiocin inhibit the growth of a variety of tumour cells in culture (Reddel et al., Exp.Cell Res. 161.2 (1985) 277-84 and Basset et al., supra) and greatly reduce T-cell proliferation (Polson et al., Immunology 71.2 (1990) 176-81 and Pattanapanyasat et al., Br. J. Haematol. 82.1 (1992) 13-19). Here, the possible cell growth inhibitory mechanism is iron deprivation, associated with reduced ribonucleotide reductase activity and reduced amount of deoxyribonucleotides. This subsequently leads to mitotic arrest in S-phase. The addition of iron to the medium reverses the growth inhibition (Lederman et al., Blood 64.3 (1984) 748-53).

WO 98/08934 describes the use of a medium supplemented with zinc and iron (amongst other components) in suspension culture to support high-density growth of mammalian cells.

Copper plays an essential role in cellular homeostasis, though the metabolic fates of copper and iron are closely linked (Arredondo et al., Molecular Aspects of Medicine 26.4 (2005) 313-27). Systemic copper deficiency generates cellular iron deficiency, which causes phenotypically similar effects like diminished cell growth. Furthermore, reducing bioavailability by chelating copper has been associated with a reduced capacity of growth factor-stimulated receptor phosphorylation and finally inhibition of cell proliferation pathways in cell culture studies (Turski et al., J. Biol. Chem. 284.4 (2009) 717-21). On the other hand, copper has been shown to stimulate blood vessel formation and copper privation by diet or copper chelators diminishes the ability of a tumour to support an angiogenic response (Harris et al., Nutr. Rev. 62.2 (2004) 60-64) and cancer growth (Gupte et al., Cancer Treat. Rev. 35.1 (2009) 32-46).

US 2009/0068705 describes methods for large scale production of proteins and/or polypeptides in cell culture where the culture medium comprises copper and/or glutamate and whereby the polypeptide has a more extensive or more desirable glycosylation pattern.

Although work in this area has focused on the activity of metal cofactors in stimulating glycoenzymes, various authors, such as Kaminska et al., supra, have also shown that the same or different metal co-factors can inhibit the activity of another glycoenzyme (e.g. $Ca^{2+}$ activates mannosyl-oligosaccharide 1,2-$\alpha$-mannosidase and simultaneously inhibits $\alpha$-1,3-mannosyl-glycoprotein 2-$\beta$-N-acetylglucosaminyl-transferase). Information on *in vitro* metal cofactors required for activation, or for structural arrangements of the protein, and metal inhibitors of a number of enzymes involved in glycosylation can be obtained from the BRENDA database (http://www.brenda-enzymes.org). This dual activity of various metal co-factors highlights the need for tight regulation of trace element usage in glycoprotein production.

Glycoengineering, i.e. changing protein-associated carbohydrate by recombinant co-expression of endogenous or heterologous glycoenzymes, has been shown to affect pharmacokinetic properties of proteins, such as molecular stability, solubility, *in* vivo biological activity and immunogenicity. In antibodies in particular, increasing the amount of galactosylation has been shown to affect antibody effector functions, such as the CDC (Raju, Curr. Opin. Immunol. 20.4 (2008) 471-8) or the antibody-dependent cellular toxicity (ADCC), for example galactosylation of human IgG monoclonal anti-D affects Fc receptor-mediated functional activity (Kumpel et al., Hum. Antib. Hyb. 5.3-4 (1994) 143-51); or an increase in the amount of galactosylation has been shown to enhance the blocking of complement-mediated inflammation by IgG immune complexes (Karsten et al., Nat. Med. 18.9 (2012) 1401-6). N-glycosylation of antibody Fc regions is essential for binding to the Fc receptors, which engage the antibody effector functions. Fucose is a core residue in the N-glycans of many antibodies, but non-fucosylated forms have been shown to have an enhanced ADCC over their fucosylated counterparts. Methods to increase production of non-fucosylated proteins and/or to influence fucosylation of antibodies are thus also desirable.

Accordingly, there remains a need in the art for methods to enhance cell culture strategies, including to improve cell growth and biomass generation and/or to direct glycosylation and hence maturation of glycoproteins.

SUMMARY OF THE INVENTION

The inventors have determined the role of bioactive trace elements in cell culture performance and in particular on cell growth and glycan formation in glycoproteins and hence in glycoprotein maturation. Accordingly, the present invention relates to methods for selecting between conditions which affect cell growth or biomass generation and conditions which affect N-glycan maturity in expressed glycoprotein produced by the cells. Thus, in the methods of the present invention, the glycoprotein producing cells are cultured in a medium which is tailored to a desired end result. The invention is defined by the claims.

Described is a method for production of a recombinant glycoprotein under fermentation culture conditions in a eukaryotic cell, the method comprising adjusting the concentrations of each of iron, copper, zinc and manganese in the culture medium during the culture to affect biomass generation and/or N-glycan maturity in the expressed glycoprotein.

The method encompasses adjusting concentrations of iron, copper, zinc and manganese to affect biomass generation and/or N-glycan maturity of the expressed glycoprotein. The adjustment of the concentrations of iron, copper, zinc and manganese will mean that the conditions in the cell will favour biomass generation or will have an effect on the maturity of the expressed glycoprotein. By "favour" as used herein is meant that the activity of the cell will be in the stated direction as compared to a cell grown under the same conditions but in which the concentrations of each of iron, copper, zinc and manganese have not been so adjusted. Thus, when biomass generation is favoured, the cell will be directed more towards multiplying than production of glycoprotein. Accordingly, there will be an enhancement or increase of biomass generation. Likewise, when the adjustment of the concentrations of iron, copper, zinc and manganese, or of zinc and manganese alone, affects N-glycan maturity of the expressed glycoprotein, the cell will be directed more towards production of such glycoproteins than towards e.g. biomass generation or production of other glycoprotein types. Thus the production of the desired glycoprotein type will be enhanced or increased.

The methods affect the N-glycan maturity of the expressed glycoprotein, for example to result in production of a glycoprotein with a desired maturity level. Thus, the methods affect the production of mature N-glycosylated glycoproteins and/or immature N-glycosylated glycoproteins, mature non-fucosylated glycoproteins and/or immature non-fucosylated glycoproteins. The methods include increasing the maturity of expressed N-glycosylated glycoproteins, increasing production of mature N-glycosylated glycoproteins, increasing production of mature non-fucosylated glycoproteins and/or increasing production of immature non-fucosylated glycoproteins. Concomitant with increasing production of mature N-glycosylated glycoproteins may be a decrease in production of mature and/or immature non-fucosylated glycoprotein species and immature N-glycosylated glycoproteins. Concomitant with increasing production of immature non-fucosylated glycoproteins may be a decrease in production of mature non-fucosylated glycoproteins and/or mature or immature N-glycosylated glycoproteins.

In certain cases, the concentrations of iron, copper, zinc and manganese may be adjusted in the culture medium to favour biomass generation, that is to enhance growth of the cells, and thereby increase biomass. In other cases, the concentrations of iron, copper, zinc and manganese may be adjusted in the culture medium to increase the maturity of the expressed glycoprotein, wherein the carbohydrate portion of the expressed glycoprotein has a G0, G1 or G2 structure. In one case, the concentrations of iron, copper, zinc and manganese, or of only zinc and manganese, may be adjusted in the culture medium to favour, i.e. increase production of, mature N-glycosylated glycoproteins. In another case, the concentrations of iron, copper, zinc and manganese may be adjusted to favour, i.e. increase the production of, immature non-fucosylated glycoproteins. In yet another case, the concentrations of iron, copper, zinc and manganese in the culture medium may be adjusted first to enhance growth and thereby increase biomass and then again to affect glycoprotein maturity, be that increasing maturity in expressed glycoproteins, increasing production of mature N-glycosylated glycoproteins or increasing production of mature or immature non-fucosylated glycoproteins.

Thus, the invention involves:

- favouring biomass generation by increasing the concentration of each of iron, copper, zinc and manganese in the culture medium;
- favouring increased maturity in expressed N-glycosylated glycoproteins by increasing the concentration of each of zinc and manganese and, optionally, reducing the concentration of iron and copper in the culture medium; and
- favouring production of immature non-fucosylated glycoproteins by either (i) decreasing the concentrations of each of iron, copper, zinc and manganese in the culture medium, or (ii) increasing the concentrations of each of copper and iron and decreasing the concentration of each of zinc and manganese in the culture medium.

In one aspect, the invention includes methods as described above wherein in order to favour biomass generation the concentrations of iron, copper, zinc and manganese are adjusted in the culture medium to:

- (a) iron - from 15 μM to more than 80 μM;
- (b) copper - from 0.3 μM to more than 2.5 μM;
- (c) zinc - from 20 μM to more than 50 μM; and
- (d) manganese - from 0.01 μM to more than 3 μM.

In one aspect, the invention includes methods as described above wherein in order to favour increased maturity in expressed N-glycosylated glycoproteins, the concentrations of iron, copper, zinc and manganese [in (i)] or the concentrations of zinc and manganese [in (ii)], are adjusted in the culture medium to:

(i)

- (a) iron - from 0 μM to 25 μM;
- (b) copper - from 0 μM to 0.1 μM;
- (c) zinc - from 20 μM to more than 50 μM; and
- (d) manganese - from 0.01 μM to more than 3 μM; or

(ii)

- (a) zinc - from 20 μM to more than 50 μM; and
- (b) manganese - from 0.01 μM to more than 3 μM.

In one aspect, the invention includes methods as described above wherein in order to favour production of immature non-fucosylated glycoproteins either the concentrations of iron, copper, zinc and manganese are adjusted in the culture medium to either:

(i)

- (a) iron - from 0 μM to 35 μM;
- (b) copper - from 0 μM to 1 μM;
- (c) zinc - from 0 μM to 20 μM; and
- (d) manganese - from 0 μM to 0.01 μM; or

(ii)

- (a) iron - from 15 μM to more than 80 μM;
- (b) copper - from 0.3 μM to more than 2.5 μM;
- (c) zinc - from 0 μM to 20 μM; and
- (d) manganese - from 0 μM to 0.01 μM.

In one aspect, the invention includes methods as described above wherein the glycoprotein is exogenous or endogenous to the eukaryotic cell, optionally wherein the glycoprotein is a structural glycoprotein, hormone, antibody or enzyme.
In one aspect, the invention includes methods as described above wherein the glycoprotein is an antibody, optionally wherein the antibody is a therapeutic or diagnostic antibody, optionally a chimeric, humanized or human antibody.
In one aspect, the invention includes methods as described above wherein the eukaryotic cell is a mammalian cell, a yeast cell or an insect cell.
In one aspect, the invention includes methods as described above wherein the concentrations of iron, copper, zinc and manganese are adjusted during the growth and/or productions culture phases.
In one aspect, the invention includes methods as described above wherein an increase in the concentration of any or all of iron, copper, zinc and manganese in the culture medium is achieved by supplementing the medium in which the cells are cultured with any or all of iron, copper, zinc and manganese and/or by splitting the cells into a fresh medium supplemented with any or all of iron, copper, zinc and manganese.
In the invention any decrease in the bioavailable concentration of any or all of iron, copper, zinc and manganese in the culture medium is achieved by complexing the iron, copper, zinc and manganese with a chelator and/or by seeding the cells into a fresh medium containing a reduced concentration of any or all of iron, copper, zinc and manganese compared to the medium of the immediately preceding culture phase.
In one aspect, the invention includes methods as described above wherein the concentrations of each of iron, copper, zinc and manganese in the culture medium are adjusted during the culture first to favour biomass generation and then to favour either production of mature N-glycosylated glycoproteins or production of immature non-fucosylated glycoproteins.

Also described is a medium suitable for production of a recombinant glycoprotein under fermentation culture conditions in a eukaryotic cell, the medium comprising a concentration of each of iron, copper, zinc and manganese to favour biomass generation and/or to affect N-glycan maturity of the expressed glycoprotein.

In one case, the medium comprises concentrations of iron, copper, zinc and manganese of:

- (a) iron - from 15 $\mu$M to more than 80 $\mu$M;
- (b) copper - from 0.3 $\mu$M to more than 2.5 $\mu$M;
- (c) zinc - from 20 $\mu$M to more than 50 $\mu$M; and
- (d) manganese - from 0.01 $\mu$M to more than 3 $\mu$M.

The use of such a medium in the production of a recombinant glycoprotein under fermentation culture conditions in a eukaryotic cell favours biomass generation.

In one case, the medium comprises concentrations of iron, copper, zinc and manganese, or of zinc and manganese, of:

(i)

- (a) iron - from 0 $\mu$M to 25 $\mu$M;
- (b) copper - from 0 $\mu$M to 0.1 $\mu$M;
- (c) zinc - from 20 $\mu$M to more than 50 $\mu$M; and
- (d) manganese - from 0.01 $\mu$M to more than 3 $\mu$M; or

(ii)

- (a) zinc - from 20 $\mu$M to more than 50 $\mu$M; and
- (b) manganese - from 0.01 $\mu$M to more than 3 $\mu$M.

Use of a medium comprising iron, copper, zinc and manganese concentrations or of zinc and manganese of (i) or (ii) respectively, in the production of a recombinant glycoprotein under fermentation culture conditions in a eukaryotic cell favours increased maturity in expressed N-glycosylated glycoproteins.

In one case, the medium comprises concentrations of iron, copper, zinc and manganese of either:

(i)

- (a) iron - from 0 $\mu$M to 35 $\mu$M;
- (b) copper - from 0 $\mu$M to 1 $\mu$M;
- (c) zinc - from 0 $\mu$M to 20 $\mu$M; and
- (d) manganese - from 0 $\mu$M to 0.01 $\mu$M; or

(ii)

- (a) iron - from 15 $\mu$M to more than 80 $\mu$M;
- (b) copper - from 0.3 $\mu$M to more than 2.5 $\mu$M;
- (c) zinc - from 0 $\mu$M to 20 $\mu$M; and
- (d) manganese - from 0 $\mu$M to 0.01 $\mu$M.

Use of a medium comprising iron, copper, zinc and manganese concentrations of (i) or (ii), above in the production of a recombinant glycoprotein under fermentation culture conditions in a eukaryotic cell favours production of immature non-fucosylated glycoproteins.

DESCRIPTION OF THE FIGURES

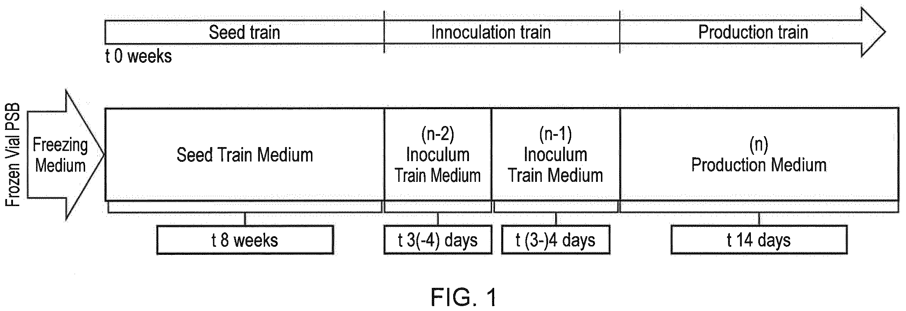

Figure 1: **Process steps of a fermentation campaign.** Frozen vials e.g. of a Primary Seed Bank (PSB), are thawed in seed train medium. Here, cells are cultivated for roughly three weeks to recover form thawing stress (normalizing cell doubling times). The cells are subsequently passed to inoculation train n-1 and n-2 for the expansion of the culture. After approximately one week the cells are transferred to the production scale for a fed-batch fermentation process of two weeks.

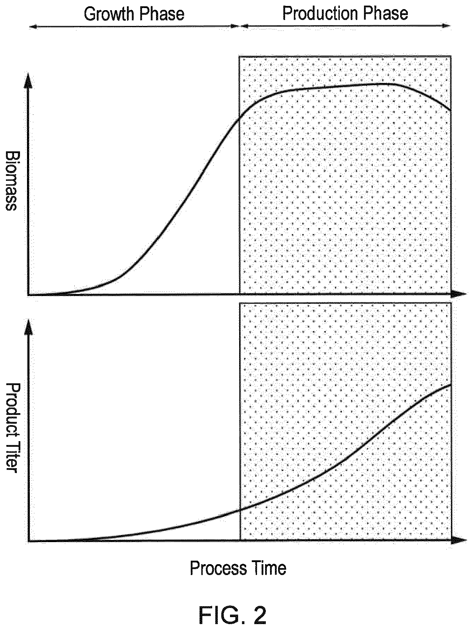

Figure 2: **Fed-batch processes are characterized by two phenotypically distinct phases.** The production phase is separated by two segments where either cell growth or target protein production dominate.

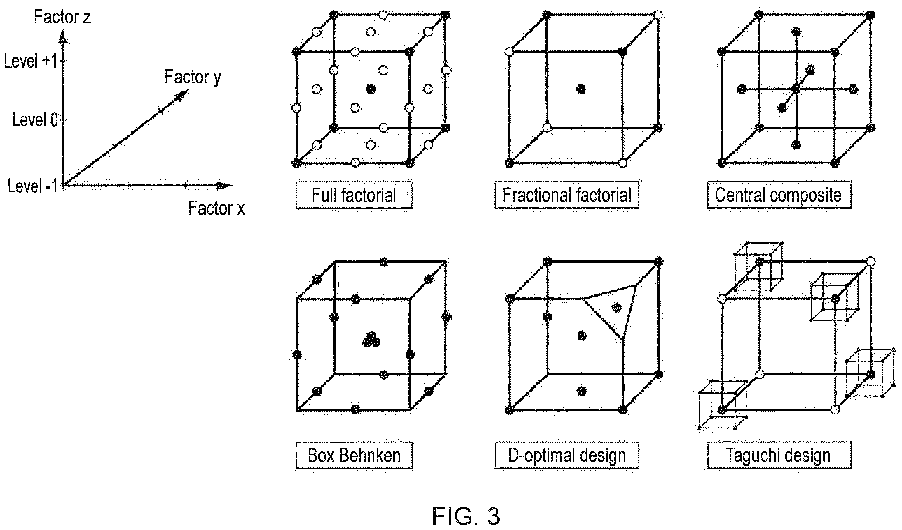

Figure 3: **Different Approaches for DoE Experiments.** Dependent on desired experiment outcome, different DoE approaches can be used for process optimization. For example, optimization of three factors are normally varied between a minimum (-1), medium (0), and maximum level (+1) for optimization DoEs. (Picture adopted from http://www.gmpua.com and modified)

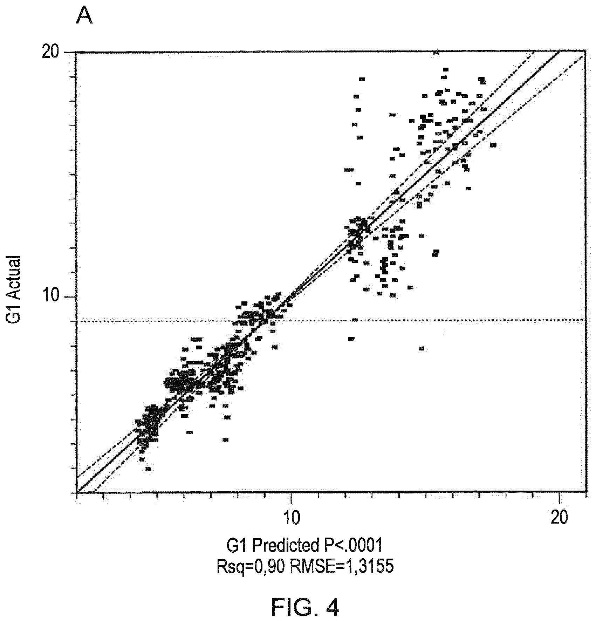

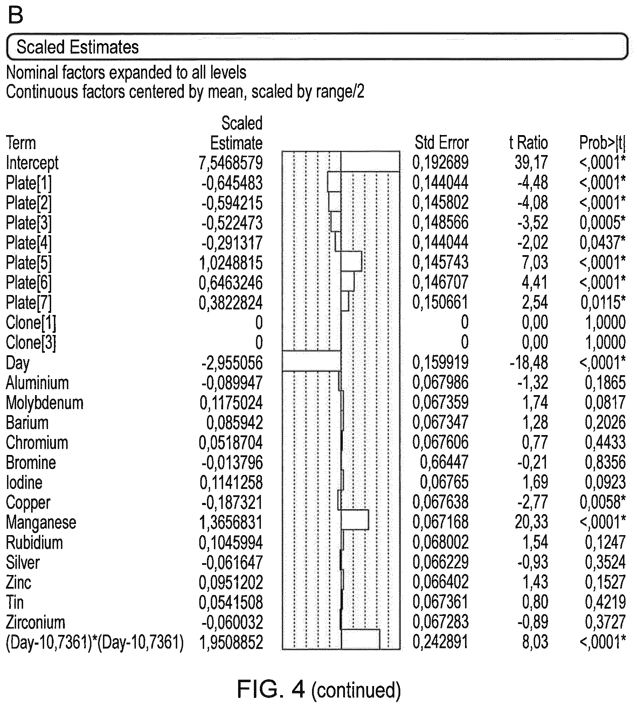

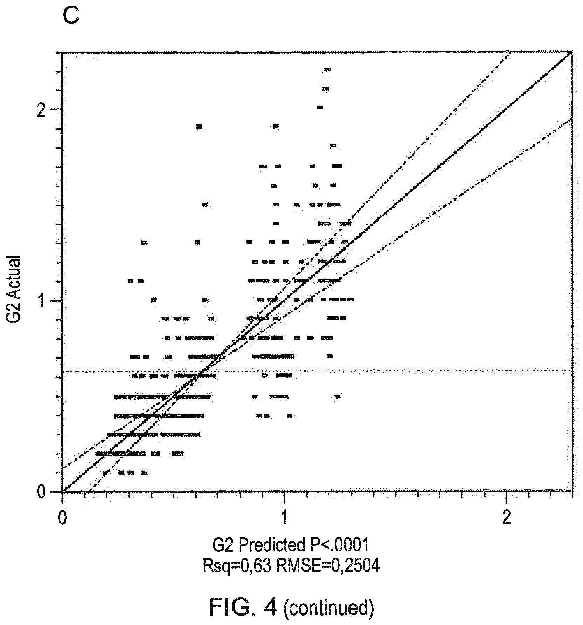

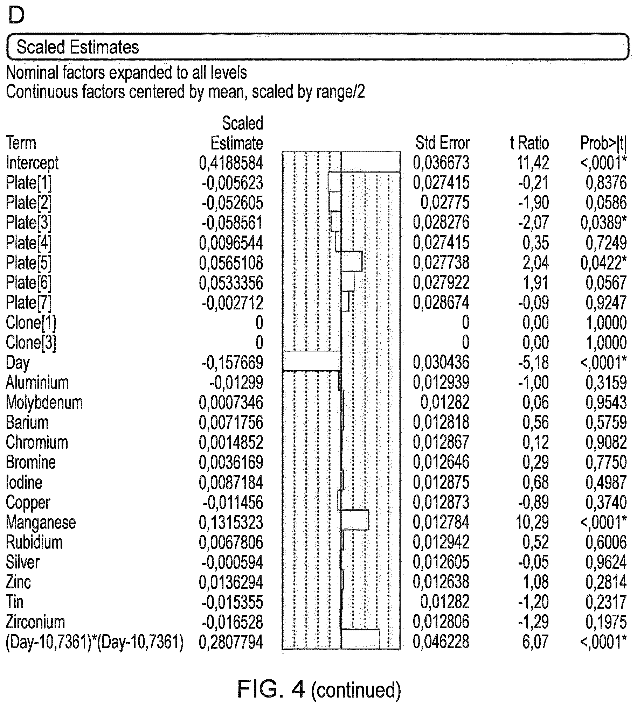

Figure 4: **Effect of trace elements on clone independent mAb galactosylation.** Aluminum, molybdenum, barium, chromium, bromine, iodine, copper, manganese, rubidium, silver, zinc, tin, and zirconium salt stocks were supplemented to a fed-batch culture of clone 1 (n=4) and clone 3 (n=3) using a proprietary cell culture platform A (medium and process) at the start of the experiment. The effect of the trace elements on mAb galactosylation at day 7, 12, and 13 was analyzed by modelling G1 (A, B) and G2 (C, D) levels with existing experimental data. Each point in the *actual* vs *predicted* graphs (A, C) represents a single cultivation experiment. The statistical significance of each trace element on G1 and G2 levels was centred by mean and scaled by range/2 for G1 (B) and G2 (D).

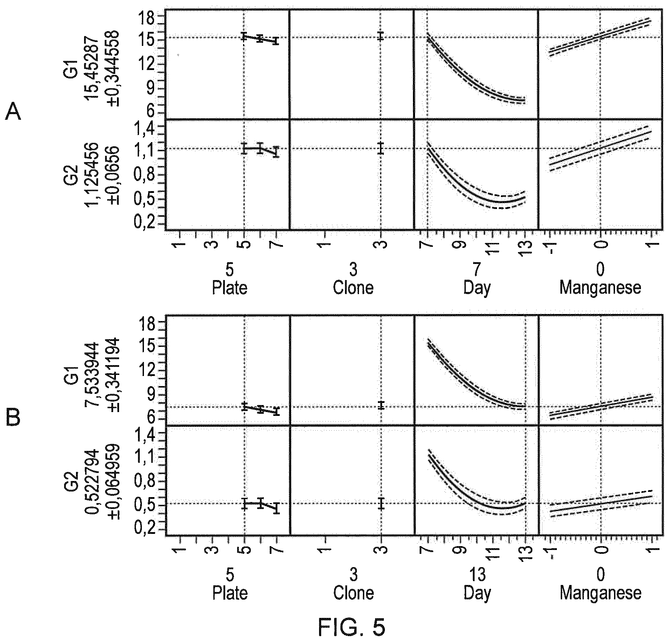

Figure 5: **Timing of regulation of mAb galactosylation by manganese.** The significant effect of manganese on G1 (A) and G2 (B) of clone 3 (plate 5, 6, and 7) glycan formation was analyzed by the prediction profiler tool of JMP software. The positive effect of manganese on G1 and G2 differs for early (day 7) and late process phases (day 13) up to 100% (slope of prediction profiler curve). The experiment was performed with n=3.

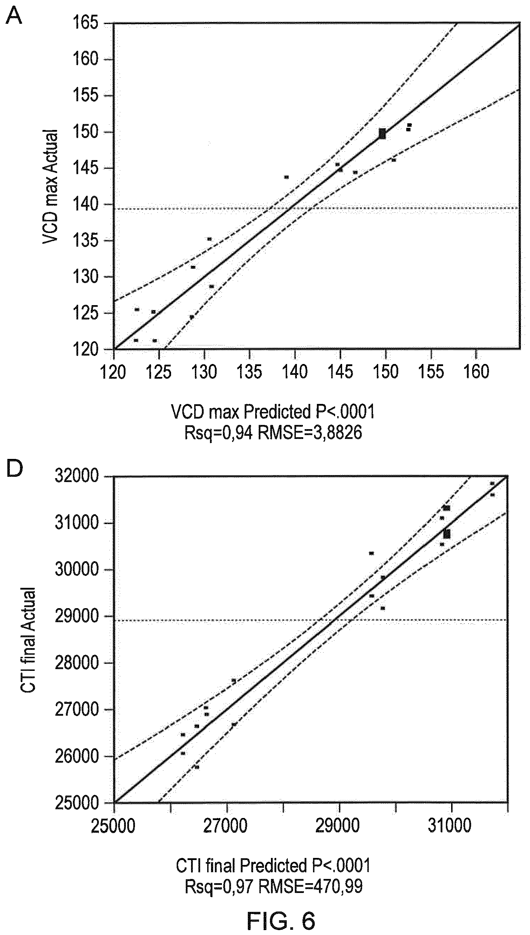

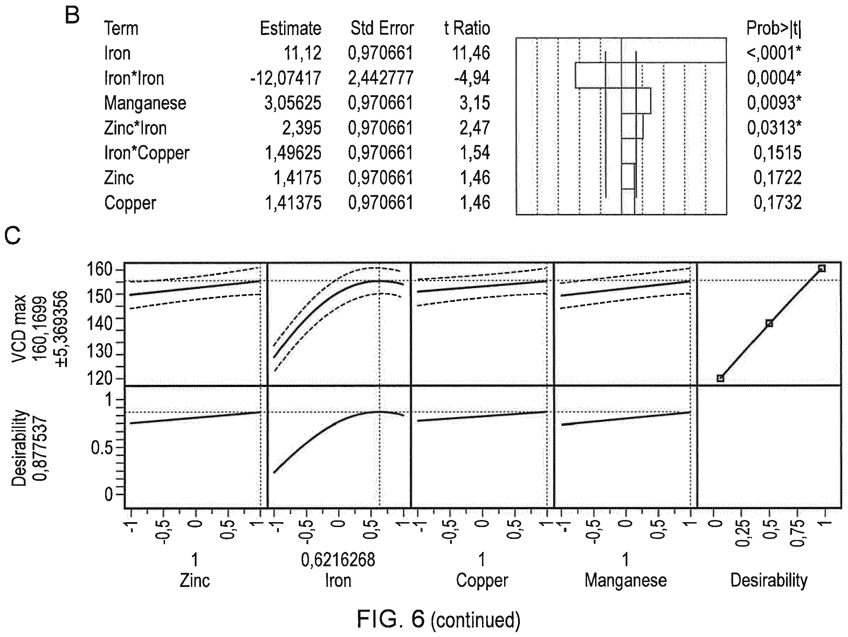

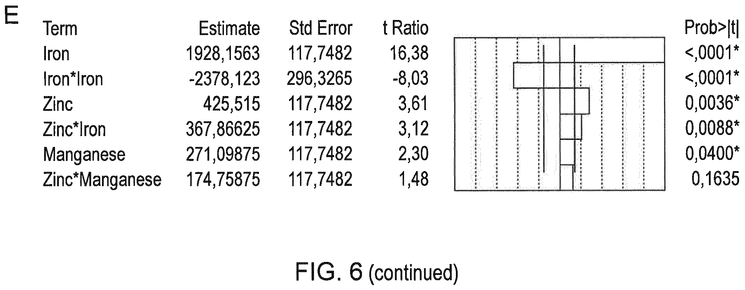

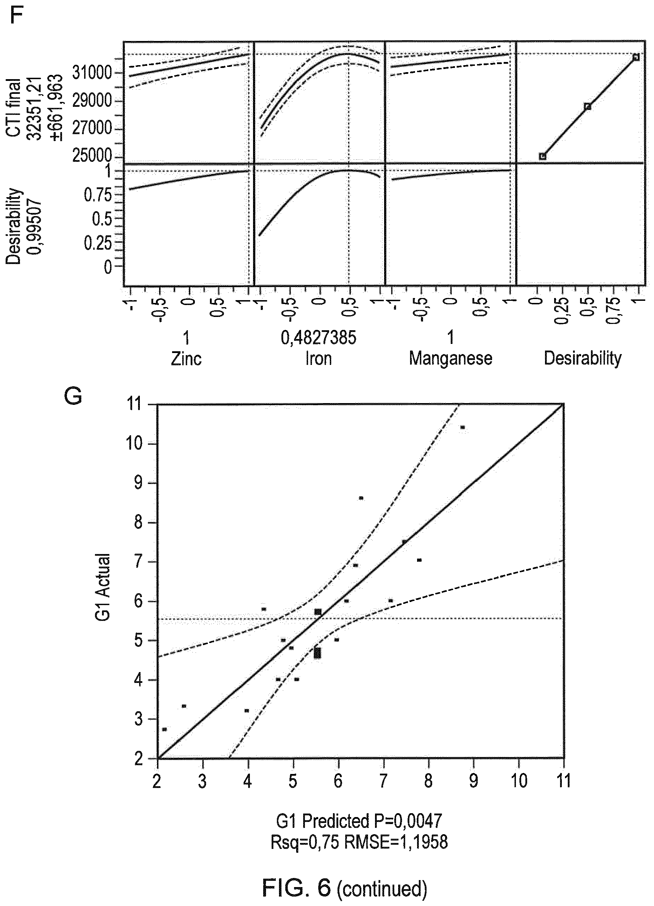

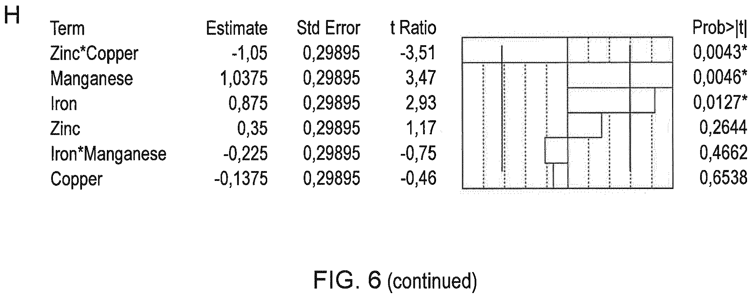

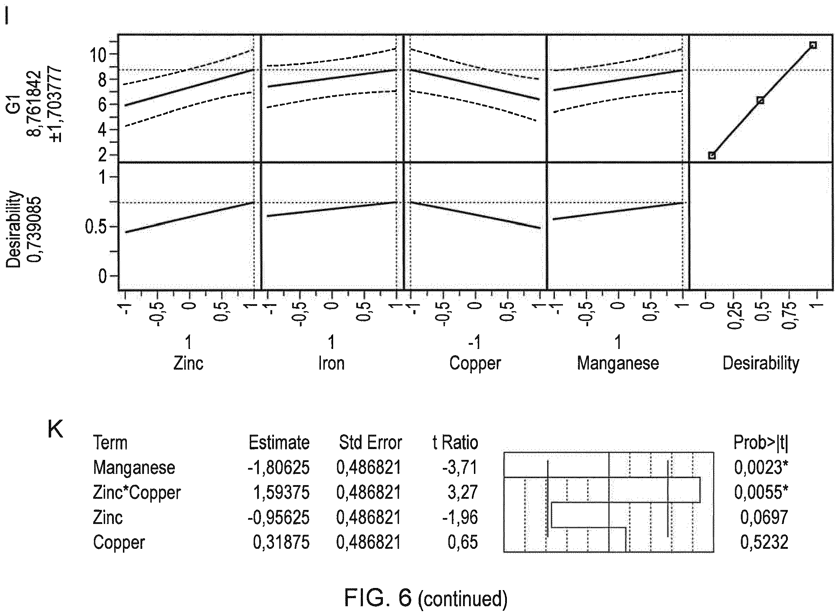

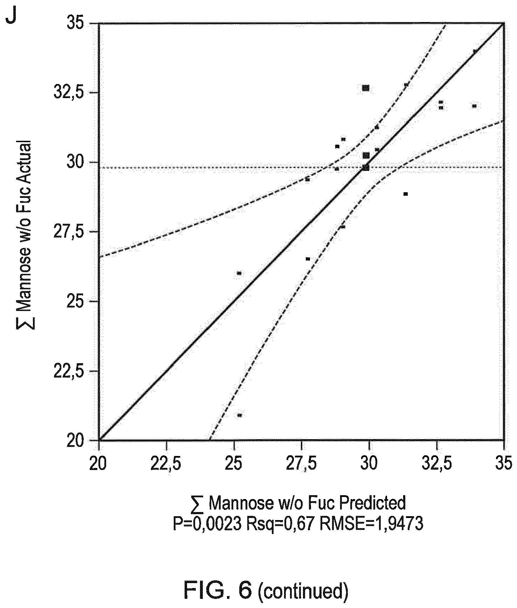

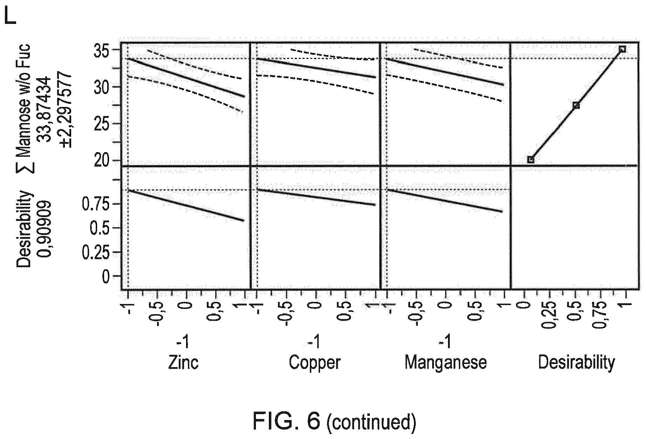

Figure 6: **Different effects of combined zinc, iron, copper, and manganese supplementation on cell growth, galactosylated and non-fucosylated glycoprotein species.** Fed-batch experiments with clone 2 were supplemented with varying concentrations of zinc, iron, copper, and manganese in a DoE experiment. The effects were analyzed for maximum viable cell density (A-C), final cell time integral (D-F), G1 species (G-I), and sum of mannosylated species w/o Fuc (J-L). The predicted models (A, D, G, J) and sorted parameter estimates (B, E, H, K) show the quality and trace elements for each specific model with the significant factors for cell growth and glycosylation, respectively. The optimal concentrations (grey numbers: normalized concentration, dotted horizontal line: maximum value) of zinc, iron, copper, and manganese in fed-batch cultures for optimal cell growth (C, F), optimal galactosylation of the glycoprotein (I), and high non-fucosylated (afucosylated) glycoprotein species (L) were determined with the maximization tool for the JMP prediction profiler. Note, for glycosylation the trace metals zinc and copper show inverted interactions.

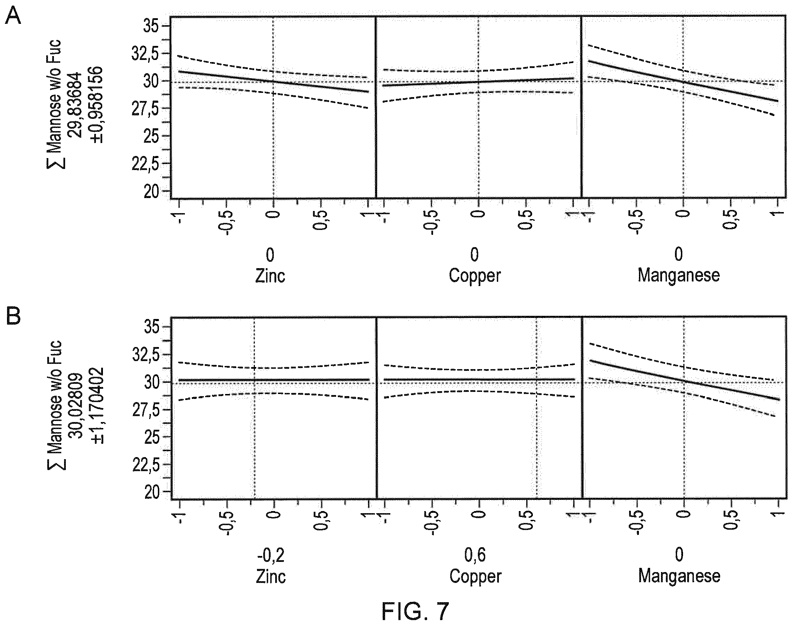

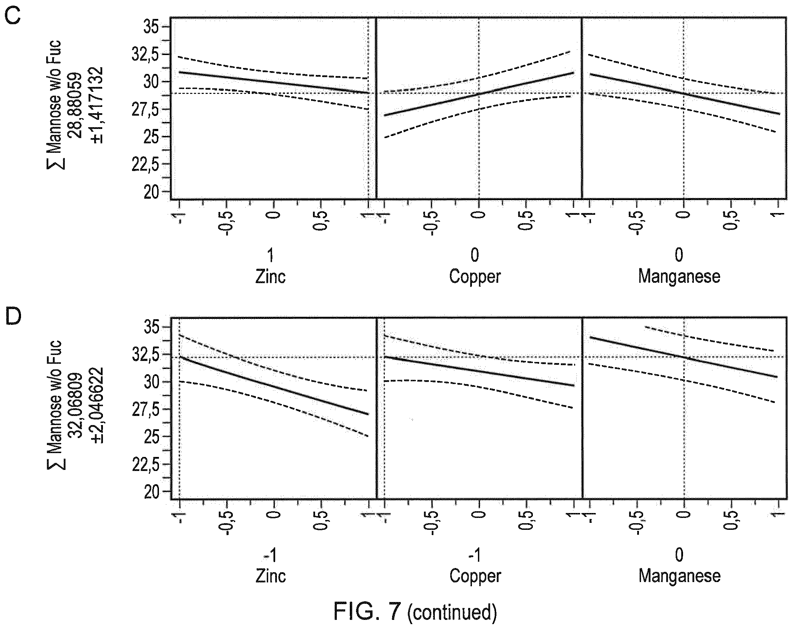

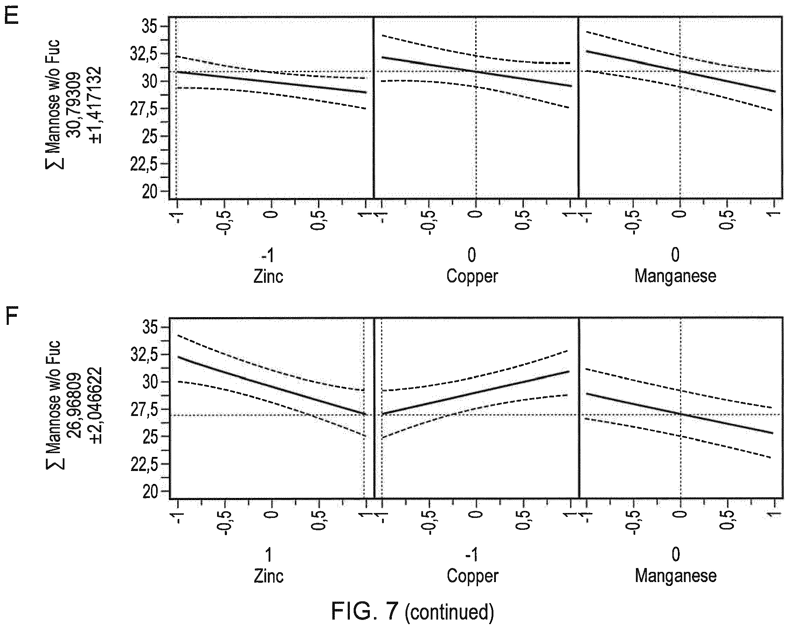

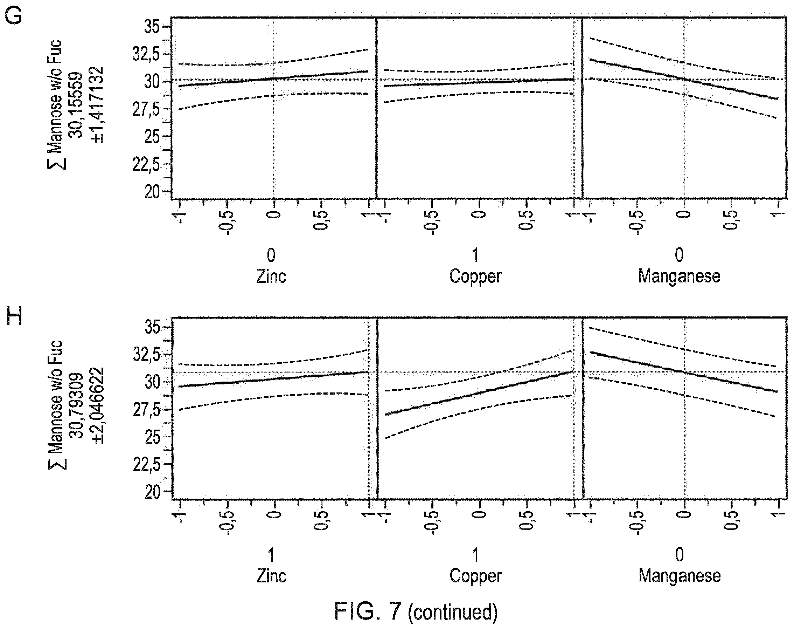

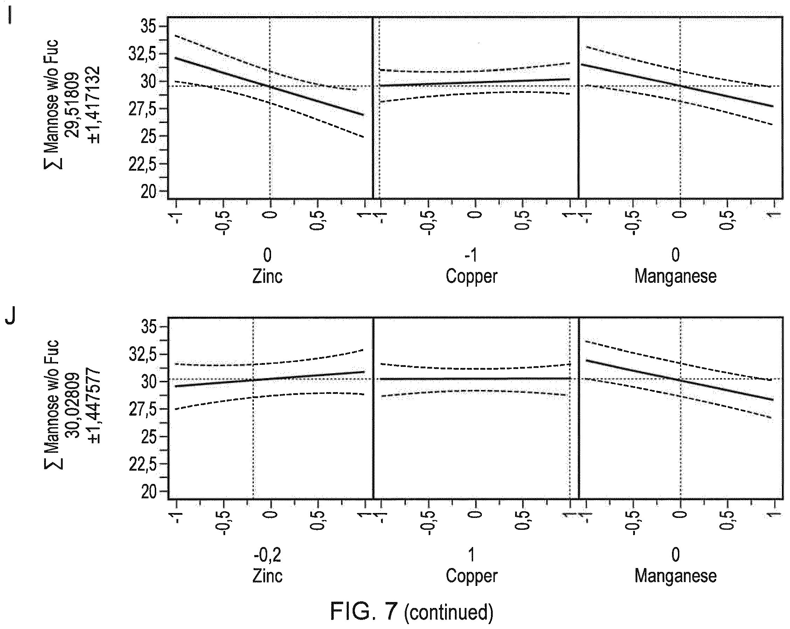

Figure 7: **Interaction of zinc and copper on non-fucosylated glycoprotein species.** Using the JMP prediction profiler tool the impact of zinc levels on copper's correlation for mannosylated glycan species was analyzed. Moderate levels of zinc and copper (A), balanced zinc and copper levels (B), high zinc and moderate copper levels (C), low zinc and low copper levels (D), low zinc and moderate copper levels (E), high zinc and low copper levels (F), moderate zinc and high copper levels (G), high zinc and high copper levels (H), moderate zinc and low copper levels (I), and zinc level -0.2 (i.e. 25.416 $\mu$M) at high copper concentrations (J).

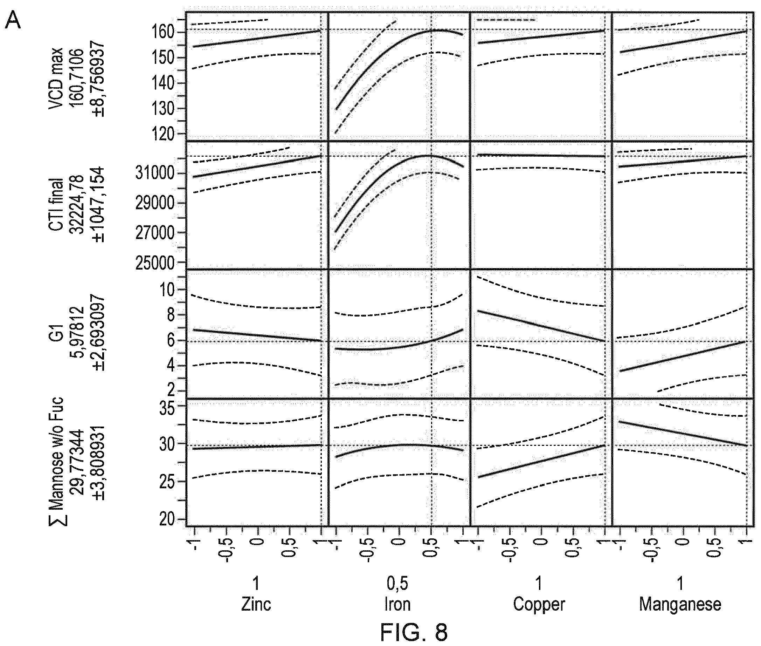

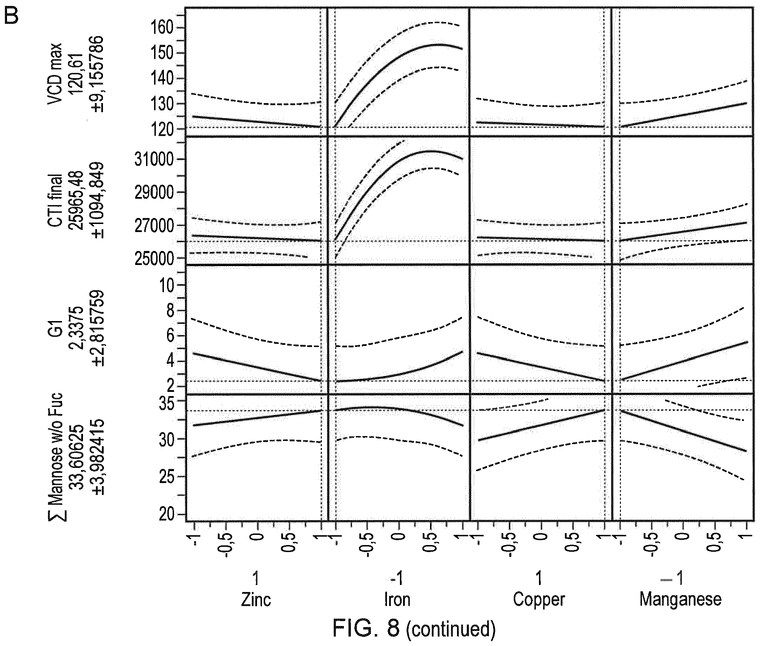

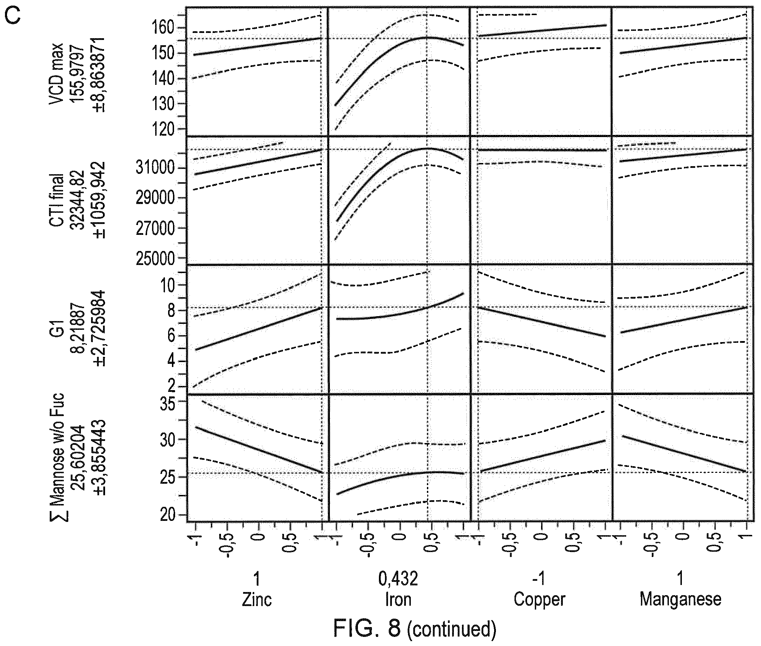

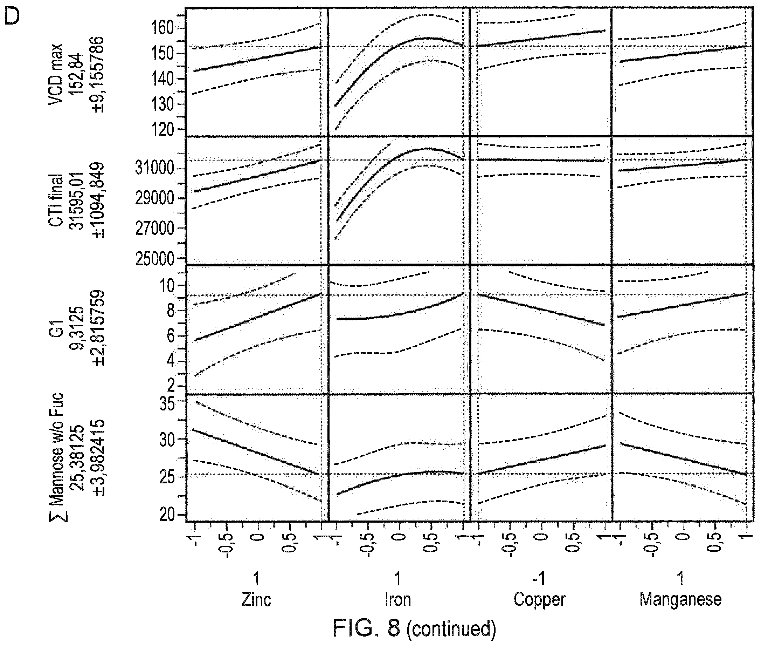

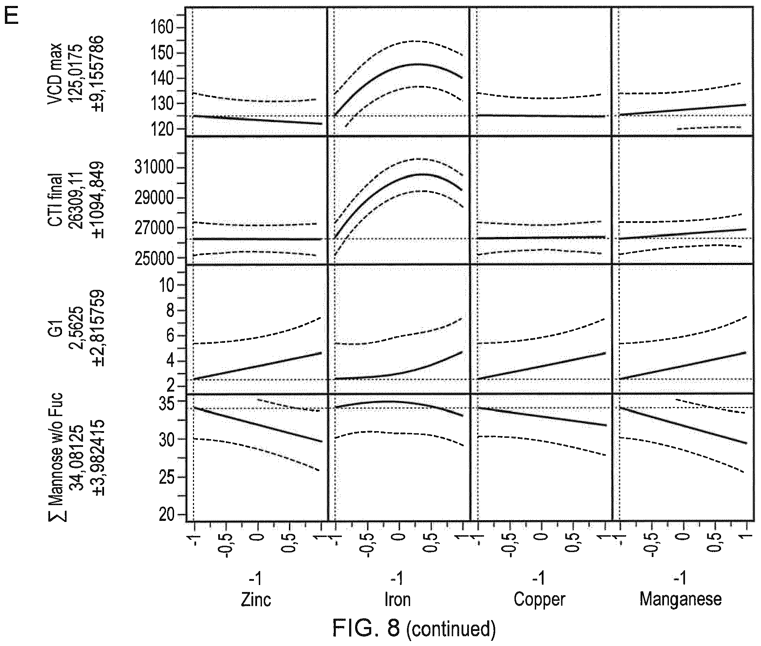

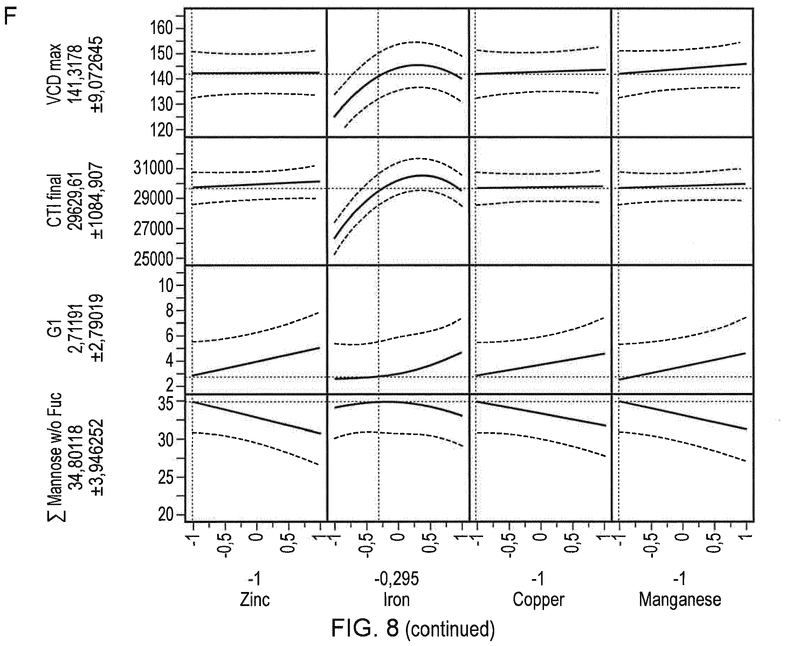

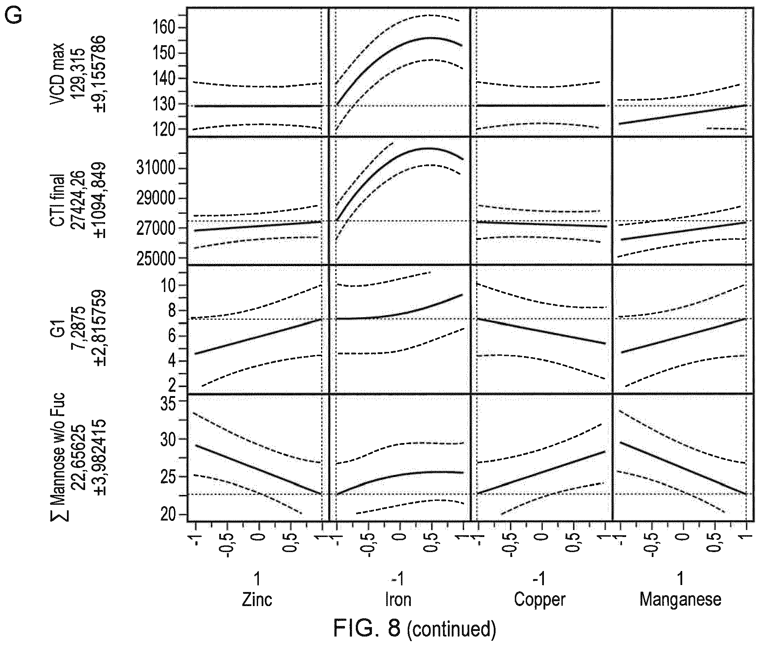

Figure 8: **Optimal concentrations for zinc, iron, copper, and manganese on cell growth, galactosylated, and non-fucosylated glycoprotein species.** Using a single valid prediction model for VCD, CTI, level of mAb galactosylation and high mannose glycans (Man5) the effects of different zinc, iron, copper, and manganese variations are illustrated.

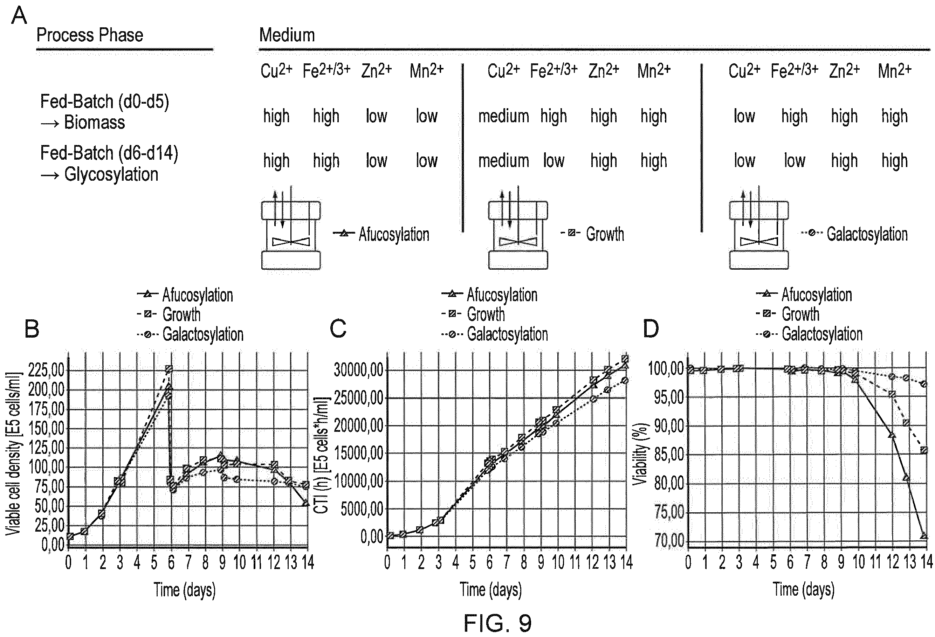

Figure 9: **Modulation of cell growth by targeted trace element concentrations.** The predicted trace element concentrations favouring cell growth and mAb glycosylation were tested in three bioreactor fed-batch experiments, "*afucosylation*" (triangle), "*Growth*" (square), and "*Galactosylation*" (circle) using clone 2 and the proprietary medium and process platform B (A). The effect on viable cell density (B), cell time integral (C), and cell viability (D) were analyzed. Afucosylation is the same as non-fucosylation.

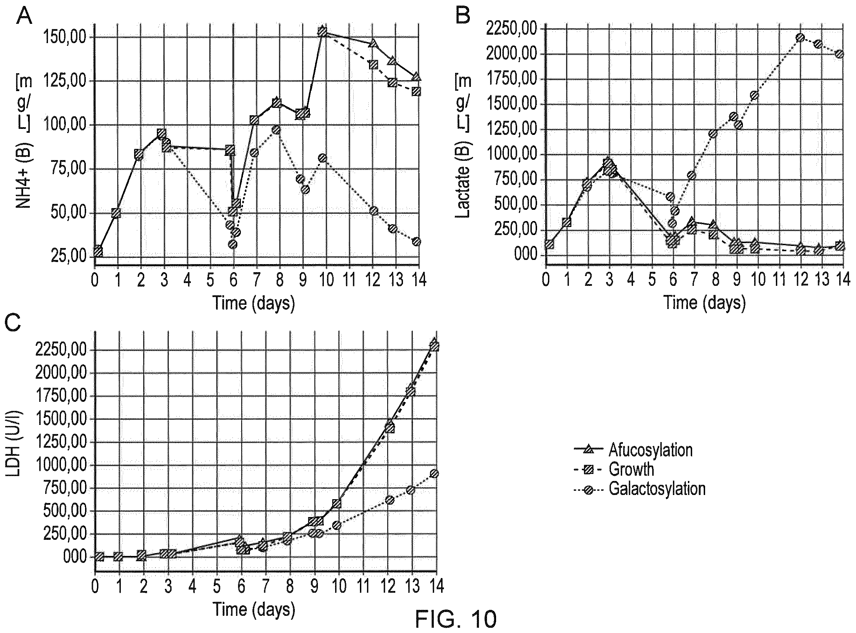

Figure 10: **Different cell metabolism by supplementation of copper.** Using "Galactosylation" process in the "glycosylation" phase (d6-d14) reduced final ammonium (A) and increased lactate (B) levels. LDH assessment as marker for lysed cells show a 50% reduced level compared to control and "Growth" process.

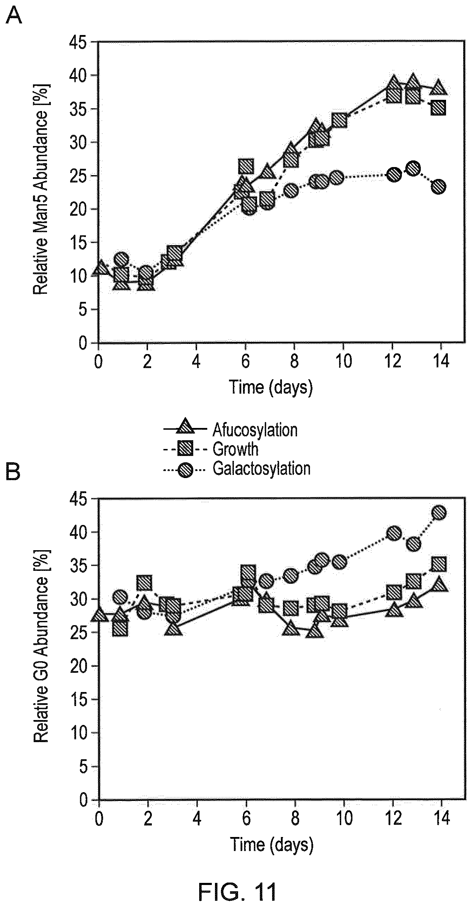

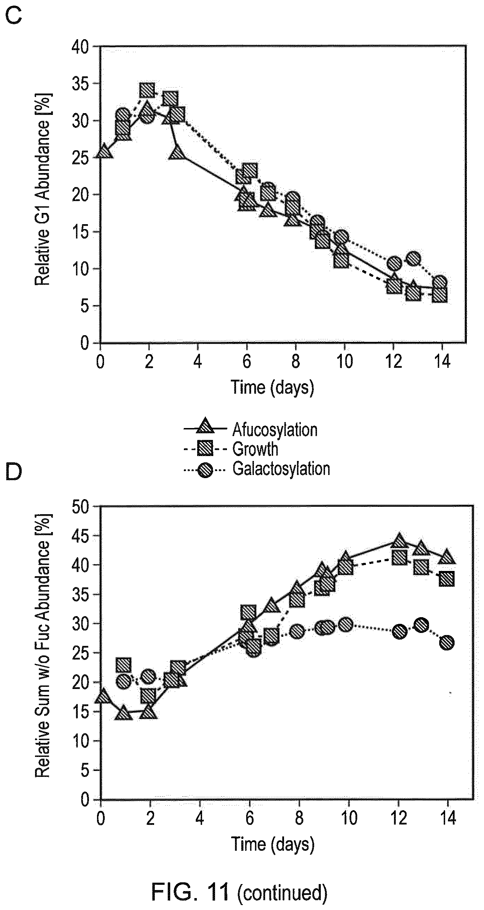

Figure 11: **Modulation of galactosylated and non-fucosylated species by targeted trace element concentrations.** The predicted trace element concentrations favouring mAb glycosylation were tested in three bioreactor fed-batch experiments, "*afucosylation*" (triangle), "*Growth*" (square), and "*Galactosylation*" (circle) using clone 2 and the proprietary medium and process platform B. The effect of targeted trace element modulation on relative abundance of Man5 (A), G0 (B), and G1 (C) species as well for cumulative non-fucosylated species (D) was analyzed.

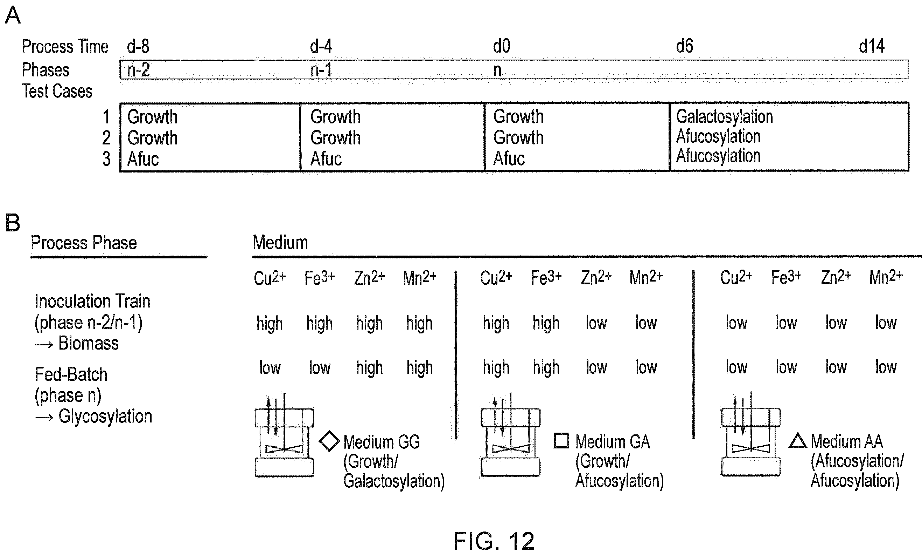

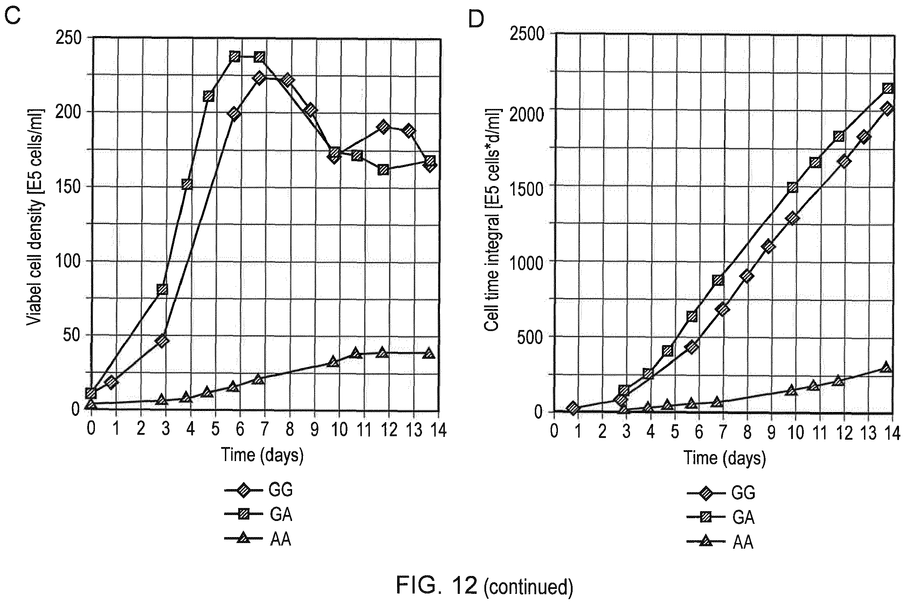

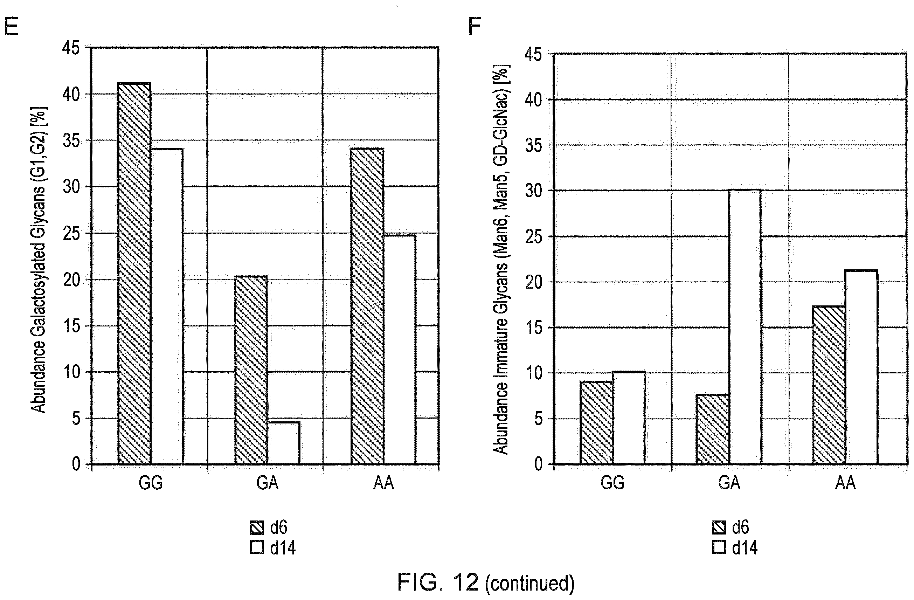

Figure 12: **Role of trace metals on cell growth and galactosylation/non-fucosylation on clone 1 - Bioreactor Fed-Batch experiment** Predicted trace element concentrations favouring cell growth and modulation of mAb glycosylation maturation were tested in three bioreactor fed-batch experiments, combining good and bad cell growth during the inoculation train (phase n-2/n-1) and mAb galactosylation and non-fucosylation (formation of immature glycans) in fed-batch (phase n). The test cases should specifically modulate cell growth, protein galactosylation and immature glycosylation during inoculation train and production phase (A). Different trace element concentrations for intended experiment set-up are depicted in (B). Bioreactor "GG" (diamond) supports cell growth and protein galactosylation, bioreactor "GA" (square) supports cell growth and mAb non-fucosylation and bioreactor "AA" (triangle) repress cell growth and trigger protein non-fucosylation. The effect on viable cell density (C), cell time integral (D), mAb maturation (sum G1 and G2 species) on day 6 and 14 (E) and mAb immaturation (sum Man6, Man5 and G0-GlcNAc) on day 6 and 14 (F) were analyzed.

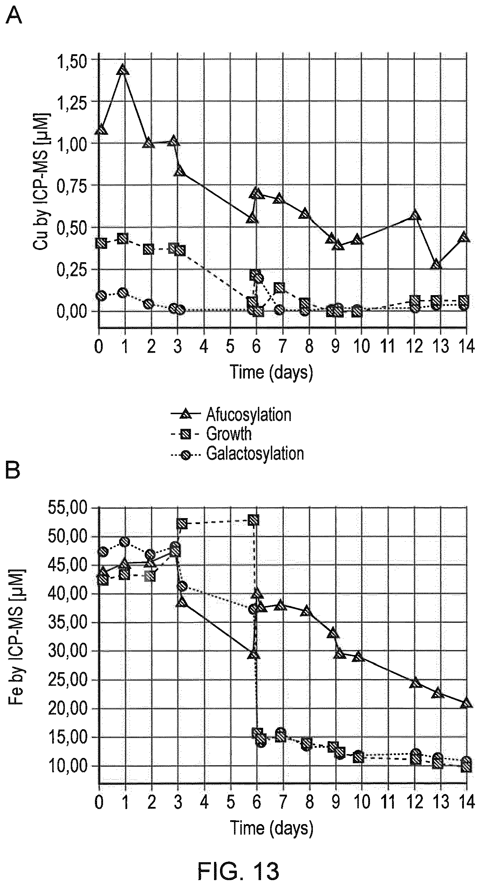

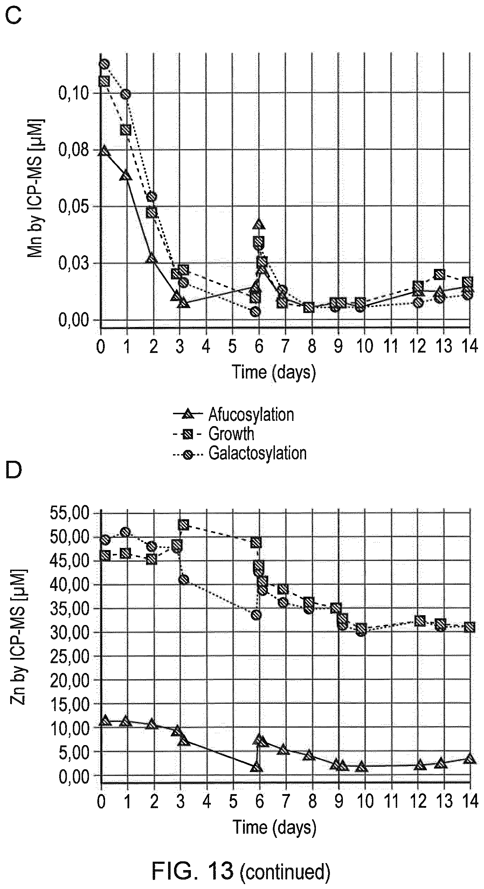

Figure 13: **Measured concentrations of iron, zinc, manganese and copper for bioreactor experiment 1.** Actual concentrations of copper (A), iron (B), manganese (C) and zinc (D) measured by ICP-MS analysis of cell culture supernatant are shown. The gradual increase of theoretical zinc, iron, manganese and copper concentration is the consequence of bolus feeding at day 3, 5 and 9. At day 6 the culture was split.

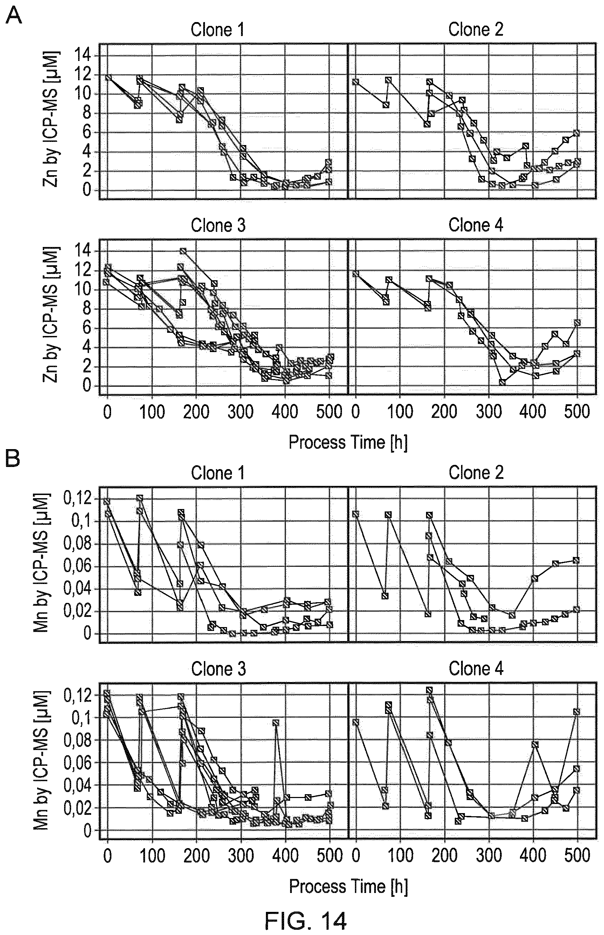

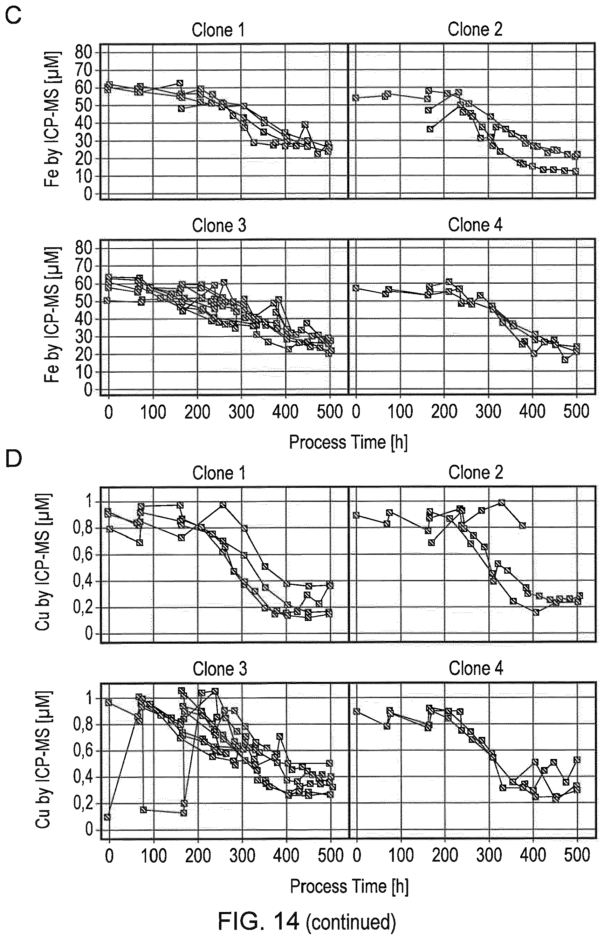

Figure 14: **Iron, zinc, manganese and copper concentrations for medium calculations in bioreactor experiment 2.** Actual concentrations of zinc (A), manganese (B), iron (C) and copper (D) for representative fed-batch cultivations inclusive phases n-2, n-1, n of clone 1, clone 2, clone 3, clone 4 (n>3) measured by ICP-MS analysis of cell culture supernatant.

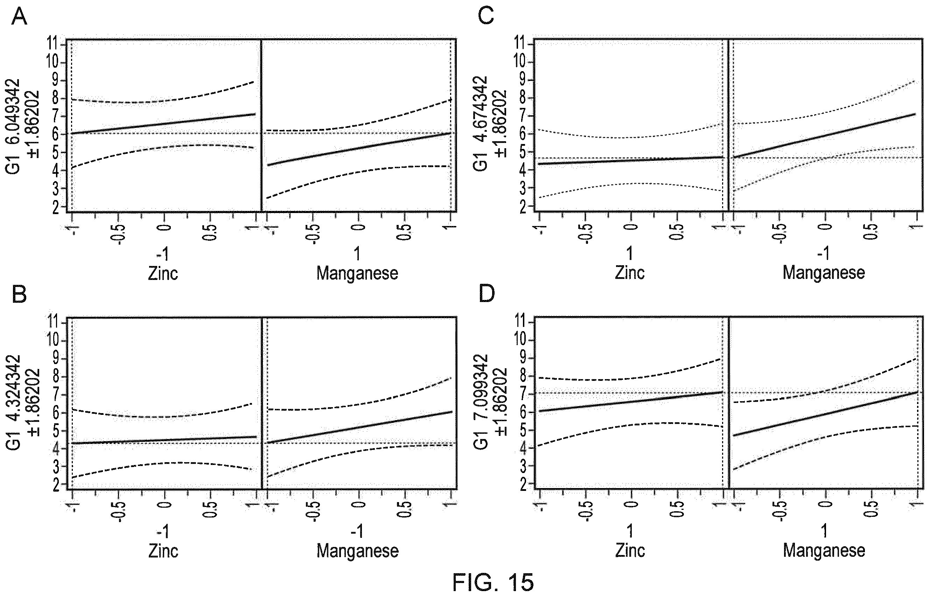

Figure 15: **Interaction of zinc and manganese on galactosylated glycoprotein species.** Using the JMP prediction profiler tool the impact of zinc levels on manganese's correlation for galactosylated G1 glycan species for clone 2 was analyzed. Low levels of zinc and high levels of manganese (A), low levels of zinc and low levels of manganese (B), high levels of zinc and low levels of manganese (C), high levels of zinc and high levels of manganese (D).

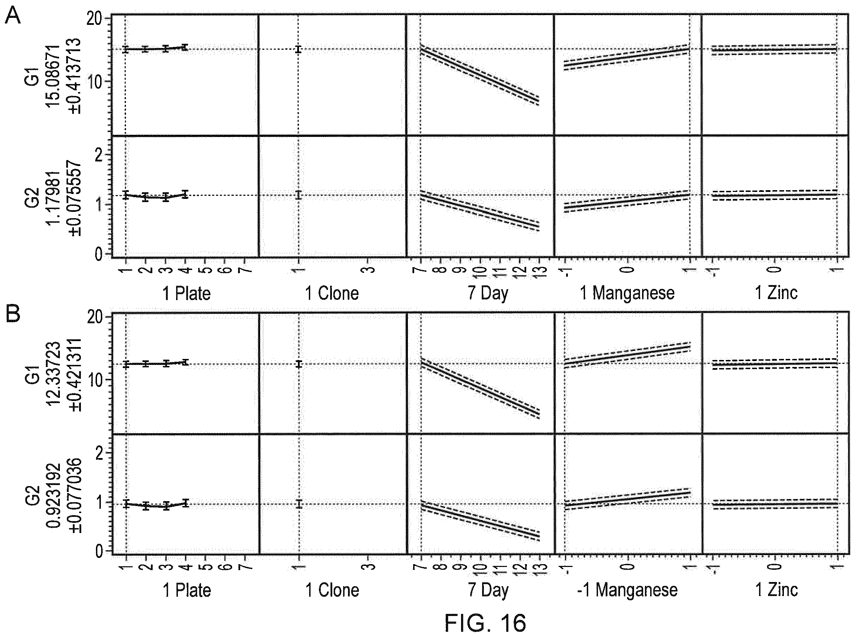

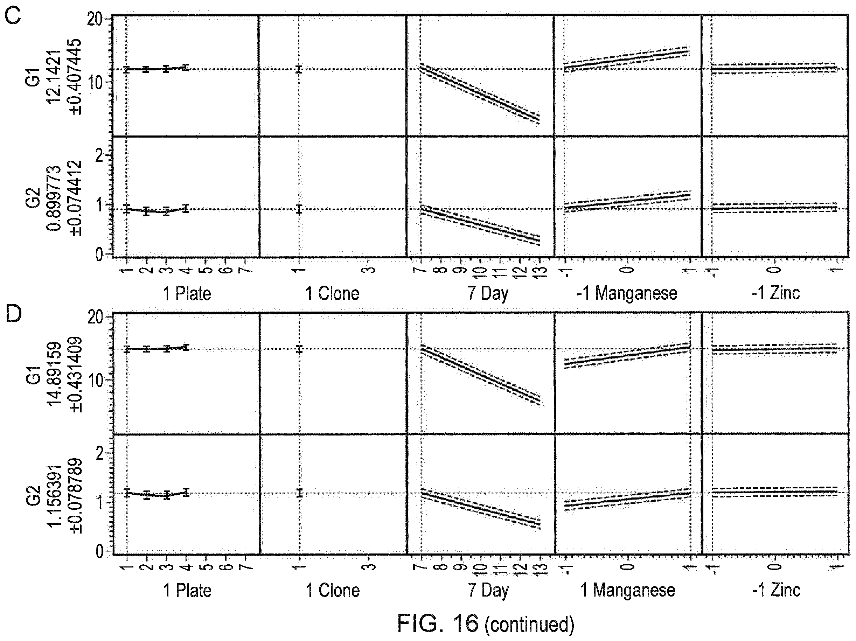

Figure 16: **Interaction of zinc and manganese on galactosylated glycoprotein species is time dependent.** Using the JMP prediction profiler tool the impact of zinc levels on manganese's correlation for galactosylated G1 and G2 glycan species for clone 1 (n=4) and clone 3 (n=3) were analyzed. High levels of zinc and high levels of manganese (A), high levels of zinc and low levels of manganese (B), low levels of zinc and low levels of manganese (C), low levels of zinc and high levels of manganese (D).

TERMINOLOGY

"Adjusting" as used herein refers to increasing or decreasing the concentration of an element in the culture medium. The increase or decrease in the concentration of the element is relative to the concentration of the element in the medium in the culture phase immediately preceding the adjustment. For example, if the adjustment is an increase in trace elements and is required at the start of the production phase, this is an increase in the concentration of those trace elements over the concentration of those elements included in the medium of the immediately preceding growth phase.

"Adjusting the concentration" as used herein refers to a change in the measured or measurable or in the calculated or calculable actual concentration of the element in the medium surrounding the cells at a given time point.

"Affect" as used herein refers to an action which results in a change in the processes of the cell, be that either biomass generation or N-glycan maturity. The effect resulting therefrom may be e.g. an increase in biomass generation or an increase in production of a glycoprotein with the desired glycosylation pattern.

"And/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

"Antibody" as used herein refers to an immunoglobulin molecule or an immunologically active portion of an immunoglobulin molecule, i.e. a molecule that contains an antigen binding site, such as a Fab or F(ab')$_2$ fragment, whether natural or partly or wholly synthetically produced. The term "antibody" is used in its broadest sense and covers monoclonal antibodies (including full length antibodies which have an immunoglobulin Fc region or intact monoclonal antibodies), antibody compositions with polyepitopic specificity, polyclonal antibodies, multivalent antibodies (typically engineered to have three or more antigen binding sites), multispecific antibodies (e.g. bispecific antibodies) formed from at least two intact antibodies, diabodies and single chain molecules such as scFv molecules, as well as antibody fragments (e.g. Fab, F(ab')$_2$ and Fv). Included within the definition of antibody are antibody conjugates, such as antibody drug conjugates (ADCs) or antibodies conjugated to e.g. labeling elements.

"Biomass" as used herein refers to the quantity or weight of cultured cells in the culture medium. Biomass may be measured directly or indirectly by determining viable cell density, total cell density, cell time integral (for viable and total cell density), cell volume time integral (for viable and total cell density), packed cell volume, dry weight or wet weight.

"Bioreactor" as used herein refers to any vessel used for the growth of a mammalian cell culture. Typically a bioreactor will be at least 1 litre and may be 10, 100, 250, 500, 1000, 2500, 5000, 8000, 10,000, 12,000 litres or more, or any volume in between. The internal conditions of the bioreactor, including but not limited to pH, dissolved oxygen and temperature, are typically controlled during the culture period. A bioreactor can be composed of any material that is suitable for holding mammalian cell cultures suspended in media under the culture conditions of the present invention, including glass, plastic or metal.

"Cell" and "cell line" are used herein interchangeably and all such designations include progeny.

"Cell density" as used herein refers to the number of cells present in a given volume of medium.

"Cell viability" as used herein refers to the ability of cells in culture to survive under a given set of culture conditions or experimental variations. The term as used herein also refers to that portion of cells which are alive at a particular time in relation to the total number of cells, living or dead, in culture at that time.

"Chelator" as used herein refers to a compound capable of suppressing chemical activity by forming a chelate, i.e. by binding a metal ion.

"Concentration" as used herein in relation to trace elements iron, copper, zinc and manganese refers to the amount of each trace element comprised within a culture medium. The concentration may be the measured or measurable or the calculated or calculable actual concentration of the element in the medium surrounding the cells at a given point in time. Methods for measuring the concentration of these trace elements in the medium are known in the art. Examples of such methods include ICP-MS (Agilent, Böblingen, Germany). The concentration thus also refers to the amount of each trace element comprised in a culture medium surrounding the cells during culture, and is thus the actual concentration of the respective trace element at a given point in time. This concentration can be determined analytically and results from e.g. the introduction of the element into the culture (by weighing, by transferring cells and medium from a pre-culture, by introduction of impurities, by leaching etc), from release by cells (e.g. by the death of cells or by active secretion), from uptake by cells and other factors.

"Copper" as used herein refers to the Cu$^{2+}$ cation.

"Culture" or "cell culture" as used herein refers to a cell population that is suspended in a medium under conditions suitable for survival and/or growth of the cell population. These terms will also be applied to the combination of the medium and cell population suspended therein.

"Culture conditions" and "fermentation conditions" are used herein interchangeably and are those conditions that must be satisfied to achieve successful cell culture. Typically these conditions include provision of an appropriate medium, as well as control of e.g.

temperature, which should be about 37°C, but could also include a temperature shift during culture (e.g. 37°C to 34°C) and pH, which is normally between 6.8 and 7.2, as well as the provision of oxygen and carbon dioxide. Such conditions also include the manner in which the cells are cultured, e.g. shaker or robotic cultivation.

"Decrease" as used herein in respect of concentrations of the trace elements iron, copper, zinc or manganese, means a decrease in the concentration of those trace elements in the culture medium relative to their concentration in the medium in which the cells were cultured in the immediately preceding phase or partial phase.

"Favour" as used herein means that the activity of the cell in the stated direction will be enhanced as compared to a cell grown under the same conditions but in which the concentrations of each of iron, copper, zinc and manganese have not been adjusted according to the present methods. For example, when biomass generation is favoured, the adjustment of the trace element concentration in the medium will result in the cell being directed more towards multiplying than production of glycoprotein. There will be an increase in biomass generation as a result. Synonymous with "favour" herein are "increase" and "enhance".

"Fed-batch culture" as used herein refers to a method of culturing cells in which additional components are provided to the culture at a time or times subsequent to the beginning of the culture process. A fed-batch culture is typically stopped at some point and the cells and/or components in the medium are harvested and optionally purified.

"Galactosylated" as used herein in respect of glycoproteins, refers to glycoprotein comprising one or more galactose residues, resulting in G1 and G2 glycostructures.

"Gene" as used herein refers to any nucleotide sequence, DNA or RNA, at least some portion of which encodes a polypeptide. Optionally, the gene comprises not only the coding sequence for the polypeptide but also comprises regions preceding and/or following the coding sequence that modulate the basal level of expression and/or introns between the coding segments or exons.

"Glycoform" as used herein refers to any of several different forms of a glycoprotein having different saccharides attached.

"Glycoprotein" as used herein refers to a protein or polypeptide that contains one or more covalently linked oligosaccharide chains. The oligosaccharide chains may be composed of a single sugar residue, a single unbranched chain or sugar residues or may be composed of a chain of sugar residues that branches one or more times. The oligosaccharide chains may be N-linked or O-linked.

"Host cell" as used herein denotes any kind of cellular system which can be engineered to generate glycoproteins.

"Increase" as used herein in respect of concentrations of the trace elements iron, copper, zinc or manganese, means an increase in the concentration of those trace elements in the culture medium relative to their concentration in the medium in which the cells were cultured in the immediately preceding phase or partial phase.

"Iron" as used herein means either the Fe(III) ($Fe^{3+}$) or Fe(II) ($Fe^{2+}$) cation.

"Manganese" as used herein refers to the $Mn^{2+}$ cation.

"Maturity" and "glycoprotein maturity" as used herein refers to the glycosylation pattern in recombinantly produced glycoproteins. All N-glycosylated proteins have a pentasaccharide core in common (core = $GlcNAc_2 Man_3$) and terminal glycosylation, taking place in the Golgi Complex, results in enormous structural diversification due to the differing combinations of oligosaccharides added to that core. Thus increasing maturity in the glycoprotein relates to the addition to the core, and/or subsequent modification, of oligosaccharide units. An effect on N-glycan maturity thus includes increasing the maturity of the glycoprotein relative to the core structure or relative to the immediately preceding carbohydrate structure. Therefore maturity of the glycoprotein is increased as the core structure is modified by the addition of oligosaccharide units or by removal of, for example, mannose residues from an immature hybrid structure. An increase in maturity therefore covers e.g. production of glycoproteins with a G0 structure from e.g. a $Man_{3-5}$ structure or from a core structure. Fully mature glycoprotein species may be galactosylated species containing one or two galactose residues or sialylated species that contain one or two sialic acid residues, such as $GlcNAc_3Man_3GlcNAc_2Gal_1$, $GlcNAc_3Man_3GlcNAc_2Gal_2$, $GlcNAc_3Man_3GlcNAc_2Gal_1Sia_1$, $GlcNAc_3Man_3GlcNAc_2Gal_2Sia_1$ and $GlcNAc_3Man_3GlcNAc_2Gal_2Sia_2$. Immature glycoproteins may be high mannose species containing from four to nine mannose residues or immature hybrid structures such as $GlcNAc_3Man_5GlcNAc$, $GlcNAc_3Man_4GlcNAc$ and $GlcNAc_3Man_3GlcNAc$. In some cases it is desirable to produce glycoproteins in which sugar residues that are normally incorporated are not so incorporated. Included herein are partially or fully non-fucosylated glycoproteins which lack core fucose residues, e.g. G0-F, G1-F and G2-F. Mature and immature glycoprotein species may be non-fucosylated, i.e. they may lack core fucose residues.

"Medium", "cell culture medium" and "culture medium" are used herein interchangeably and refer to a solution containing nutrients which sustain growth of mammalian cells. Typically such solutions provide essential and non-essential amino acids, vitamins, energy sources, lipids and trace elements required by the cell for minimal growth and/or survival. Such a solution may also contain supplementary components that enhance growth and/or survival above the minimal rate including, but not limited to, hormones and/or other growth factors, particular ions, such as sodium, chloride, calcium, magnesium and phosphate, buffers, vitamins, nucleosides or nucleotides, trace elements, amino acids, lipids and/or glucose or other energy source. A medium is advantageously formulated to a pH and salt concentration optimal for cell survival and proliferation. A medium may be a reduced serum or serum free medium, i.e. wherein the medium contains about 1-5% serum or when the medium is essentially free of any mammalian serum (e.g. fetal bovine serum), respectively. By essentially free is meant that the medium comprises between 0-5% serum, preferably between about 0-1% serum and most preferably about 0-0.1% serum. A serum-free defined medium may be used, where the identity and concentration of each of the components of the medium is known. A medium may be a protein-free medium, i.e. this will contain no protein but will contain undefined peptides e.g. from plant hydrolysates. Media could include human serum albumin and human transferrin but potentially animal-derived insulin and lipids, or a xeno-free medium containing human serum albumin, human transferrin, human insulin and chemically defined lipids. Alternatively, a medium may be a chemically-defined medium, that is a medium wherein all substances are defined and present in defined concentrations. These media could contain only recombinant proteins and/or hormones or a protein-free chemically defined medium, i.e. containing only low molecular weight constituents and synthetic peptides/hormones if required. Chemically defined media could also be completely free of any protein.

"Non-fucosylated glycoprotein" is a mature or an immature glycoprotein lacking one or more core N-fucose residues. These structures may be per se non-fucosylated, in that the glycoprotein naturally does not contain fucose residues, or they may be non-fucosylated due to the absence of a fucose residue that naturally would be expected to be present in the glycoprotein. In the present application, non-fucosylated and afucosylated are used interchangeably.

"Perfusion culture" as used herein refers to a method of culturing cells comprising growing cells on an inoculation base medium and, when cells achieve a desired cell density replacing the spent medium with a fresh medium. Perfusing may comprise either continuous or intermittent perfusion and may include delivery of at least one bolus feed to the cell culture. A perfusion culture may be followed by a fed-batch culture.

"Polypeptide" as used herein refers to a sequential chain of amino acids linked together via peptide bonds. No length limitation is imposed on such an amino acid chain, which may comprise from 2 to many amino acids. Polypeptides may be processed and/or modified, such as by glycosylation.

"Protein" as used herein refers to one or more polypeptides that function as a discrete unit. When the protein contains only one polypeptide to function, the terms polypeptide and protein are interchangeable.

"Recombinant glycoprotein" or "recombinantly expressed glycoprotein" as used herein refer to a glycoprotein expressed from a host cell manipulated for the purposes of such expression. Manipulation includes one or more genetic modifications such as introduction of one or more heterologous genes encoding the glycoprotein to be expressed. The heterologous gene may encode a glycoprotein either that is normally expressed in that cell or that is foreign to the host cell. Manipulation may alternatively be to up- or down-regulate one or more endogenous genes.

"Splitting" as used herein is also known as passaging or subculture of cells. This involves transferring a small number of cells into a fresh medium, whereby the split cells seed the new culture. In suspension cultures, a small amount of the culture containing a few cells is diluted into a larger volume of fresh medium.

"Titre" as used herein refers to the total amount of recombinantly expressed glycoprotein produced by a mammalian cell culture in a given amount of medium volume. Titre is typically expressed in units of milligrams of glycoprotein per millilitre of medium.

"Zinc" as used herein refers to the $Zn^{2+}$ cation.

The following abbreviations are used herein:

| | |
|---|---|
| Asn | Asparagine |
| ADCC | Antibody dependent cell cytotoxicity |
| CTI | Cell time integral |
| CDC | Complement dependent cytotoxicity |
| CMP | Cytidine monophosphate |
| CTP | Cytidine triphosphate |
| GDP | Guanosine diphosphate |
| GTP | Guanosine triphosphate |
| ICP-MS | Inductively coupled plasma-mass spectrometry |
| LDH | Lactate dehydrogenase |
| UDP | Uridine diphosphate |
| UTP | Uridine triphosphate |
| mAb | monoclonal antibody |
| PSB | Primary Seed Bank |
| PSE | Pseudo standard-error |
| RSME | Design standard-error |
| Fuc | L-Fucose |
| Gal | D-Galactose |
| GlcNAc | N-acetylglucosamine |
| NANA | N-acetylneuraminic acid |
| Man | D-Mannose |
| Man5 | $GlcNAc_2 Man_5$ |
| Man6 | $GlcNAc_2 Man_6$ |
| High Mannose | $GlcNAc_2 Man_{5-8}$ |
| Core | $GlcNAc_2 Man_3$ |
| G0 | $GlcNAc Fuc GlcNAc Man_3 GlcNAc_2$ |
| G0-F | $GlcNAc GlcNAc Man_3 GlcNAc_2$ |
| G1 | $GlcNAc Fuc GlcNAc Man_3 GlcNAc_2 Gal$ |
| G1-F | $GlcNAc GlcNAc Man_3 GlcNAc_2 Gal$ |
| G2 | $GlcNAc Fuc GlcNAc Man_3 GlcNAc_2 Gal_2$ |
| G2 1SA | $GlcNAc Fuc GlcNAc Man_3 GlcNAc_2 Gal_2 NANA_1$ |
| Complex | $GlcNAc Fuc GlcNAc Man_3 GlcNAc_2 Gal_{0-2}$ |
| Complex-F | $GlcNAc GlcNAc Man_3 GlcNAc_2 Gal_{0-2}$ |

DETAILED DESCRIPTION

Described are methods and media for production of a recombinant glycoprotein under fermentation culture conditions in a eukaryotic cell, the method comprising adjusting the concentrations of each of iron, copper, zinc and manganese in the culture medium during the culture to affect biomass generation and/or N-glycan maturity of the expressed glycoprotein.

In one aspect, the method of the invention comprises adjusting the concentrations of each of iron, copper, zinc and manganese in the culture medium to favour biomass generation. As a result of this, there will be an increase in the quantity or weight of cultured cells in the culture medium. Biomass may be measured by determining viable cell density, total cell density, cell time integral (for viable and total cell density), cell volume time integral (for viable and total cell density), packed cell volume, dry weight or wet weight using techniques that are known in the art.

The method of the present invention, in one aspect, comprises adjusting the concentrations of each of iron, copper, zinc and manganese, or of only zinc and manganese, in the culture medium to affect N-glycan maturity in the expressed glycoprotein. N-glycan maturity refers to the pattern of glycosylation, such that the glycoprotein will either contain all, substantially all or less than all of the genetically intended glycan residues, that is the glycan residues added by endogenous genetically encoded glycoenzymes.

In a preferred embodiment, the adjustment of the concentrations of each of iron, copper, zinc and manganese, or of only zinc and manganese, in the culture medium would be to increase maturity in expressed N-glycoproteins. Examples of the carbohydrate pattern in such expressed glycoproteins are: GlcNAc Fuc GlcNAc $Man_3$ $GlcNAc_2$ (G0); GlcNAc Fuc GlcNAc $Man_3$ $GlcNAc_2$ Gal (G1) and GlcNAc Fuc GlcNAc $Man_3$ $GlcNAc_2$ $Gal_2$ (G2). Also included here are mature non-fucosylated glycoproteins, such as GlcNAc GlcNAc $Man_3$ $GlcNAc_2$ (G0-F) and GlcNAc GlcNAc $Man_3$ $GlcNAc_2$ $Gal_{0-2}$ (G1-F, G2-F). In a particularly preferred embodiment, the favouring would be for glycoproteins with a G0, G1 and/or a G2 carbohydrate structure.

In an alternative preferred embodiment, the adjustment of the concentrations of each of iron, copper, zinc and manganese in the culture medium would be to affect production of glycoproteins which are immature non-fucosylated glycoproteins. Examples of the glycosylation pattern in such immature non-fucosylated glycoproteins are high mannose glycoproteins, i.e. those containing $Man_5$, $Man_6$ and $Man_7$, such as: $GlcNAc_2$ $Man_{5-8}$, e.g. $GlcNAc_2$ $Man_5$ and $GlcNAc_2$ $Man_6$.

The present invention involves the production of a recombinant glycoprotein. There is no limitation on the nature of the glycoprotein, as long as it can be produced by fermentation culture and expressed in a eukaryotic cell. Glycoproteins suitable for production by this method include secreted and membrane-bound glycoproteins and/or glycoproteins which may be exogenous or endogenous to the eukaryotic cell, including, for example, structural glycoproteins, hormones, antibodies, enzymes and the like.

In a preferred embodiment of the invention, the glycoprotein is an antibody, typically a therapeutic or diagnostic antibody, and in a further embodiment, the antibody is a chimeric, humanized or human antibody.

When the glycoprotein is an antibody, the antibody could be a therapeutically effective antibody and may bind to any protein, including a member of the angiopoietin family, such as Ang1, Ang2, Ang3 and Ang4 and antibodies bi-specific for a member of the angiopoietin family and e.g. VEGF, such as Ang2/VEGF; a member of the HER receptor family, such as HER1 (EGFR), HER2, HER3 and HER4; CD proteins such as CD3, CD4, CD8, CD18, CD19, CD20, CD21, CD22, CD25, CD33, CD34, CD38, CD40, CD44 and CD52; cell adhesion molecules, such as LFA-1, VLA04, ICAM-1, VCAM and an integrin, including either $\alpha$ or $\beta$ subunits thereof (e.g. anti-CD11a, anti-CD18 or anti-CD11b antibodies); growth factors such as vascular endothelial growth factor (VEGF); cytokine receptors such as thymic stromal lymphopoietin receptor (TSLP-R); IgE; blood group antigens; flk2/flt3 receptor; obesity (OB) receptor and protein C. Other exemplary proteins include growth hormone (GH), including human growth hormone (hGH) and bovine growth hormone (bGH); growth hormone releasing factor; parathyroid hormone, thyroid stimulating hormone; lipoproteins; $\alpha$-1-antitrypsin; insulin A chain; insulin B chain; proinsulin, follicle stimulating hormone; calcitonin; luteinizing hormone; glucagon; clotting factors such as factor VIIIC; tissue factor (TF); von Willebrands factor; atrial natriuretic factor; lung surfactant; a plasminogen activator such as urokinase or tissue-type plasminogen activator (t-PA), bombazine, thrombin, tumour necrosis factor-a and -$\beta$; enkephalinase; RANTES (regulated on activation normally T-cell expressed and secreted); human macrophage inflammatory protein (MIP-1-$\alpha$); serum albumin such as human serum albumin (HSA); mullerian-inhibiting substance; relaxin A-chain, relaxin B-chain; prorelaxin; mouse gonadotropin-associated peptide; DNase; inhibin; activin; receptors for hormones or growth factors; protein A or D; fibroblast activation protein (FAP); carcinoembryonic antigen (CEA); rheumatoid factors; a neurotrophic factor such as bone-derived neurotrophic factor (BDNF); neurotrophin-3, -4, -5 or -6 (NT-3, NT-4, NT-5 or NT-6) or a nerve growth factor such as NGF-$\beta$; platelet-derived growth factor (PDGF); fibroblast growth factor such as aFGF and bFGF; epidermal growth factor (EGF) and epidermal growth factor receptor (EGFR); transforming growth factor (TGF) such as TGF-$\alpha$ and TGF-$\beta$, including TGF-$\beta$1, TGF-$\beta$2, TGF-$\beta$3, TGF-$\beta$4 or TGF-$\beta$5; insulin-like growth factor-I and -II (IGF-I and IGF-II); des(1-3)-IGF-I (brain IGF-I); insulin-like growth factor binding proteins (IGFBPs); erythropoietin (EPO); thrombopoietin (TPO); osteo-inductive factors; immunotoxins; a bone morphogenetic protein (BMP); an interferon (interferon-a, -β or -γ); colony stimulating factors (CSFs) e.g. M-CSF, GM-CSF and G-CSF; interleukins (ILs), e.g. IL-1 to IL-10 and IL-17; superoxide dismutase; T-cell receptors; BlyS (Br3) receptor; Br3-Fc immunoadhesin; Apo-2 receptor; Fc receptor; surface membrane proteins; decay accelerating factor (DAF); a viral antigen, such as for example a portion of the AIDS envelope; transport proteins; homing receptors; addressins; regulatory proteins; immunoadhesins; and biologically active fragments or variants of any of the above. Alternatively, the antibody could be an antibody directed against breast epithelial cells or binding to colon carcinoma cells, anti-EpCAM antibodies, anti-GpIIb/IIIa antibodies, anti-RSV antibodies, anti-CMV antibodies, anti-HIV antibodies, anti-hepatitis antibodies, anti-CA 125 antibodies, anti-$\alpha_v\beta_3$ antibodies, anti-human renal cell carcinoma antibodies, anti-human 17-1A antibodies, anti-human colorectal tumour antibodies, anti-human melanoma antibody R24 directed against GD3 ganglioside, anti-human squamous-cell carcinoma, anti-human leukocyte antigen (HLA) antibodies, anti-HLA DR antibodies.

According to the method of the present invention, the recombinant glycoprotein is produced in a eukaryotic cell. Any eukaryotic cell susceptible to cell culture and to expression of glycoproteins may be used in accordance with the present invention. The eukaryotic cell is preferably a eukaryotic cell line which is capable of growth and survival when placed in suspension culture in a medium containing the appropriate nutrients and growth factors and which is typically capable of expressing and secreting large quantities of a particular glycoprotein of interest into the culture medium.

In a preferred embodiment, the eukaryotic cell is a mammalian cell, a yeast cell or an insect cell.

When the eukaryotic cell is a mammalian cell, this may be, for example, an NSO murine myeloma cell line, a monkey kidney CVI line transformed by SV40 (COS-7, ATCC® CRL 1651); human embryonic kidney line 293S (Graham et al., J. Gen. Virol. 36 (1977) 59); baby hamster kidney cells (BHK, ATCC® CCL 10); mouse sertoli cells (TM4, Mather, Biol. Reprod. 23 (1980) 243); monkey kidney cells (CVI-76, ATCC® CCL 70); African green monkey kidney cells (VERO-76, ATCC® CRL 1587); human cervical carcinoma cells (HELA, ATCC® CCL 2); canine kidney cells (MDCK, ATCC® CCL 34); buffalo rat liver cells (BRL 3A, ATCC® CRL 1442); human lung cells (W138, ATCC® CCL 75); human liver cells (Hep G2, HB 8065); mouse mammary tumour cells (MMT 060562, ATCC® CCL 5I); rat hepatoma cells (HTC, MI.54, Baumann et al., J. Cell Biol., 85 (1980) 1); and TR-1 cells (Mather et al., Annals N.Y. Acad. Sci. 383 (1982) 44), the PER.C6 cell line (Percivia LLC) and hybridoma cell lines.

Chinese Hamster Ovary cells (CHO, Urlaub and Chasin P. N. A. S. 77 (1980) 4216) or PER.C6 are preferred cell lines for practicing this invention. Known CHO derivatives suitable for use herein include, for example CHO/-DHFR (Urlab & Chasin, supra), CHOK1SV (Lonza), CHO-K1 DUC B11 (Simonsen and Levinson P. N. A. S. 80 (1983) 2495-2499) and DP12 CHO cells (EP 307,247) and CHO DG44 cells (Derouazi et al., Biochem. Biophys. Res. Commun. 340 (2006) 1069-77).

When the eukaryotic cell is a yeast cell, this may be, for example, *Saccharomyces cerevisiae* or *Pichia pastoris.*

When the eukaryotic cell is an insect cell this may be, for example, Sf-9.

The eukaryotic cell used in the present invention may be a glycoengineered cell, which cell has been engineered in order to modify its glycosylation profile. Such engineering includes, for example, either the knocking-out or knocking-in of genes relevant to synthesis of N-glycans.

It is most preferred that in the present invention, the cell is a CHO cell, optionally a glycoengineered CHO cell.

The eukaryotic cell used in the present invention is selected or manipulated to produce recombinant glycoprotein. Manipulation includes one or more genetic modifications such as introduction of one or more heterologous genes encoding the glycoprotein to be expressed.

The heterologous gene may encode a glycoprotein either that is normally expressed in that cell or that is foreign to the host cell. Manipulation may additionally or alternatively be to up- or down-regulate one or more endogenous genes. Often, cells are manipulated to produce recombinant glycoprotein by, for example, introduction of a gene encoding the glycoprotein and/or by introduction of control elements that regulate expression of the gene encoding the glycoprotein of interest. Genes encoding recombinant glycoproteins and/or control elements may be introduced into the host cell via vectors, such as a plasmid, phage or viral vector. Certain vectors are capable of autonomous replication in a host cell into which they are introduced whilst other vectors can be integrated into the genome of a host cell and are thereby replicated along with the host genome. Various vectors are publicly available and the precise nature of the vectors is not essential to the present invention. Typically vector components include one or more of a signal sequence, an origin of replication, one or more marker genes, a promoter and a transcription termination sequence. Such components are as described in WO 97/25428.

Biomass generation and glycoprotein expression from eukaryotic cells is achieved according to the method of the invention by culture of the cells under fermentation conditions. Any fermentation cell culture method or system that is amenable to the growth of the cells for biomass generation and expression of glycoproteins may be used with the present invention. For example, the cells may be grown in batch, fed-batch or split-batch cultures, where the culture is terminated after sufficient expression of the glycoprotein has occurred, after which the glycoprotein is harvested and, if required, purified. If a fed-batch culture is used, feeding of the culture may take place once or more than once during culture. When multiple feeds are given, these may be with the same or different feeding solutions. In the alternative, the cells may be grown in perfusion cultures, where the culture is not terminated and new nutrients and components are added periodically or continuously to the culture and expressed glycoprotein is removed, either periodically or continuously. In a preferred embodiment, the cell culture method used in the present invention is fed-batch or split-batch or a combination of the two.

Reactors, temperatures and other conditions for fermentation culture of cells for biomass generation and the production of glycoproteins, such as oxygen concentration and pH are known in the art. Any conditions appropriate for culture of the selected eukaryotic cell can be chosen using information available in the art. The culture conditions, such as temperature, pH and the like, are typically those previously used with the host cell selected for expression and will be apparent to the person skilled in the art. If desired, the temperature and/or the pH and/or $CO_2$ could be altered during cultivation in order to increase yield and/or increase the relative amount of the desired glycoprotein quality.

The medium in which the cells are cultured and in which the concentrations of the trace elements iron, copper, zinc and manganese are adjusted according to the method of the present invention can be any of a wide variety known in the art. If desired, the medium could be a chemically defined medium where the components of the medium are known and controlled, or the medium could be a complex medium in which not all of the components are known and/or controlled.

Chemically defined media have been extensively developed and published in recent history, including such media for culture of mammalian cells. All components of defined media are well characterized and such media do not contain complex additives such as serum and hydrolysates. Typically these media include defined quantities of purified growth factors, proteins, lipoproteins and other substances which may otherwise be provided by serum or extract supplement. Such media have been produced with the sole purpose of supporting highly productive cell cultures. Certain defined media may be termed low protein media or may be protein free if the typical components of low protein media, insulin and transferrin, are not included. Serum free media may otherwise be used in the methods of the present invention. Such media normally do not contain serum or protein fractions, but may contain undefined components.

Examples of commercially available culture media include Ham's F10 (Sigma), Minimal Essential Medium (MEM, Sigma), RPMI-1640 (Sigma) and Dulbecco's Modified Eagle's Medium (DMEM, Sigma) and chemically defined media and feed supplements sold by Life Technologies. Any such media may be supplemented as necessary with hormones and/or other growth factors (such as insulin, transferrin or epidermal growth factor); salts (such as sodium chloride, calcium, magnesium and phosphate), buffers (such as HEPES); nucleosides (such as adenosine and thymidine), antibiotics (such as GENTAMYCIN™), and glucose or an equivalent energy source.

The necessary nutrients and growth factors for the medium including their concentrations, for a particular cell line, are determined empirically and without undue experimentation as described in, for example, Mammalian Cell Culture, Mather (Plenum Press: NY 1984); Barnes and Sato, Cell 22 (1980) 649 or Mammalian Cell Biotechnology: A Practical Approach M. Butler (IRL Press, 1991). A suitable medium contains a basal medium component, such as DMEM/HAM F12-based formulation with modified concentrations of some components, such as amino acids, salts, sugar and vitamins, and optionally containing glycine, hypoxanthine, thymidine, recombinant human insulin, hydrolyzed peptone, such as PRIMATONE HS™ or PRIMATONE RL™ (Sheffield, England) or the equivalent, a cell protective agent, such as PLURONIC F68™ or the equivalent pluronic polyol and GENTAMYCIN™.

In the following, Table 1 shows the differing amounts of the trace elements iron, copper, zinc and manganese in different commercially available cell culture media, any of which may be used for cell culture.

Table 1: Calculated Concentrations of Iron, Copper, Zinc and Manganese in Commercial Media

|  | DMEM/F12 | DMEM | HamF12 | HamF10 | RPMI | RPMI1640 | 199 |
|---|---|---|---|---|---|---|---|
| μM | | | | | | | |
| $Cu^{2+}$ | 0.0052 | 0.0000 | 0.0100 | 0.0100 | 0.0000 | 0.0050 | 0.0000 |
| $Fe^{3+}$ | 0.1240 | 0.2480 | 0.0000 | 0.0000 | 0.0000 | 0.0000 | 1.7822 |
| $Fe^{2+}$ | 1.5000 | 0.0000 | 3.0000 | 3.0000 | 0.0000 | 0.0000 | 0.0000 |
| $Zn^{2+}$ | 1.5000 | 0.0000 | 3.0000 | 0.1000 | 0.0000 | 3.0300 | 0.0000 |
| $Mn^{2+}$ | 0.0000 | 0.0000 | 0.0000 | 0.0000 | 0.0000 | 0.0003 | 0.0000 |
| RPMI1640 and 199 are Advanced Media | | | | | | | |

The concentrations of iron, copper, zinc and manganese calculated to be in commercial media, as above, will change if those media are supplemented with complex ingredients, such as serum or peptones.

When a method of the invention comprises adjusting the concentrations of iron, copper, zinc and manganese in the culture medium by adding those trace elements to the medium, or a medium comprises adjusted concentrations of those elements, the trace elements can be added to or are present in the medium in the form of a metal salt. Any iron, copper, zinc and manganese salt appropriate for inclusion in a culture medium for the production of a recombinant glycoprotein can be used. It is generally preferred that the metal salt is in the form of the appropriate metal sulphate, halide, oxide, nitrate, citrate, acetate or phosphate, in hydrated or anhydrous form, or that the metal ion is bound to a chelator such as transferrin or lactoferrin.

Typically iron (II) and (III) salts appropriate for use in the present invention include Fe(III)-citrate, $FeSO_4$, $FeCl_2$, $FeCl_3$, $Fe(NO_3)_3$ and $FePO_4$ as well as iron bound to transferrin or lactoferrin.

Typically copper (II) salts appropriate for use in the present invention include $CuSO_4$, $CuCl_2$, and Cu-acetate.

Typically zinc (II) salts appropriate for use in the present invention include $ZnSO_4$ and $ZnCl_2$.

Typically manganese salts appropriate for use in the present invention include $MnSO_4$, $MnCl_2$, $MnF_2$ and $MnI_2$.

The present invention provides cell culture under fermentation culture conditions. This is typically a multi-step culture procedure where the cells are cultivated in a number of steps or phases. According to this preferred procedure, the fermentation culture process, e.g. from frozen vials of cells, typically covers three distinct phases, as depicted in Figure 1, i.e.:

i) the seed train, for recovery of the cells after the stress of thawing and to normalize cell doubling times, which can last between 14 and e.g. more than 60 days, depending on the speed of cell recovery and the scale of production. In Figure 1 this is depicted as lasting 21 days;

ii) the growth phase, or inoculation train, called n-x phases (n is the production phase), wherein x is typically 1 to 5, preferably 1 or 2. In Figure 1, an n-1 and an n-2 phase are illustrated. These phases may also be referred to as a growth phase(s) wherein cells are inoculated into a medium suitable for promoting growth and biomass generation. Thus, the n-x phases are typically for the expansion of the culture for larger cultivation formats and the wash-out of the selected compound. When the n-x phases consist of an n-1 and n-2 phase, each of the n-1 and n-2 stages takes e.g. from 2 to 7 days, typically each lasting 3 or 4 days; and

iii) the production phase, or n-phase, for the production of the recombinant glycoprotein in appropriate quantity and/or quality. The duration of this phase may depend on, for example, the nature of the recombinant cell as well as the quantity and/or quality of the expressed glycoprotein. Typically this phase will last between about 11 and about 20 days. In Figure 1, this phase is depicted as lasting 14 days.

The cells may be maintained in the seed train or in the growth phase for a suitable period of time by, e.g. the addition of fresh medium or nutrient supplementation to existing medium as appropriate.

Typically, the production or n-phase is characterized by two distinct phases, as depicted in Figure 2. The first of these is a growth phase, for generation of sufficient viable biomass for cell specific protein production and the second of which is for generation of the recombinant glycoprotein, with no significant cell growth.

Any or all of the seed train, the growth phase and production phase may be continuous, or the cells from one phase may be used to inoculate the next phase.

Recovery of the expressed glycoprotein either during or at the end of a culture period, preferably the production phase, can be achieved using methods known in the art. The glycoprotein is preferably recovered from the culture medium as a secreted polypeptide, although it may be recovered from host cell lysates when directly produced without a secretory signal. If the glycoprotein is membrane-bound, it can be released from the membrane using a suitable detergent solution (e.g. Triton-X 100) or its extracellular region may be released by enzymatic cleavage. The expressed glycoprotein may be isolated and/or purified as necessary using techniques known in the art.

In the method of the present invention, the concentrations of iron, copper, zinc and manganese are adjusted in any or all of the growth or production phases of the fermentation process. In a preferred embodiment, the concentrations of iron, copper, zinc and manganese are adjusted at the start of or during the growth phase and/or at the start of or during the production phase. It is most preferred that the concentrations of iron, copper, zinc and manganese are adjusted at the start of the growth phase and at the start of the production phase or that concentrations of iron, copper, zinc and manganese are adjusted at any or all of the start of the growth phase, during the growth phase, at the start of the production phase and during the production phase.

Concentrations of iron, copper, zinc and manganese are adjusted by increasing or decreasing the concentration of those trace elements in the culture medium. In the present invention, when the concentration of those elements is increased or decreased, this increase or decrease in concentration is relative to the concentration of those elements in the medium in the culture phase immediately preceding the increase or decrease. Thus, if there is an increase in the concentration of, for example, each of iron, copper, zinc and manganese in the medium at the start of the production phase, this is an increase in the concentration of those trace elements over the concentration of those trace elements in the medium of the immediately preceding growth phase. Similarly, if there is to be an increase in the concentration of, for example, any or all of iron, copper, zinc and manganese in the medium during the production phase, this is an increase in the concentration of those trace elements over the concentration of those elements in the medium of the immediately preceding part of the production phase. Similarly, if there is to be a decrease in the concentration of, for example, any or all of iron, copper, zinc and manganese in the medium at the start of or during any of the growth or production phases, this is a decrease in the concentration of those trace elements over the concentration of those trace elements in the medium of the immediately preceding culture phase. Similarly, if there is to be a decrease in the concentration of, for example, iron and copper and an increase in the concentration of, for example, zinc and manganese in the medium at the start of or during the production phase, this is a decrease in the concentration of iron and copper and an increase in the concentration of zinc and manganese as compared to the concentrations of those trace elements in the medium of the immediately preceding culture phase.

It is generally preferred that when the concentrations of any or all of iron, copper, zinc and manganese are adjusted in the culture medium, the concentrations of all of these trace elements are adjusted at the same time. However, the method of the present invention also includes adjustment of iron, copper, zinc and manganese individually, in pairs, or by adjusting three of these trace elements at one time and then the fourth, or vice versa. In particular, if the concentration of two of the trace elements is to increase and the concentration of two of the elements is to decrease, the adjustment to increase and decrease may take place at the same time or at different times. It is preferred, in that case, that the adjustment to increase the concentration of two trace elements and to decrease the concentration of two elements takes place at the same point in time or in the same medium.

In the method of the present invention, adjustment of the concentrations of iron, copper, zinc and manganese can be achieved by any technique appropriate to the fermentation conditions being used. The method by which the concentrations of these trace elements are adjusted is not essential to the present invention and appropriate methods are known in the art. Adjustment of trace element concentration can thus take place either by supplementing the medium (in the case of an increase in concentration of any or all of the trace elements) in which the cells are being cultured, or by transferring all or a portion of the cells (i.e. splitting) to a fresh medium containing the desired concentrations of the trace elements. A combination of these two methods may be used if required.

Adjustment of the concentration of iron, zinc, copper and manganese can therefore be continuous, over the whole or a portion of the culture period, or may be intermittent, for example as a reaction to the assumed, calculated or measured concentration of the trace element(s) in the culture medium. The invention defines adjustment of the concentration of the trace elements within ranges. If actual measurement or calculation of the concentration of each or all of the trace elements during a defined culture period, for example, during the growth or production phase, indicates that the concentration of each or all of the trace elements falls within the ranges recited herein, adjustment of that concentration may nonetheless take place, so long as the resulting concentration of the trace element remains within the range recited.

If required, known techniques can be used to measure the actual concentrations of the trace elements in the culture medium before the adjustments are made. These include online and offline analyses of the respective trace element concentration.

Thus, if batch fermentation conditions are being used, achieving an increase in the concentrations of the trace elements may be by, for example, seeding into a fresh medium containing or supplemented with concentrations of the appropriate trace elements that are increased over the existing culture medium, or by splitting the cells into a medium containing or supplemented with the increased concentrations of the appropriate trace elements over the existing medium. If fed-batch fermentation conditions are being used, achieving an increase in the concentrations of the trace elements may be by, for example, seeding into a fresh medium containing or supplemented with the increased concentrations of the appropriate trace elements, giving one or more bolus or continuous feeds of the appropriate trace elements to the culture medium; by determining a feed rate based on cell number or calculated according to known metabolic models, metabolic surrogate markers etc, or by splitting the culture into a medium which contains or has been supplemented with the increased concentrations of the appropriate trace elements. If bolus or continuous feed is being added, this may contain other nutrients/components required for the culture in addition to any or all of the iron, copper, zinc and manganese. If perfusion fermentation conditions are being used, achieving an increase in the concentrations of the trace elements may be achieved by, for example, a continual or intermittent addition of trace elements to the reactor either at the same time or separately to other nutrients/components being added to the perfusion culture.

If a decrease in the concentrations of any or all of iron, zinc, copper and manganese is required, this may be achieved by seeding the cells into a fresh medium in which the concentrations of the trace elements is decreased in comparison to the concentration of those trace elements in the medium of the immediately preceding culture phase. Alternatively, or in addition, a decrease in the concentrations of any or all of iron, zinc, copper or manganese can be achieved in an existing or fresh medium by complexation of the metal ion, for example by addition of a metal ion chelator to the culture and washing out of the complexed metal ion. Any metal ion chelator capable of complexing the appropriate metal ion to result in a decrease in the bioavailable concentration of that metal ion in the culture medium can be used. Suitable such chelators for iron and copper include, for example, small molecules or metal binding proteins, such as, for example, EDTA, EGTA, siderophores such as desferrioxamine, desferrithiocin, tetracycline, quinolone, phosphonates, polyphenols, proteins such as transferrin or ceruloplamin, polysaccharides, organic acids such as maleate, suprizone, bathocuproine sulphonate, bathophenanthroline sulphonate, and D-pencillamine. In addition, the effect of a decrease of the concentration of the trace elements could be mimicked by inhibition/activation of glycosylation enzymes by alternative trace elements, by targeted competition with cellular transporters or by targeted modulation of cellular transporter activity.

The specific values of decreased or increased concentrations of a metal are based either on actual measurements of the metal in the culture medium or on theoretical concentrations or calculations of the concentration of the metal in the culture solution surrounding the cells. The practicioner will appreciate that some concentration of the trace elements, introduced for example via impurities and leaching may be present and will take these into account when calculating a decreased or increased concentration of the trace elements in accordance with the invention.

In the methods of the present invention, it is preferred that a decrease in the concentration of any or all of the trace elements, as appropriate, is achieved by seeding the cells into a fresh medium in which the concentration of the trace elements is decreased in comparison to the concentration of those trace elements in the medium of the immediately preceding culture phase.

In certain embodiments of the present invention, the concentrations of iron, copper, zinc and manganese may be adjusted in the culture medium to favour growth of the cells, and thereby increase biomass. In other embodiments, the concentrations of iron, copper, zinc and manganese, or the concentrations of only zinc and manganese, may be adjusted in the culture medium to increase maturity in expressed N-glycoproteins and the production of glycoproteins with a mature glycosylation pattern. In another embodiment, the concentrations of iron, copper, zinc and manganese may be adjusted to favour increasing the production of immature non-fucosylated glycoproteins. In yet another embodiment, the concentrations of iron, copper, zinc and manganese in the culture medium may be adjusted first to enhance growth and then again to affect glycoprotein maturity, be that either increasing the maturity of expressed N-glycosylated glycoproteins or increasing the production of immature non-fucosylated glycoproteins.

In the method of the present invention, applying different trace element concentrations at the start or during the growth and/or production phases can cause a direct effect on biomass generation and N-glycosylation maturity in the expressed glycoprotein as illustrated in Table 2, below. Adjustment of the concentrations of iron, copper, zinc and manganese during these culture phases results in a switch between biomass generation and N-glycosylation maturity in expressed glycoproteins. "Afucosylation" in Table 2 is the same as non-fucosylation.

## Table 2: Process Strategies for Targeted Protein Production

| (n – 2)<br>Inoculum Train<br>Medium | (n – 1)<br>Inoculum Train<br>Medium | (n)<br>Production Medium |
|---|---|---|

A | ---- Growth ----→ | ---- Galactosylation ----→ |

B | ---- Growth ----→ | ---- Growth ----→ | ---- Galactosylation ----→ |

C | ---- Growth ----→ | ---- Afucosylation ----→ |

D | ---- Growth ----→ | ---- Growth ----→ | ---- Afucosylation ----→ |

E | ---- Growth ----→ | ---- Growth ----→ |

F | ---- Galactosylation ----→ | ---- Galactosylation ----→ |

G | ---- Afucosylation ----→ | ---- Afucosylation ----→ |

As a result of the method of the present invention, the glycoprotein producing cells are cultured in a medium which is tailored to the desired end result. Thus, as illustrated in Table 3, below, a growth medium primarily promotes biomass generation, a galactosylation medium primarily promotes an increase in maturity of expressed N-glycoproteins and production of mature glycoproteins, and a non-fucosylation medium primarily promotes the production of immature non-fucosylated glycoproteins. There may be some inter-related effects of each medium such that the other results may be affected to a certain degree, as shown below, but the desired end result is the one favoured.

### Table 3: Relationship between Tailored Media and Desired Outcome

| Medium / Support | Growth | Galactosylation | Non-fucosylation |
|---|---|---|---|
| Biomass Generation | +++ | + | ++ |
| Protein Galactosylation | + | +++ | - |
| Protein Non-fucosylation | ++ | - | +++ |

In a preferred embodiment, the present invention provides increasing the concentration of each of iron, copper, zinc and manganese in the culture medium to favour cell growth/ biomass generation. In this embodiment, the concentration of each of iron, copper, zinc and manganese is increased at the start of and/or during the growth phase and, optionally, also at the start of and/or during the production phase. This embodiment is particularly suitable for cells with low growth capacity, cell lines where biomass and productivity are coupled and if low levels of mature galactosylated glycoprotein are desired.

In an alternative preferred embodiment, the present invention provides increasing the concentration of each of zinc and manganese and, optionally, reducing the concentration of iron and copper in the culture medium to increase maturity in expressed N-glycoproteins, suitably production of glycoproteins with a mature glycosylation pattern and thereby to decrease production of immature glycoproteins, including immature non-fucosylated glycoproteins. In this embodiment, the concentration of each of iron, copper, zinc and manganese or of only zinc and manganese is adjusted at the start of the growth phase and/or at the start of and/or during the production phase. Favouring increased maturity of glycoproteins and production of glycoproteins with a mature glycosylation pattern by adjustment of the concentrations of the trace elements during the growth and production phases is best where low cell growth is desirable, and particularly where the expressed glycoprotein is complex with many unexpected side products. Favouring increased maturity of glycoproteins and production of glycoproteins with a mature glycosylation pattern by adjustment of the concentrations of the trace elements in the medium at the start of the production phase is particularly useful when good growth has been achieved during the growth phase. Favouring increased maturity of glycoproteins and production of glycoproteins with a mature glycosylation pattern by adjustment of the concentrations of the trace elements in the medium during the production phase is particularly suitable when the concentrations of the trace elements have been adjusted for the growth phase and at the start of the production phase to ensure good biomass generation.

In an alternative preferred embodiment, the present invention provides decreasing the concentrations of each of iron, copper, zinc and manganese in the culture medium to favour production of immature non-fucosylated glycoproteins. In this embodiment, the concentration of each of iron, copper, zinc and manganese is adjusted at the start of the growth phase and/or at the start of and/or during the production phase. Favouring production of immature non-fucosylated glycoproteins by adjustment of the concentrations of the trace elements at the start of the growth and production phases is best where cell growth is good. Favouring production of immature non-fucosylated glycoproteins by adjustment of the concentrations of the trace elements at the start of the production phase is particularly useful when good growth has been achieved during the growth phase. Favouring production of immature non-fucosylated glycoproteins by adjustment of the concentrations of the trace elements in the medium during the production phase is particularly suitable when the concentrations of the trace elements have been adjusted for the growth phase and at the start of the production phase to ensure good biomass generation.

In an alternative preferred embodiment, the present invention provides increasing the concentration of each of iron, copper, zinc and manganese in the culture medium at the start of the growth phase and, optionally at the start of the production phase, to favour cell growth/biomass generation and additionally, increasing the concentration of each of zinc and manganese and reducing the concentration of iron and copper in the culture medium at the start of or during the production phase to increase maturity of glycoproteins, for example to favour production of glycoproteins with a mature N-glycosylation pattern over the production of immature glycoproteins, including immature non-fucosylated glycoproteins. In this embodiment, when the concentrations of iron, copper, zinc and manganese are increased at the start of the production phase to favour biomass generation, increasing maturity of glycoproteins, for example favouring production of glycoproteins with a mature N-glycosylation pattern takes place by further adjustment of the concentrations of those trace elements during the production phase.

In an alternative preferred embodiment, the present invention provides increasing the concentration of each of iron, copper, zinc and manganese in the culture medium at the start of the growth phase and, optionally at the start of the production phase to favour cell growth/biomass generation and additionally decreasing the concentrations of each of iron, copper, zinc and manganese in the culture medium at the start of or during the production phase to favour production of immature non-fucosylated glycoproteins. In this embodiment, when the concentrations of iron, copper, zinc and manganese are increased at the start of the production phase to favour biomass generation, favouring production of immature non-fucosylated glycoproteins takes place by further adjustment of the concentrations of those trace elements during the production phase.

In a preferred embodiment of the present invention, it is preferred that the concentration of iron, as $Fe^{2+}$ or $Fe^{3+}$, in the culture medium to favour biomass generation is adjusted to between 15 $\mu$M to more than 80 $\mu$M, preferably 20 $\mu$M to more than 60 $\mu$M and most preferably 25 $\mu$M to more than 50 $\mu$M.

In a preferred embodiment of the present invention, it is preferred that the concentration of iron, as $Fe^{2+}$ or $Fe^{3+}$, in the culture medium to increase maturity in expressed N-glycoproteins, e.g. to favour production of mature N-glycosylated glycoprotein species is adjusted to between 0 $\mu$M to 25 $\mu$M, preferably 0 $\mu$M to 20 $\mu$M and most preferably 0 $\mu$M to 16 $\mu$M.

In a preferred embodiment of the present invention, it is preferred that the concentration of iron, as $Fe^{2+}$ or $Fe^{3+}$, in the culture medium to favour production of immature non-fucosylated glycoprotein species is adjusted either to between 0 $\mu$M to 35 $\mu$M, preferably 0 $\mu$M to 30 $\mu$M and most preferably 0 $\mu$M to 25 $\mu$M or to between 15 $\mu$M to more than 80 $\mu$M, preferably 20 $\mu$M to more than 60 $\mu$M and most preferably 25 $\mu$M to more than 50 $\mu$M.

In a preferred embodiment of the present invention, it is preferred that the concentration of copper, as $Cu^{2+}$, in the culture medium to favour biomass generation is adjusted to between 0.3 $\mu$M to more than 2.5 $\mu$M, preferably 0.5 $\mu$M to more than 1.5 $\mu$M and most preferably 0.3 $\mu$M to more than 1 $\mu$M.

In a preferred embodiment of the present invention, it is preferred that the concentration of copper, as $Cu^{2+}$, in the culture medium to increase maturity in expressed N-glycoproteins, e.g. to favour production of mature N-glycosylated glycoprotein species is adjusted to between 0 $\mu$M and 0.1 $\mu$M, preferably 0 $\mu$M to 0.08 $\mu$M and most preferably 0 $\mu$M to 0.06 $\mu$M.

In a preferred embodiment of the present invention, it is preferred that the concentration of copper, as $Cu^{2+}$, in the culture medium to favour production of immature non-fucosylated glycoprotein species is adjusted either to between 0 $\mu$M to 1 $\mu$M, preferably 0 $\mu$M to 0.5 $\mu$M and most preferably 0 $\mu$M to 0.3 $\mu$M or to between 0.3 $\mu$M to more than 2.5 $\mu$M, preferably 0.5 $\mu$M to more than 1.5 $\mu$M and most preferably 0.3 $\mu$M to more than 1 $\mu$M.

In a preferred embodiment of the present invention, it is preferred that the concentration of zinc, as $Zn^{2+}$, in the culture medium to favour biomass generation is adjusted to between 20 $\mu$M to more than 50 $\mu$M, preferably 25 $\mu$M to more than 45 $\mu$M and most preferably 28 $\mu$M to more than 43 $\mu$M.

In a preferred embodiment of the present invention, it is preferred that the concentration of zinc, as $Zn^{2+}$, in the culture medium to increase maturity in expressed N-glycoproteins, e.g. to favour production of mature N-glycosylated glycoprotein species is adjusted to between 20 $\mu$M to more than 50 $\mu$M, preferably 25 $\mu$M to more than 45 $\mu$M and most preferably 28 $\mu$M to more than 43 $\mu$M.

In a preferred embodiment of the present invention, it is preferred that the concentration of zinc, as $Zn^{2+}$, in the culture medium to favour production of immature non-fucosylated glycoprotein species is adjusted to between 0 $\mu$M to 20 $\mu$M, preferably 0 $\mu$M to 15 $\mu$M and most preferably 0 $\mu$M to 13 $\mu$M.

In a preferred embodiment of the present invention, it is preferred that the concentration of manganese, as $Mn^{2+}$, in the culture medium to favour biomass generation is adjusted to between 0.01 $\mu$M to more than 3 $\mu$M, preferably 0.05 $\mu$M to more than 2 $\mu$M and most preferably 0.1 $\mu$M to more than 1 $\mu$M.

In a preferred embodiment of the present invention, it is preferred that the concentration of manganese, as $Mn^{2+}$, in the culture medium to increase maturity in expressed N-glycoproteins, e.g. to favour production of mature N-glycosylated glycoprotein species is adjusted to between 0.01 $\mu$M to more than 3 $\mu$M, preferably 0.05 $\mu$M to more than 2 $\mu$M and most preferably 0.1 $\mu$M to more than 1 $\mu$M.

In a preferred embodiment of the present invention, it is preferred that the concentration of manganese, as $Mn^{2+}$, in the culture medium to favour production of immature non-fucosylated glycoprotein species is adjusted to between 0 $\mu$M to 0.01 $\mu$M, preferably 0 $\mu$M to 0.05 $\mu$M and most preferably 0 $\mu$M to 0.07 $\mu$M.

Preferred concentrations of the combinations of iron, copper, zinc and manganese in the culture medium for the tailored results of the present invention are:

A. to favour biomass generation:

- (a) iron: from 15 μM to more than 80 μM;
- (b) copper: from 0.3 μM to more than 2.5 μM;
- (c) zinc: from 20 μM to more than 50 μM; and
- (d) manganese : from 0.01 μM to more than 3 μM.

B. to increase maturity in expressed N-glycoproteins, e.g. to favour production of mature N-glycosylated glycoproteins:

(i)

- (a) iron: from 0 μM to 25 μM;
- (b) copper: from 0 μM to 0.1 μM;
- (c) zinc: from 20 μM to more than 50 μM; and
- (d) manganese : from 0.01 μM to more than 3 μM; or

(ii)

- (a) zinc: from 20 μM to more than 50 μM; and
- (b) manganese : from 0.01 μM to more than 3 μM.

C. to favour production of immature non-fucosylated glycoproteins, either:

(i)

- (a) iron: from 0 μM to 35 μM;
- (b) copper: from 0 μM to 1 μM;
- (c) zinc: from 0 μM to 20 μM; and
- (d) manganese : from 0 μM to 0.01 μM; or

(ii)

- (a) iron: from 15 μM to more than 80 μM;
- (b) copper: from 0.3 μM to more than 2.5 μM;
- (c) zinc: from 0 μM to 20 μM; and
- (d) manganese : from 0 μM to 0.01 μM.

Particularly preferred combinations of the concentrations of iron, copper, zinc and manganese in the culture medium to favour biomass generation, increase maturity in expressed N-glycoproteins, e.g. production of glycoproteins with a mature N-glycosylation pattern, and/or to favour production of immature non-fucosylated glycoproteins are as shown in Table 4, below.

## Table 4: Trace Element Concentrations in Cultivation Phases

| Medium / Element | Growth (µM) | Galactosylation (µM) Option (i) | Galactosylation (µM) Option (ii) | Non-fucosylation (µM) Option (i) | Non-fucosylation (µM) Option (ii) |
|---|---|---|---|---|---|
| $Zn^{2+}$ | 28.295 – > 43.301 | 28.295 – > 43.301 | 28.295 – > 43.301 | 0.000 – 13.900 | 0.000 – 13.900 |
| $Mn^{2+}$ | 0.110 – > 1.000 | 0.110 – > 1.000 | 0.110 – > 1.000 | 0.000 – 0.075 | 0.000 – 0.075 |
| $Fe^{2+}/Fe^{3+}$ | 25.472 – > 50.000 | 0.000 – 16.055 | n/a | 0.000 – 25.472 | 25.472 – >50.000 |
| $Cu^{2+}$ | 0.329 – > 1.000 | 0.000 – 0.064 | n/a | 0.000 – 0.329 | 0.329 – > 1.000 |

(n/a = not applicable)

According to the methods of the present invention, increasing the concentration levels of iron, copper, zinc and manganese in the medium to favour, promote or increase biomass generation during culture under fermentation conditions to produce a recombinant glycoprotein will result in an increase in biomass of at least 5%, preferably at least 10%, 15%, 20%, 25%, 30%, 40%, 45%, 50%, 55% or 60% over comparable culture in which the concentration levels of iron, copper, zinc and manganese are not adjusted. Methods for measuring and quantifying an increase in cell growth are known in the art. Typically this can be done by analyzing the maximum viable cell density and final cell time integral.

As is apparent to the person skilled in the art, in the production of a recombinant glycoprotein, an increase in biomass during one stage of cell culture will result in an increase in the amount of glycoprotein produced during a later stage of cell culture.

According to the methods of the present invention, targeted adjustment of the concentration levels of iron, copper, zinc and manganese, or of zinc and manganese only, in the medium during culture under fermentation conditions to increase maturity in expressed N-glycoproteins, e.g. to favour, promote or increase production of glycoproteins with a mature N-glycosylation pattern will result in an increase in production of mature N-glycosylated glycoproteins of at least 5%, preferably at least 10%, 15%, 20%, 25%, 30%, 40%, 45%, 50%, 55% or 60% over comparable culture in which the concentration levels of iron and copper, and/or zinc and manganese are not adjusted during culture.

According to the methods of the present invention, targeted adjustment of the concentration levels of iron, copper, zinc and manganese, or of zinc and manganese only, in the medium during culture under fermentation conditions to favour, promote or increase production of glycoproteins with a G0, G1 or G2 pattern will result in an increase in production of glycoproteins with a G0, G1 or G2 pattern of at least 5%, preferably at least 10%, 15%, 20%, 25%, 30%, 40%, 45%, 50%, 55% or 60% over comparable culture in which the concentration levels of iron, copper, zinc and manganese are not adjusted during culture.

According to the methods of the present invention, targeted adjustment of the concentration levels of iron, copper, zinc and manganese in the medium during culture under fermentation conditions to favour, promote or increase production of immature non-fucosylated glycoproteins will result in an increase in production of immature non-fucosylated glycoproteins of at least 5%, preferably at least 10%, 15%, 20%, 25%, 30%, 40%, 45%, 50%, 55% or 60% over comparable culture in which the concentration levels of iron, copper, zinc and manganese are not adjusted during culture.

The following examples illustrate the invention.

EXAMPLES

*Materials and chemical substances*

*Cell lines*

For the study described we used nine in-house generated recombinant CHO-K1 cell lines: clone 1 and clone 2, both expressing the same monoclonal antibody, clone 3, expressing another monoclonal antibody and clone A, clone B, clone C, clone D, clone E and clone F expressing a glycoengineered antibody. Compared to clone 1, clone 2 showed high levels of high-mannose glycan species (Man5). All clones were cultivated using proprietary chemically defined protein-free in-house medium and process platforms, referred to in the following as platform A and platform B.

*In process control - cell growth and analyses of metabolites*

Cell growth and viability were analyzed by using the trypan blue exclusion method (Strober, Curr. Protoc. Immunol. Appendix 3 (2001)) and an automated CedexHiRes device (Roche Innovatis, Bielefeld, Germany). For quantification of the metabolites lactate and ammonium, cell culture fluid was centrifuged to separate cells and analyzed using a Cobas Integra 400 plus system (Roche, Mannheim, Germany). Product titre was either quantified by a Cobas Integra 400 plus system (Roche, Mannheim, Germany) or by PorosA HPLC method as described by Zeck et al., (PLoS. One 7.7 (2012) e40328).

*In process control - trace elements*

Trace element concentrations were analyzed using cell-free cultivation supernatant and an in-house method for an ICP-MS system (Agilent, Böblingen, Germany).

*DoE Experiments*

Statistical experiment planning by DoE (*Design of Experiments*) is a powerful and well-known technique especially for biological and chemical reaction optimization where many factors interdependently influence the final result. For example, DoE was successfully used for optimization of cell culture media (Zhang et al., Cytotechnology 65.3 (2013) 363-78), fermentation processes (Fu et al., Biotechnology Progress 28.4 (2012) 1095-105), protein purification processes (Pezzini et al., J. Chromatogr. A 1218.45 (2011) 8197-208) and, cell culture conditions (Chen et al., Tissue Eng. Part C. Methods 17.12 (2011) 1211-21).

Based on the desired outcome different DoE approaches can be applied to the present process (Figure 3). *Fractional factorial* designs, often used for systems with a huge number of factors of influence, allow the identification of significant principal modulators in the process. On the other hand, experiment designs like *Full factorial, Box Behnken,* or *Central composite* not only identify critical modulators but also allow quantification of discrete optimal concentrations or settings thereof. For that, so called *Prediction profiler* tools postulate interactively experiment outcomes when concentrations or settings of critical modulators are varied *in silico* in a minimum (-1) and maximum (1) state. For calculating optimal concentrations predicted optimum level (a value between -1 and +1) has to be determined by multiplication with the set up concentration used.

All DoE approaches in this study were planned and analyzed using statistical software tool JMP (SAS Institute GmbH, Böblingen, Germany). The experiments were designed as fed-batch cultivations to simulate production process-like conditions. All DoE fed-batch experiments were performed using shaker or robotic cultivation systems and proprietary cultivation media and feed platforms A and B. The cultivation media used are chemically-defined serum- and protein-free media.

*Glyco Species Analysis*

For the analysis of mAb glycosylation harvested cell culture fluid was centrifuged for cell separation and filtered by 0.2 $\mu$M membrane filtration. mAb glycosylation species were analyzed as described by Reusch et al., (Anal. Biochem. 432.2 (2013) 82-89) and by an in-house capillary electrophoresis protocol.

## Example 1

*Inverse Regulation of Galactosylation by Manganese and Copper*

To test the general relevance of these trace elements on cell culture performance we used two in-house generated recombinant CHO-K1 cell lines, clone 1 and clone 3, expressing two different monoclonal antibodies, respectively. Using a fractional factorial DoE design (Table 5) the statistical significance of different trace elements (realized by supplementation of base medium with specific stock solutions) can be analysed. The fractional factorial DoE approach was applied to each clone and in replicates (four times for clone 1, three times for clone 3) using a fully controlled robotic cultivation system.

**Table 5 : Fractional factorial DoE screening of trace elements on cell culture performance.** Using 17 distinct fed-batch experiments we applied different symmetrical balanced levels of aluminum, molybdenum, barium, chromium, bromine, iodine, copper, manganese, rubidium, silver, zinc, tin, and zirconium stock solutions to the base medium (-1: low level, 0: medium level, +1: high level). The experiment design was applied for both clone 1 (n=4) and clone 3 (n=3). The experiment design was generated using JMP DoE tool.

| Experiment | Pattern | Al | Mo | Ba | Cr | Br | I | Cu | Mn | Rb | Ag | Zn | Sn | Zr |
|---|---|---|---|---|---|---|---|---|---|---|---|---|---|---|
| 1 | ----+--+-++-+ | -1 | -1 | -1 | -1 | 1 | -1 | -1 | 1 | -1 | 1 | 1 | -1 | 1 |
| 2 | ---+-++-+---+ | -1 | -1 | -1 | 1 | -1 | 1 | 1 | -1 | 1 | -1 | -1 | -1 | 1 |
| 3 | --+--++--+++- | -1 | -1 | 1 | -1 | -1 | 1 | 1 | -1 | -1 | 1 | 1 | 1 | -1 |
| 4 | --+++--++--+- | -1 | -1 | 1 | 1 | 1 | -1 | -1 | 1 | 1 | -1 | -1 | 1 | -1 |
| 5 | -+---+-++-++- | -1 | 1 | -1 | -1 | -1 | 1 | -1 | 1 | 1 | -1 | 1 | 1 | -1 |
| 6 | -+-++-+--+-+- | -1 | 1 | -1 | 1 | 1 | -1 | 1 | -1 | -1 | 1 | -1 | 1 | -1 |
| 7 | -++-+-+-+-+ | -1 | 1 | 1 | -1 | 1 | -1 | 1 | -1 | 1 | -1 | 1 | -1 | 1 |
| 8 | -+++-+-+-+--+ | -1 | 1 | 1 | 1 | -1 | 1 | -1 | 1 | -1 | 1 | -1 | -1 | 1 |
| 9 | +-----++++-++ | 1 | -1 | -1 | -1 | -1 | -1 | 1 | 1 | 1 | 1 | -1 | 1 | 1 |
| 10 | +--+++---+++ | 1 | -1 | -1 | 1 | 1 | 1 | -1 | -1 | -1 | -1 | 1 | 1 | 1 |
| 11 | +-+-++--++--- | 1 | -1 | 1 | -1 | 1 | 1 | -1 | -1 | 1 | 1 | -1 | -1 | -1 |
| 12 | +-++--++--+-- | 1 | -1 | 1 | 1 | -1 | -1 | 1 | 1 | -1 | -1 | 1 | 1- | -1 |
| 13 | ++--++++----- | 1 | 1 | -1 | -1 | 1 | 1 | 1 | 1 | -1 | -1 | -1 | 1- | -1 |
| 14 | ++-+---+++-- | 1 | 1 | -1 | 1 | -1 | -1 | -1 | -1 | 1 | 1 | 1 | -1 | -1 |
| 15 | +++-------++ | 1 | 1 | 1 | -1 | -1 | -1 | -1 | -1 | -1 | -1 | -1 | 1 | 1 |
| 16 | +++++++++++++ | 1 | 1 | 1 | 1 | 1 | 1 | 1 | 1 | 1 | 1 | 1 | 1 | 1 |
| 17 | 0000000000000 | 0 | 0 | 0 | 0 | 0 | 0 | 0 | 0 | 0 | 0 | 0 | 0 | 0 |

Next the theoretical effective concentrations of aluminum, molybdenum, barium, chromium, bromine, iodine, copper, manganese, rubidium, silver, zinc, tin, and zirconium ($c_{TE\ effective}$) at the start of the DoE fed-batch experiment were summarized for each condition (Table 6). Theoretical $c_{TE\ effective}$ for these trace elements at experiment start was calculated according equation 1:

(equation 1)

$$c_{TE\ effective} = \frac{((c_{TE\ Medium}*V_{Medium})+(c_{TE\ Stock}*V_{TE\ Stock}))}{V_{Experiment}} * [\mu M]$$

**Table 6 : Theoretical effective trace element concentrations $c_{TE\ effective}$ at the start of the fractional factorial DoE fed-batch experiment (Example 1).**

|  | -1 (low level) | 0 (medium level) | +1 (high level) |
|---|---|---|---|
| Component | [$\mu$M] | [$\mu$M] | [$\mu$M] |
| $Al^{3+}$ | 0.0023 | 0.0262 | 0.0502 |
| $MoO_4^{2-}$ | 0.0047 | 0.0095 | 0.0144 |
| $Ba^{2+}$ | 0.0049 | 0.0566 | 0.1083 |
| $Cr^{3+}$ | 0.0005 | 0.0056 | 0.0107 |
| $Br^-$ | 0.0007 | 0.0383 | 0.0759 |
| $I^-$ | 0.0005 | 0.0060 | 0.0114 |
| $Cu^{2+}$ | 0.3142 | 0.3471 | 0.3800 |
| $Mn^{2+}$ | 0.0832 | 0.0898 | 0.0963 |
| $Rb^+$ | 0.0024 | 0.0516 | 0.1009 |
| $Ag^+$ | 0.0006 | 0.0065 | 0.0125 |
| $Zn^{2+}$ | 13.9898 | 17.6545 | 21.3193 |
| $Sn^{2+}$ | 0.0005 | 0.0030 | 0.0054 |
| $ZrO^{2+}$ | 0.0047 | 0.0537 | 0.1027 |

The putative impact of aluminum, molybdenum, barium, chromium, bromine, iodine, copper, manganese, rubidium, silver, zinc, tin, and zirconium on galactosylation was analyzed by relative G1 and G2 glycan levels combining both clones (clone 1 and clone 3, both expressing different mAbs), and samples from day 7, 12 and 13 to identify general, clone and temporal independent effects.

Using the JMP DoE analysis tool a good linear model was found describing the impact of trace elements on mAb galactosylation. Here, a strong positive effect was observed for manganese on G1 and G2 levels (Figure 4A-D). Furthermore, zinc tends to have a positive effect on relative G1 and G2 abundance. On the other hand, copper has a statistically significant negative impact on G1 glycan formation (Figure 4A-B), indicating that the *in vitro* observed inhibitory effect (as detailed by Kaminska J et al., Glycoconj J. 15.8 (1998) 783-8) can be confirmed *in vivo* by cell culture experiments.

Interestingly, addition of zinc in combination with manganese revealed a synergistic effect on galactosylated glycan formation and the effect seems to be process time dependent. For quantification of the effect, we modelled the G1 and G2 formation alone with the effectors zinc and manganese in high and low concentrations (Figure 16). The highest amount of G1 and G2 species are predicted for high manganese in combination with high zinc levels.

Using the scaled estimates of the screening experiment (Figure 4B, D) the optimal concentration for galactosylation can be summarized as:

- Manganese: > **0.0963 $\mu$M,** since the linear model shows best results at highest concentration → optimum concentration not yet reached)
- Copper: < **0.3142 $\mu$M,** since the in a linear model show best result at lowest concentration → optimum concentration not yet reached).

We further analyzed the timing of the effect of the above significant elements on galactosylation by using clone 3 as model cell line. By switching from day 7 to day 13 using the prediction profiler tool, the impact of manganese in an early and late production phase was quantified. For both G1 and G2 the effect of manganese declined in late process phases (day 13). The decrease of slope in the prediction profiler curve indicates that bioavailability of manganese is not as good as in early process phases (day 7) (Figure 5).

**Example 2**

*Inverse Effects of Copper and Iron on Cell Growth and Glycosylation*

To identify single trace elements and combined effects responsible for cell growth and glycosylation modulation we analyzed the impact of zinc, copper, iron and manganese, known as highly bioactive trace elements, on cell growth and mAb glycosylation by a full factorial DoE experiment using the recombinant clone 2 (Table 7). Clone 2 was used as reporter cell line because of the endogenous elevated formation level of high mannose glycan structures.

**Table 7 : Full factorial DoE screening of zinc, iron, copper, and manganese on cell growth, galactosylated and non-fucosylated glycoprotein species.** Using 19 distinct fed-batch shaker experiments with clone 2 we applied different symmetrical balanced levels of zinc, iron, copper, and manganese to the base medium (-1: low level, 0: medium level, +1: high level). Three centre points ("0000" - shaker 5, 8, and 11) were used for identification of intrinsic experiment variance. The experiment design was generated using JMP DoE tool.

| Shaker | Pattern | Zinc | Iron | Copper | Manganese |
|--------|---------|------|------|--------|-----------|
| 1 | ++-+ | 1 | 1 | -1 | 1 |
| 2 | ++-- | 1 | 1 | -1 | -1 |
| 3 | +--- | 1 | -1 | -1 | -1 |
| 4 | --++ | -1 | -1 | 1 | 1 |
| 5 | 0000 | 0 | 0 | 0 | 0 |
| 6 | -+-+ | -1 | 1 | -1 | 1 |
| 7 | +--+ | 1 | -1 | -1 | 1 |
| 8 | 0000 | 0 | 0 | 0 | 0 |
| 9 | ++++ | 1 | 1 | 1 | 1 |
| 10 | -++- | -1 | 1 | 1 | -1 |
| 11 | 0000 | 0 | 0 | 0 | 0 |
| 12 | --+- | -1 | -1 | 1 | -1 |
| 13 | +-+- | 1 | -1 | 1 | -1 |
| 14 | ---- | -1 | -1 | -1 | -1 |
| 15 | +++- | 1 | 1 | 1 | -1 |
| 16 | +-++ | 1 | -1 | 1 | 1 |
| 17 | -+-- | -1 | 1 | -1 | -1 |
| 18 | -+++ | -1 | 1 | 1 | 1 |
| 19 | ---+ | -1 | -1 | -1 | 1 |

In this example the theoretical effective zinc, iron, copper, and manganese concentrations $c_{TE\ effective}$ at start of the DoE fed-batch experiment were summarized for each condition (Table 8). Theoretical $c_{TE\ effective}$ for zinc, iron, copper, and manganese at experiment start was calculated as described above.

**Table 8 : Theoretical effective zinc, iron, copper, and manganese concentrations $c_{TE\ effective}$ at start of the DoE fed-batch experiment (Example 2).**

| | -1 (low level) | 0 (medium level) | +1 (high level) |
|---|---|---|---|
| Component | [$\mu$M] | [$\mu$M] | [$\mu$M] |
| $Cu^{2+}$ | 0.312 | 0.330 | 0.362 |
| $Fe^{2+}/Fe^{3+}$ | 1.890 | 25.386 | 48.965 |
| $Mn^{2+}$ | 0.071 | 0.081 | 0.091 |
| $Zn^{2+}$ | 13.900 | 28.295 | 43.301 |

The highest effect on cell growth, analyzed by maximum viable cell density (Figure 6A-C) and final cell time integral (Figure 6D-F) was shown by supplementation of iron. In general, high concentrations of zinc, manganese and copper used in the experiment tend to result in higher cell growth and biomass generation (Figure 6C, F). On the other hand, the targeted combination of zinc, copper, iron and manganese modulated the amount of galactosylated or non-fucosylated species. High concentrations of manganese improves galactosylation, shown here for G1 (Figure 6G-I), whereas the combined effect of zinc and copper and the lack of manganese results in higher amounts of immature glycosylated mAb species (Figure 6J-L).

Copper and zinc show a strong interaction on immature glycan formation. Therefore, we further analyzed this interaction on non-fucosylated glycan species (glycoproteins with a high Mannose content) in detail using the JMP prediction profiler tool. In general, on moderate levels (level 0 in experiment), zinc (level 0 means 28.295 $\mu$M) displayed a negative and copper (level 0 means 0.330 $\mu$M) a positive correlation to the amount of high mannose glycans (Figure 7A). This is confirmed by the fact that the negative zinc level of -0.2 (means 25.416 $\mu$M) and a positive copper level of 0.6 (means 0.349 $\mu$M) no longer show an impact on the sum of mannosylated glycan species (Figure 7B). In the following, different scenarios of significant modulators zinc, copper and manganese on high mannose glycan species were simulated:

- Moderate and high levels (-0.2 to 1 means 25.416 $\mu$M to 43.301 $\mu$M) of zinc induce a positive effect of copper (Figure 7C, F, G, H, I)
- Low levels (-1 to -0.2 means 13.900 $\mu$M to 25.416 $\mu$M) of zinc induce a negative effect of copper (Figure 7D, E)
- A moderate level (-0.2 means 25.416 $\mu$M) of zinc annihilate the effect of copper (Figure 7B, J)
- The negative effect of manganese is independent of zinc and copper levels

We used the prediction profiler tool of JMP to determine the optimum concentrations of zinc, copper, iron and manganese for a combined optimization of cell growth and mAb glycan formation. Here, one valid model considering all trace element parameters was used (quadratic effect of iron).

According to the prediction profiler zinc, copper, and manganese in the highest concentrations and iron at levels > 0.42 (i.e. > 36.704 $\mu$M) will improve cell growth (Figure 8A, C). Using high concentrations of zinc, iron, and manganese and low concentration of copper will increase the amount of galactosylated mAb species (Figure 8D). On the other hand, low concentrations, level -1, of manganese (i.e. 0.071 $\mu$M), copper (i.e. 0.312 $\mu$M) and zinc (i.e.13.900 $\mu$M) and iron at levels of -0.3 (i.e. 11.288 $\mu$M) in parallel increase the amount of non-fucosylated mAb species (Figure 8F).

A minimum for VCD and CTI is realized when choosing a high level of zinc and copper and low levels for iron and manganese (Figure 8B). Low galactosylated glycan species are generated by choosing low concentrations of all of the four elements (Figure 8D). Minimum level of mannosylated glycans are generated for high zinc and manganese levels and low iron and copper levels (Figure 8G).

Again, we also tested the option for addition of zinc in combination with manganese alone to increase galactosylated glycan formation by JMP modelling of the data. As shown before, zinc and manganese have a synergistic effect on galactosylated glycan formation. For quantification of the effect, we modelled the G1 formation with effectors zinc and manganese at high and low concentrations (Figure 15). Addition of high levels of zinc with high levels of manganese can increase G1 species by 17% (Figures 15A, D).

For modulating degree of glycan species maturity, the optimal trace element levels are a combination of G1 and high mannose predictions (Figure 8D-G).

- For mature glycans: optimal levels for high G1 and low Man5
- For immature glycans: optimal levels for high Man5 and low G1.

## Example 3

*Targeted Production of Galactosylated and Non-fucosylated Glycan Species by Modulation of Copper, Iron, Zinc, and Manganese : Bioreactor Experiment 1*

CHO-K1 clone 2 was grown for 14 days in controlled 2L Quad fermentation systems (Sartorius, Gottingen, Germany) using the proprietary medium and bolus feed process platform B. Concentrated nutrient feed were supplemented at 10% of cultivation start volume on day 3, 6, and 9. Targeted trace element modulation was performed by supplementation of specific medium favouring mAb glycosylation on day 6.

The ratios and targeted concentrations of zinc, copper, iron and manganese identified in previous experiments are summarized in Table 9. These concentrations and ratios of zinc, copper, iron and manganese were tested in a bioreactor fed-batch verification experiment using clone 2 with endogenous high levels of Man5 glycan species. We modulated the trace element ratios using a culture splitting strategy and targeted supplementation to support cell growth in the first fed-batch phase (d0-d5) and enable mAb glycosylation maturation in the second fed-batch phase (d6-d14), by using two different cultivation media for "Biomass" and "Glycosylation" phases as described in Table 9A and Table 9B, respectively.

**Table 9A : "Biomass" medium used in bioreactor experiment for growth phase from d0-d5.**

| Component | Galactosylation [$\mu$M] | Growth [$\mu$M] | Non-fucosylation [$\mu$M] |
|---|---|---|---|
| $Cu^{2+}$ | 0.064 | 0.329 | 1.000 |
| $Fe^{2+}/Fe^{3+}$ | 47.158 | 47.158 | 50.000 |
| $Mn^{2+}$ | 0.029 | 0.029 | 0.004 |
| $Zn^{2+}$ | 41.072 | 41.072 | 11.061 |

**Table 9B : "Glycosylation" medium used in bioreactor experiment for glycosylation phase from d6-d14.**

| Component | Galactosylation [$\mu$M] | Growth [$\mu$M] | Non-fucosylation [$\mu$M] |
|---|---|---|---|
| $Cu^{2+}$ | 0.000 | 0.329 | 1.000 |
| $Fe^{2+}/Fe^{3+}$ | 0.000 | 0.000 | 50.000 |
| $Mn^{2+}$ | 0.029 | 0.029 | 0.004 |
| $Zn^{2+}$ | 41.072 | 41.072 | 11.061 |

Compared to a set up to favour immature glycan formation, called "*non-fucosylation*", with constant concentrations of zinc, copper, iron and manganese over the whole process we tested the predicted trace element concentrations for directed cell growth and mAb glycosylation maturation by just varying copper concentration between two levels, in the following called "*Growth*" and "*Galactosylation*" (Figure 9A). We decided to do so, since copper seems to have a stronger effect on glycosylation than on maximal viable cell density (Figure 8).

The following table (Table 10) shows the theoretical target concentrations used for bioreactor experiment 1.

**Table 10 : Target theoretical trace element concentrations modulating cell growth, mAb galactosylation, and non-fucosylation.** Concentrations of copper, manganese, iron, and zinc used in bioreactor experiment 1, see set up in Figure 9A.

| Component | low [$\mu$M] | medium [$\mu$M] | high [$\mu$M] |
|---|---|---|---|
| $Cu^{2+}$ | 0.064 | 0.329 | 1.000 |
| $Fe^{2+}/Fe^{3+}$ | 16.055 | n/a | 50.000 |
| $Mn^{2+}$ | 0.075 | n/a | 0.099 |
| $Zn^{2+}$ | 11.250 | n/a | 41.072 |

(n/a, not applicable)

Using this set up we proved that until day 6 cell growth capacity can be modulated by trace element balancing showing highest viable cell density and CTI for "Growth", followed by the "*non-fucosylation*" and *"Galactosylation"* processes (Figure 9B, C). The subsequent switch to conditions favouring glycosylation and to reduced cell growth from day 6 on was clearly shown for the *"Galactosylation"* process for viable cell density and cell time integral. Interestingly, the modulation of trace element ratios shows a strong effect on cell viability in late process phases (Figure 9D). Reducing copper tends to increase final viable cell density up to almost 30% viability (for the "*Galactosylation*" process) compared to the "*Growth*" process. The effect of increased cell viability by reduced copper concentration could also by confirmed by LDH release analysis (Figure 10C).

On the other hand, copper has been shown to positively influence lactate remetabolization in late process phases (Luo et al., Biotechnol. Bioeng. 109.1 (2012) 146-56). In the verification experiment described above we confirmed the relevance of copper for lactate remetabolization, since "*Galactosylation*" showed an increased but uncritical ($\rightarrow$ high cell viability) lactate concentration in late process phases compared to "*non-fucosylation*" and "*Growth*", where until day 14 almost all lactate is consumed (Figure 10B). As it is known that high levels of lactate can favour detoxification of ammonium (Li et al., Biotechnol. Bioeng. 109.5 (2012) 1173-86) we could confirm for the "*Galactosylation*" process the inverse correlation of lactate accumulation and ammonium re-assimilation (Figure 10A).

To study the effect of trace element modulation on mAb glycosylation kinetics we analyzed in process samples of bioreactor runs for non-fucosylated and galactosylated species. High mannose species Man5 (Figure 11A) as well as cumulative non-fucosylated species (Figure 11D) can be reduced (up to 30% for Man5) by the predicted trace element modulation in a copper concentration dependent manner (copper concentration: "*non-fucosylation*" > "*Growth*" > "*Galactosylation*"). Modulation of trace elements results in a shift from high mannose species to G0 (up to 30%) and subsequently to G1 species (up to 30% on day 13) once the process is switched from growth to glycosylation conditions (Figure 11A-C). The drop of G1 species in the *"Galactosylation"* set-up on day 14 may be caused by limitation of bioavailable $Mn^{2+}$ and $Zn^{2+}$ in late process phases.

## Example 4

*Targeted Modulation of Cell Growth and mAb Glycosylation by Iron, Copper, Zinc and Manganese : Bioreactor Experiment 2*

CHO-K1 clone 2 was grown in shake flasks for culture expansion and 14 days in controlled 2L Quad fermentation systems (Sartorius, Göttingen, Germany) for production fed-batch cultivation to evaluate the impact of iron, copper, zinc and manganese for cell growth and protein glycosylation. The proprietary medium and process platform B were used. Concentrated nutrient feed were supplemented continuously at 2.73% (v/v) per day and of initial culture start volume. Targeted trace element modulation was performed using specific metal concentrations in cultivation media and continuous feed (Table 11-13) and by splitting of the culture during inoculation train (phase n-2 and n-1) and transfer to production scale (phase n). Respective concentrations of iron, zinc, copper and manganese were calculated on the basis of specific cell and cultivation system consumption rates (Figure 13 and 14) and considering volume balancing during splitting and cultivation (e.g. supplementation of correction agents such as glucose stock, antifoam, base). Actual concentrations of iron, zinc, copper and manganese can be measured by ICP-MS (Agilent, Böblingen, Germany).

The test cases were designed either to support cell growth during cell culture expansion, initial biomass generation in fed-batch and protein galactosylation in production phase during fed-batch (day 6-14) or to support cell growth during cell culture expansion, initial biomass generation in fed-batch and protein non-fucosylation in production phase during fed-batch (day 6-14) or to interfere with cell growth during cell culture expansion, initial biomass generation in fed-batch and to support protein non-fucosylation in production phase during fed-batch (day 6-14) (Figure 12A).

**Table 11 : Target theoretical trace element concentrations at start of each cultivation phase for modulation of cell growth and mAb glycosylation - "Growth/Growth" (GG).** Concentrations of copper, manganese, iron, and zinc used in bioreactor verification experiment 2, see set up in Figure 12A.

| | n-2 Phase | n-1 Phase | n Phase |
|---|---|---|---|
| Component in Medium: | [μM] | [μM] | [μM] |
| $Cu^{2+}$ | 1.000 | 1.000 | 0.550 |
| $Fe^{2+}/Fe^{3+}$ | 50.000 | 50.000 | 40.000 |
| $Mn^{2+}$ | 1.000 | 1.000 | 1.000 |
| $Zn^{2+}$ | 43.000 | 43.000 | 43.000 |

(continued)

| Component in Feed: | | | |
|---|---|---|---|
| $Cu^{2+}$ | na | na | na |
| $Fe^{2+}/Fe^{3+}$ | na | na | na |
| $Mn^{2+}$ | na | na | na |
| $Zn^{2+}$ | na | 73.000 | na |

(na, not applicable)

**Table 12 : Target theoretical trace element concentrations at start of each cultivation phase for modulation of cell growth and mAb glycosylation - "Growth/non-fucosylation" (GA).** Concentrations of copper, manganese, iron, and zinc used in bioreactor verification experiment 2, see set up in Figure 12A.

| | n-2 Phase | n-1 Phase | n Phase |
|---|---|---|---|
| Component in medium: | [μM] | [μM] | [μM] |
| $Cu^{2+}$ | 1.000 | 1.000 | 1.000 |
| $Fe^{2+}/Fe^{3+}$ | 50.000 | 50.000 | 50.000 |
| $Mn^{2+}$ | 0.110 | 0.110 | 0.110 |
| $Zn^{2+}$ | 12.000 | 12.000 | 12.000 |
| Component in Feed: | | | |
| $Cu^{2+}$ | na | na | na |
| $Fe^{2+}/Fe^{3+}$ | na | na | na |
| $Mn^{2+}$ | na | na | na |
| $Zn^{2+}$ | na | na | na |

(na, not applicable)

**Table 13 : Target theoretical trace element concentrations at start of each cultivation phase for modulation of cell growth and mAb glycosylation - "Non-fucosylation/Non-fucyosylation" (AA).** Concentrations of copper, manganese, iron, and zinc used in bioreactor verification experiment 2, see set up in Figure 12A.

| | n-2 Phase | n-1 Phase | n Phase |
|---|---|---|---|
| Component in Medium: | [μM] | [μM] | [μM] |
| $Cu^{2+}$ | 0.330 | 0.330 | 0.330 |
| $Fe^{2+}/Fe^{3+}$ | 18.000 | 18.000 | 18.000 |
| $Mn^{2+}$ | 0.075 | 0.075 | 0.075 |
| $Zn^{2+}$ | 14.000 | 14.000 | 14.000 |
| Component in Feed: | | | |
| $Cu^{2+}$ | na | na | na |
| $Fe^{2+}/Fe^{3+}$ | na | na | na |
| $Mn^{2+}$ | na | na | na |
| $Zn^{2+}$ | na | na | na |

(na, not applicable)

As intended, test case "AA" clearly interferes with cell growth during inoculation train and production phase as shown by viable cell density and cell time integral in phase n (Figure 12 C-D). On the other hand, no difference in biomass generation was observed for test cases "GG" and "GA" during cell culture expansion and cell growth phase in fed-batch.

The effects on mAb maturation were analysed by glycan abundance measurement. For mature and immature mAb glycosylation species, G1 and G2 or Man6, Man5 and G0-GlcNAc were pooled. As intended, test case "GG" favours formation of mature glycan species (1.4-7 fold increase compared to "AA" and "GA") (Figure 12E) and test cases "GA" and "AA" support immature glycan formation (2-3 fold increase compared to "GG") (Figure 12F).

In summary, the procedure described allows the modulation of glycoproteins, and in particular mAb glycosylation maturation, by targeted trace element balancing using a biotechnological production process. By switching from "Biomass" to "Glycosylation" conditions using modulation strategies for zinc, copper, iron and manganese concentration and ratio the method fulfils the requirements for an economical high titre process.

**Claims**

1. A method for production of a recombinant glycoprotein under fermentation culture conditions in a eukaryotic cell, the method comprising adjusting the concentrations of each of iron, copper, zinc and manganese in the culture medium during the culture to affect biomass generation and/or N-glycan maturity in the expressed glycoprotein wherein the adjustment is:

   - increasing the concentration of each of iron, copper, zinc and manganese in order to increase biomass, wherein the concentrations are adjusted to:

     (a) iron - from 15 $\mu$M to more than 80 $\mu$M;
     (b) copper - from 0.3 $\mu$M to more than 2.5 $\mu$M;
     (c) zinc - from 20 $\mu$M to more than 50 $\mu$M; and
     (d) manganese - from 0.01 $\mu$M to more than 3 $\mu$M; and/or

   - increasing the concentration of each of zinc and manganese and, optionally, decreasing the concentration of each of iron and copper to increase N-glycan maturity in the expressed glycoprotein, wherein the carbohydrate portion of the expressed glycoprotein has a G0, G1 or G2 structure; or
   - (i) decreasing the concentration of each of iron, copper, zinc and manganese to increase production of immature non-fucosylated glycoproteins or (ii) increasing the concentration of each of copper and iron and decreasing the concentration of each of zinc and manganese to increase production of immature non-fucosylated glycoproteins;

   wherein a decrease in the concentration of any or all of iron, copper, zinc and manganese in the culture medium is achieved by complexing the iron, copper, zinc and manganese with a chelator and/or by seeding the cells into a fresh medium containing a reduced concentration of any or all of iron, copper, zinc and manganese compared to the medium of the immediately preceding culture phase.

2. The method of claim 1 wherein the expressed glycoprotein is a mature N-glycosylated glycoprotein, a mature non-fucosylated glycoprotein or an immature non-fucosylated glycoprotein.

3. The method according to claim 1 wherein to increase N-glycan maturity in the expressed glycoprotein the concentrations are adjusted to:

   (a) iron - from 0 $\mu$M to 25 $\mu$M;
   (b) copper - from 0 $\mu$M to 0.1 $\mu$M;
   (c) zinc - from 20 $\mu$M to more than 50 $\mu$M; and
   (d) manganese - from 0.01 $\mu$M to more than 3 $\mu$M.

4. The method according to claim 1 wherein to increase N-glycan maturity in the expressed glycoprotein the concentrations are adjusted to:

   (a) zinc - from 20 $\mu$M to more than 50 $\mu$M; and
   (b) manganese - from 0.01 $\mu$M to more than 3 $\mu$M.

5. The method according to claim 1 wherein to increase production of immature non-fucosylated glycoproteins the concentrations are adjusted to either:

(i)

    (a) iron - from 0 μM to 35 μM;
    (b) copper - from 0 μM to 1 μM;
    (c) zinc - from 0 μM to 20 μM; and
    (d) manganese - from 0 μM to 0.01 μM; or

(ii)

    (a) iron - from 15 μM to more than 80 μM;
    (b) copper - from 0.3 μM to more than 2.5 μM;
    (c) zinc - from 0 μM to 20 μM; and
    (d) manganese - from 0 μM to 0.01 μM.

6. The method according to any one of claims 1 to 5 wherein the glycoprotein is exogenous or endogenous to the eukaryotic cell, optionally wherein the glycoprotein is a structural glycoprotein, hormone, antibody or enzyme.

7. The method according to claim 6 wherein the glycoprotein is an antibody, optionally wherein the antibody is a therapeutic or diagnostic antibody, optionally a chimeric, humanized or human antibody.

8. The method according to any one of claims 1 to 7 wherein the eukaryotic cell is a mammalian cell, a yeast cell or an insect cell.

9. The method according to any one of claims 1 to 7 wherein the concentrations of iron, copper, zinc and manganese are adjusted during the growth and/or production culture phases.

10. The method according to any one of claims 1 to 9 wherein an increase in the concentration of any or all of iron, copper, zinc and manganese in the culture medium is achieved by supplementing the medium in which the cells are cultured and/or by splitting the cells into a fresh medium supplemented with any or all of iron, copper, zinc and manganese.

11. The method according to any of claims 1 to 10 in which the concentrations of each of iron, copper, zinc and manganese in the culture medium are adjusted during the culture first to favour biomass generation and then to increase N-glycan maturity in expressed N-glycoproteins, or to increase production of immature non-fucosylated glycoproteins.

**Patentansprüche**

1. Verfahren zur Herstellung eines rekombinanten Glykoproteins unter Fermentationskulturbedingungen in einer eukaryotischen Zelle, wobei das Verfahren das Anpassen der Konzentrationen von Eisen, Kupfer, Zink und Mangan in dem Kulturmedium während des Kultivierens umfasst, um die Erzeugung von Biomasse und/oder N-Glykanreife in dem exprimierten Glykoprotein zu beeinflussen, wobei die Anpassung Folgendes ist:

    - Erhöhen der Konzentrationen von Eisen, Kupfer, Zink und Mangan, um die Biomasse zu erhöhen, wobei die Konzentrationen auf Folgendes angepasst werden:

        (a) Eisen - von 15 μM bis mehr als 80 μM;
        (b) Kupfer - von 0,3 μM bis mehr als 2,5 μM;
        (c) Zink - von 20 μM bis mehr als 50 μM; und
        (d) Mangan - von 0,01 μM bis mehr als 3 μM; und/oder

    - Erhöhen der Konzentrationen von Zink und Mangan und gegebenenfalls Senken der Konzentrationen von Eisen und Kupfer, um die N-Glykanreife in dem exprimierten Glykoprotein zu erhöhen, wobei der Kohlenhydratanteil des exprimierten Glykoproteins eine G0-, G1- oder G2-Struktur aufweist; oder
    - (i) Senken der Konzentrationen von Eisen, Kupfer, Zink und Mangan, um die Produktion von unreifen nicht fucosylierten Glykoproteinen zu steigern, oder (ii) Erhöhen der Konzentrationen von Kupfer und Eisen und Senken der Konzentrationen von Zink und Mangan, um die Produktion von unreifen nicht fucosylierten Glykoproteinen zu steigern;

wobei ein Senken der Konzentrationen von Eisen, Kupfer, Zink und/oder Mangan oder allen davon in dem Kulturmedium erzielt wird, indem das Eisen, Kupfer, Zink und Mangan mit einem Chelatbildner komplexiert wird und/oder die Zellen in ein frisches Medium beimpft werden, das eine verringerte Konzentration von Eisen, Kupfer, Zink und/oder Mangan oder allen davon im Vergleich zum Medium der unmittelbar vorangegangenen Kulturphase enthält.

2. Verfahren nach Anspruch 1, wobei das exprimierte Glykoprotein ein reifes N-glykosyliertes Glykoprotein, ein reifes nicht fucosyliertes Glykoprotein oder ein unreifes nicht fucosyliertes Glykoprotein ist.

3. Verfahren nach Anspruch 1, wobei die Konzentrationen auf Folgendes angepasst werden, um die N-Glykanreife in dem exprimierten Glykoprotein zu erhöhen:

   (a) Eisen - von 0 $\mu$M bis 25 $\mu$M;
   (b) Kupfer-von 0 $\mu$M bis 0,1 $\mu$M;
   (c) Zink - von 20 $\mu$M bis mehr als 50 $\mu$M; und
   (d) Mangan - von 0,01 $\mu$M bis mehr als 3 $\mu$M.

4. Verfahren nach Anspruch 1, wobei die Konzentrationen auf Folgendes angepasst werden, um die N-Glykanreife in dem exprimierten Glykoprotein zu erhöhen:

   (a) Zink - von 20 $\mu$M bis mehr als 50 $\mu$M; und
   (b) Mangan - von 0,01 $\mu$M bis mehr als 3 $\mu$M.

5. Verfahren nach Anspruch 1, wobei zur Steigerung der Produktion von unreifen nicht fucosylierten Glykoproteinen die Konzentrationen angepasst werden, und zwar auf entweder:

   (i)

   (a) Eisen - von 0 $\mu$M bis 35 $\mu$M;
   (b) Kupfer - von 0 $\mu$M bis 1 $\mu$M;
   (c) Zink - von 0 $\mu$M bis 20 $\mu$M; und
   (d) Mangan - von 0 $\mu$M bis 0,01 $\mu$M; oder

   (ii)

   (a) Eisen - von 15 $\mu$M bis mehr als 80 $\mu$M;
   (b) Kupfer - von 0,3 $\mu$M bis mehr als 2,5 $\mu$M;
   (c) Zink - von 0 $\mu$M bis 20 $\mu$M; und
   (d) Mangan - von 0 $\mu$M bis 0,01 $\mu$M.

6. Verfahren nach einem der Ansprüche 1 bis 5, wobei das Glykoprotein zu der eukaryotischen Zelle exogen oder endogen ist, wobei das Glykoprotein gegebenenfalls ein strukturelles Glykoprotein, ein Hormon, ein Antikörper oder ein Enzym ist.

7. Verfahren nach Anspruch 6, wobei das Glykoprotein ein Antikörper ist, wobei der Antikörper gegebenenfalls ein therapeutischer oder diagnostischer Antikörper, gegebenenfalls ein chimärer, humanisierter oder humaner Antikörper ist.

8. Verfahren nach einem der Ansprüche 1 bis 7, wobei die eukaryotische Zelle eine Säugetierzelle, eine Hefezelle oder eine Insektenzelle ist.

9. Verfahren nach einem der Ansprüche 1 bis 7, wobei die Konzentrationen von Eisen, Kupfer, Zink und Mangan während der Wachstums- und/oder Produktionskulturphase angepasst werden.

10. Verfahren nach einem der Ansprüche 1 bis 9, wobei eine Erhöhung der Konzentration von Eisen, Kupfer, Zink und/oder Mangan oder allen davon in dem Kulturmedium erzielt wird, indem das Medium, in dem die Zellen kultiviert werden, mit Eisen, Kupfer, Zink und/oder Mangan oder allen davon ergänzt wird und/oder indem die Zellen in ein frisches Medium geteilt werden, das mit Eisen, Kupfer, Zink und/oder Mangan oder allen davon ergänzt ist.

**11.** Verfahren nach einem der Ansprüche 1 bis 10, wobei die Konzentrationen von Eisen, Kupfer, Zink und Mangan im Kulturmedium jeweils während der Kultivierung angepasst werden, zunächst, um die Erzeugung von Biomasse zu begünstigen, und dann, um die N-Glykanreife in exprimierten N-Glykoproteinen zu erhöhen oder die Produktion von unreifen nicht fucosylierten Glykoproteinen zu steigern.

## Revendications

**1.** Procédé de production d'une glycoprotéine recombinante dans des conditions de culture de fermentation dans une cellule eucaryote, le procédé comprenant l'ajustement des concentrations de chacun parmi le fer, le cuivre, le zinc et le manganèse dans le milieu de culture pendant la culture pour affecter la production de la biomasse et/ou la maturité des N-glycanes dans la glycoprotéine exprimée dans lequel l'ajustement est :

- l'augmentation de la concentration de chacun parmi le fer, le cuivre, le zinc et le manganèse afin d'augmenter la biomasse, les concentrations étant ajustées à :

(a) fer - de 15 $\mu$M à plus de 80 $\mu$M ;
(b) cuivre - de 0,3 $\mu$M à plus de 2,5 $\mu$M ;
(c) zinc - de 20 $\mu$M à plus de 50 $\mu$M ; et
(d) manganèse - de 0,01 $\mu$M à plus de 3 $\mu$M ; et/ou

- l'augmentation de la concentration de chacun parmi le zinc et le manganèse, et éventuellement, la diminution de la concentration de chacun parmi le fer et le cuivre pour augmenter la maturité des N-glycanes dans la glycoprotéine exprimée, la partie glucidique de la glycoprotéine exprimée ayant une structure G0, G1 ou G2 ; ou
- (i) la diminution de la concentration de chacun parmi le fer, le cuivre, le zinc et le manganèse pour augmenter la production des glycoprotéines non fucosylées immatures ou (ii) l'augmentation de la concentration de chacun parmi le cuivre et le fer et la diminution de la concentration de chacun parmi le zinc et le manganèse pour augmenter la production des glycoprotéines non fucosylées immatures ;

dans lequel une diminution de la concentration de l'un quelconque ou de tous parmi le fer, le cuivre, le zinc et le manganèse dans le milieu de culture est obtenue par complexation du fer, du cuivre, du zinc et du manganèse avec un chélateur et/ou par ensemencement des cellules dans un milieu frais contenant une concentration réduite de l'un quelconque ou de tous parmi le fer, le cuivre, le zinc et le manganèse par comparaison au milieu de la phase de culture juste avant.

**2.** Procédé selon la revendication 1 dans lequel la glycoprotéine exprimée est une glycoprotéine N-glycosylée mature, une glycoprotéine non fucosylée mature ou une glycoprotéine non fucosylée immature.

**3.** Procédé selon la revendication 1 dans lequel pour augmenter la maturité des N-glycanes dans la glycoprotéine exprimée, les concentrations sont ajustées à :

(a) fer - de 0 $\mu$M à 25 $\mu$M ;
(b) cuivre - de 0 $\mu$M à 0,1 $\mu$M ;
(c) zinc - de 20 $\mu$M à plus de 50 $\mu$M ; et
(d) manganèse - de 0,01 $\mu$M à plus de 3 $\mu$M.

**4.** Procédé selon la revendication 1 dans lequel pour augmenter la maturité des N-glycanes dans la glycoprotéine exprimée, les concentrations sont ajustées à :

(a) zinc - de 20 $\mu$M à plus de 50 $\mu$M ; et
(b) manganèse - de 0,01 $\mu$M à plus de 3 $\mu$M.

**5.** Procédé selon la revendication 1 dans lequel pour augmenter la production de glycoprotéines non fucosylées immatures, les concentrations sont ajustées à :

(i)

(a) fer - de 0 $\mu$M à 35 $\mu$M ;

(b) cuivre - de 0 μM à 1 μM ;
(c) zinc - de 0 μM à 20 μM ; et
(d) manganèse - de 0 μM à 0,01 μM ; ou

(ii)

(a) fer - de 15 μM à plus de 80 μM ;
(b) cuivre - de 0,3 μM à plus de 2,5 μM ;
(c) zinc - de 0 μM à 20 μM ; et
(d) manganèse - de 0 μM à 0,01 μM.

6. Procédé selon l'une quelconque des revendications 1 à 5 dans lequel la glycoprotéine est exogène ou endogène vis-à-vis de la cellule eucaryote, éventuellement dans lequel la glycoprotéine est une glycoprotéine structurelle, une hormone, un anticorps ou une enzyme.

7. Procédé selon la revendication 6 dans lequel la glycoprotéine est un anticorps, éventuellement dans lequel l'anticorps est un anticorps thérapeutique ou diagnostique, éventuellement un anticorps chimérique, humanisé ou humain.

8. Procédé selon l'une quelconque des revendications 1 à 7 dans lequel la cellule eucaryote est une cellule de mammifère, une cellule de levure ou une cellule d'insecte.

9. Procédé selon l'une quelconque des revendications 1 à 7 dans lequel les concentrations en fer, cuivre, zinc et manganèse sont ajustées pendant les phases de culture de croissance et/ou de production.

10. Procédé selon l'une quelconque des revendications 1 à 9 dans lequel une augmentation de la concentration de l'un quelconque ou de tous parmi le fer, le cuivre, le zinc et le manganèse dans le milieu de culture est obtenue par ajout au milieu dans lequel les cellules sont cultivées et/ou par division des cellules dans un milieu frais supplémenté avec l'un quelconque ou de tous parmi le fer, le cuivre, le zinc et le manganèse.

11. Procédé selon l'une quelconque des revendications 1 à 10 dans lequel les concentrations de chacun parmi le fer, le cuivre, le zinc et le manganèse dans le milieu de culture sont ajustées pendant la culture d'abord pour favoriser la production de la biomasse et ensuite pour augmenter la maturité des N-glycanes dans les N-glycoprotéines exprimées, ou pour augmenter la production des glycoprotéines non fucosylées immatures.

FIG. 1

FIG. 2

Factor z

Level +1

Level 0

Level -1

Factor y

Factor x

Full factorial

Fractional factorial

Central composite

Box Behnken

D-optimal design

Taguchi design

FIG. 3

EP 3 110 961 B1

A

G1 Predicted P<.0001
Rsq=0,90 RMSE=1,3155

FIG. 4

## B

Scaled Estimates

Nominal factors expanded to all levels
Continuous factors centered by mean, scaled by range/2

| Term | Scaled Estimate | | Std Error | t Ratio | Prob>\|t\| |
|---|---|---|---|---|---|
| Intercept | 7,5468579 | | 0,192689 | 39,17 | <,0001* |
| Plate[1] | -0,645483 | | 0,144044 | -4,48 | <,0001* |
| Plate[2] | -0,594215 | | 0,145802 | -4,08 | <,0001* |
| Plate[3] | -0,522473 | | 0,148566 | -3,52 | 0,0005* |
| Plate[4] | -0,291317 | | 0,144044 | -2,02 | 0,0437* |
| Plate[5] | 1,0248815 | | 0,145743 | 7,03 | <,0001* |
| Plate[6] | 0,6463246 | | 0,146707 | 4,41 | <,0001* |
| Plate[7] | 0,3822824 | | 0,150661 | 2,54 | 0,0115* |
| Clone[1] | 0 | | 0 | 0,00 | 1,0000 |
| Clone[3] | 0 | | 0 | 0,00 | 1,0000 |
| Day | -2,955056 | | 0,159919 | -18,48 | <,0001* |
| Aluminium | -0,089947 | | 0,067986 | -1,32 | 0,1865 |
| Molybdenum | 0,1175024 | | 0,067359 | 1,74 | 0,0817 |
| Barium | 0,085942 | | 0,067347 | 1,28 | 0,2026 |
| Chromium | 0,0518704 | | 0,067606 | 0,77 | 0,4433 |
| Bromine | -0,013796 | | 0,66447 | -0,21 | 0,8356 |
| Iodine | 0,1141258 | | 0,06765 | 1,69 | 0,0923 |
| Copper | -0,187321 | | 0,067638 | -2,77 | 0,0058* |
| Manganese | 1,3656831 | | 0,067168 | 20,33 | <,0001* |
| Rubidium | 0,1045994 | | 0,068002 | 1,54 | 0,1247 |
| Silver | -0,061647 | | 0,066229 | -0,93 | 0,3524 |
| Zinc | 0,0951202 | | 0,066402 | 1,43 | 0,1527 |
| Tin | 0,0541508 | | 0,067361 | 0,80 | 0,4219 |
| Zirconium | -0,060032 | | 0,067283 | -0,89 | 0,3727 |
| (Day-10,7361)*(Day-10,7361) | 1,9508852 | | 0,242891 | 8,03 | <,0001* |

## FIG. 4 (continued)

C

FIG. 4 (continued)

D

| Scaled Estimates |
|---|

Nominal factors expanded to all levels
Continuous factors centered by mean, scaled by range/2

| Term | Scaled Estimate | | Std Error | t Ratio | Prob>\|t\| |
|---|---|---|---|---|---|
| Intercept | 0,4188584 | | 0,036673 | 11,42 | <,0001* |
| Plate[1] | -0,005623 | | 0,027415 | -0,21 | 0,8376 |
| Plate[2] | -0,052605 | | 0,02775 | -1,90 | 0,0586 |
| Plate[3] | -0,058561 | | 0,028276 | -2,07 | 0,0389* |
| Plate[4] | 0,0096544 | | 0,027415 | 0,35 | 0,7249 |
| Plate[5] | 0,0565108 | | 0,027738 | 2,04 | 0,0422* |
| Plate[6] | 0,0533356 | | 0,027922 | 1,91 | 0,0567 |
| Plate[7] | -0,002712 | | 0,028674 | -0,09 | 0,9247 |
| Clone[1] | 0 | | 0 | 0,00 | 1,0000 |
| Clone[3] | 0 | | 0 | 0,00 | 1,0000 |
| Day | -0,157669 | | 0,030436 | -5,18 | <,0001* |
| Aluminium | -0,01299 | | 0,012939 | -1,00 | 0,3159 |
| Molybdenum | 0,0007346 | | 0,01282 | 0,06 | 0,9543 |
| Barium | 0,0071756 | | 0,012818 | 0,56 | 0,5759 |
| Chromium | 0,0014852 | | 0,012867 | 0,12 | 0,9082 |
| Bromine | 0,0036169 | | 0,012646 | 0,29 | 0,7750 |
| Iodine | 0,0087184 | | 0,012875 | 0,68 | 0,4987 |
| Copper | -0,011456 | | 0,012873 | -0,89 | 0,3740 |
| Manganese | 0,1315323 | | 0,012784 | 10,29 | <,0001* |
| Rubidium | 0,0067806 | | 0,012942 | 0,52 | 0,6006 |
| Silver | -0,000594 | | 0,012605 | -0,05 | 0,9624 |
| Zinc | 0,0136294 | | 0,012638 | 1,08 | 0,2814 |
| Tin | -0,015355 | | 0,01282 | -1,20 | 0,2317 |
| Zirconium | -0,016528 | | 0,012806 | -1,29 | 0,1975 |
| (Day-10,7361)*(Day-10,7361) | 0,2807794 | | 0,046228 | 6,07 | <,0001* |

FIG. 4 (continued)

FIG. 5

A

VCD max Actual

VCD max Predicted P<.0001
Rsq=0,94 RMSE=3,8826

D

CTI final Actual

CTI final Predicted P<.0001
Rsq=0,97 RMSE=470,99

FIG. 6

B

| Term | Estimate | Std Error | t Ratio | | Prob>\|t\| |
|------|----------|-----------|---------|---|---------|
| Iron | 11,12 | 0,970661 | 11,46 | | <,0001* |
| Iron*Iron | -12,07417 | 2,442777 | -4,94 | | 0,0004* |
| Manganese | 3,05625 | 0,970661 | 3,15 | | 0,0093* |
| Zinc*Iron | 2,395 | 0,970661 | 2,47 | | 0,0313* |
| Iron*Copper | 1,49625 | 0,970661 | 1,54 | | 0,1515 |
| Zinc | 1,4175 | 0,970661 | 1,46 | | 0,1722 |
| Copper | 1,41375 | 0,970661 | 1,46 | | 0,1732 |

C

FIG. 6 (continued)

E

| Term | Estimate | Std Error | t Ratio | | Prob>\|t\| |
|------|----------|-----------|---------|---|---------|
| Iron | 1928,1563 | 117,7482 | 16,38 | | <,0001* |
| Iron*Iron | -2378,123 | 296,3265 | -8,03 | | <,0001* |
| Zinc | 425,515 | 117,7482 | 3,61 | | 0,0036* |
| Zinc*Iron | 367,86625 | 117,7482 | 3,12 | | 0,0088* |
| Manganese | 271,09875 | 117,7482 | 2,30 | | 0,0400* |
| Zinc*Manganese | 174,75875 | 117,7482 | 1,48 | | 0,1635 |

FIG. 6 (continued)

F

G

G1 Predicted P=0,0047
Rsq=0,75 RMSE=1,1958

FIG. 6 (continued)

EP 3 110 961 B1

**H**

| Term | Estimate | Std Error | t Ratio | | Prob>\|t\| |
|---|---|---|---|---|---|
| Zinc*Copper | -1,05 | 0,29895 | -3,51 | | 0,0043* |
| Manganese | 1,0375 | 0,29895 | 3,47 | | 0,0046* |
| Iron | 0,875 | 0,29895 | 2,93 | | 0,0127* |
| Zinc | 0,35 | 0,29895 | 1,17 | | 0,2644 |
| Iron*Manganese | -0,225 | 0,29895 | -0,75 | | 0,4662 |
| Copper | -0,1375 | 0,29895 | -0,46 | | 0,6538 |

FIG. 6 (continued)

I

K

| Term | Estimate | Std Error | t Ratio | | Prob>|t| |
|---|---|---|---|---|---|
| Manganese | -1,80625 | 0,486821 | -3,71 | | 0,0023* |
| Zinc*Copper | 1,59375 | 0,486821 | 3,27 | | 0,0055* |
| Zinc | -0,95625 | 0,486821 | -1,96 | | 0,0697 |
| Copper | 0,31875 | 0,486821 | 0,65 | | 0,5232 |

FIG. 6 (continued)

J

FIG. 6 (continued)

L

FIG. 6 (continued)

A

B

FIG. 7

FIG. 7 (continued)

FIG. 7 (continued)

G

H

FIG. 7 (continued)

FIG. 7 (continued)

FIG. 8

B

FIG. 8 (continued)

C

VCD max
155,9797
±8,863871

CTI final
3244,82
±1059,942

G1
8,21887
±2,725984

Σ Mannose w/o Fuc
25,60204
±3,855443

Zinc
Iron
0,432
Copper
Manganese

FIG. 8 (continued)

FIG. 8 (continued)

EP 3 110 961 B1

FIG. 8 (continued)

FIG. 8 (continued)

FIG. 8 (continued)

A

| Process Phase | Medium | | | | | | | | | | | |
|---|---|---|---|---|---|---|---|---|---|---|---|---|
| | $Cu^{2+}$ | $Fe^{2+/3+}$ | $Zn^{2+}$ | $Mn^{2+}$ | $Cu^{2+}$ | $Fe^{2+/3+}$ | $Zn^{2+}$ | $Mn^{2+}$ | $Cu^{2+}$ | $Fe^{2+/3+}$ | $Zn^{2+}$ | $Mn^{2+}$ |
| Fed-Batch (d0-d5) → Biomass | high | high | low | low | medium | high | high | high | low | high | high | high |
| Fed-Batch (d6-d14) → Glycosylation | high | high | low | low | medium | low | high | high | low | low | high | high |

—▲— Afucosylation          –☒– Growth          ··◎·· Galactosylation

—▲— Afucosylation
–☒– Growth
··◎·· Galactosylation

—▲— Afucosylation
–☒– Growth
··◎·· Galactosylation

—▲— Afucosylation
–☒– Growth
··◎·· Galactosylation

B

C

D

FIG. 9

EP 3 110 961 B1

FIG. 10

FIG. 11

C

D

FIG. 11 (continued)

EP 3 110 961 B1

A

| Process Time | d-8 | d-4 | d0 | d6 | d14 |
|---|---|---|---|---|---|
| Phases | n-2 | n-1 | n | | |

Test Cases

| | | | | |
|---|---|---|---|---|
| 1 | Growth | Growth | Growth | Galactosylation |
| 2 | Growth | Growth | Growth | Afucosylation |
| 3 | Afuc | Afuc | Afuc | Afucosylation |

B

| Process Phase | Medium | | | | | | | | | | | |
|---|---|---|---|---|---|---|---|---|---|---|---|---|
| | $Cu^{2+}$ | $Fe^{3+}$ | $Zn^{2+}$ | $Mn^{2+}$ | $Cu^{2+}$ | $Fe^{3+}$ | $Zn^{2+}$ | $Mn^{2+}$ | $Cu^{2+}$ | $Fe^{3+}$ | $Zn^{2+}$ | $Mn^{2+}$ |
| Inoculation Train (phase n-2/n-1) → Biomass | high | high | high | high | high | high | low | low | low | low | low | low |
| Fed-Batch (phase n) → Glycosylation | low | low | high | high | high | high | low | low | low | low | low | low |

◇ Medium GG (Growth/ Galactosylation)

□ Medium GA (Growth/ Afucosylation)

△ Medium AA (Afucosylation/ Afucosylation)

FIG. 12

FIG. 12 (continued)

FIG. 12 (continued)

A

B

FIG. 13

C

—△— Afucosylation
- ◫ - Growth
···◉··· Galactosylation

D

FIG. 13 (continued)

FIG. 14

FIG. 14 (continued)

FIG. 15

FIG. 16

FIG. 16 (continued)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20070161084 A
- US 20080081356 A
- WO 2012149197 A
- WO 9808934 A
- US 20090068705 A
- EP 307247 A
- WO 9725428 A

### Non-patent literature cited in the description

- **YANNONE et al.** *Current Opinion in Biotechnology,* 2013, vol. 23.1, 89-95
- **FRAGA ; FRAGA.** *Mol. Aspects Med.,* 2005, vol. 26.4-5, 235-44
- **HAM et al.** *Proc. Natl. Acad. Sci. USA,* 1965, vol. 53, 288-93
- **KAMINSKA et al.** *Glyconj. J.,* 1998, vol. 15.8, 783-88
- **WITSELL et al.** *J. Biol. Chem.,* 1990, vol. 265.26, 15731-37
- **CROWELL et al.** *Biotechnol. Bioeng.,* 2007, vol. 96.3, 538-49
- **GAWLITZEK et al.** *Biotechnol. Bioeng.,* 2009, vol. 103.6, 1164-75
- **AU et al.** *Neurotoxicology,* 2008, vol. 29.4, 569-76
- **ROTH et al.** *Neurotoxicology,* 2002, vol. 23.2, 147-57
- **RAMAKRISHNAN et al.** *J. Mol. Biol.,* 2006, vol. 357.5, 1619-33
- **BEYERSMANN et al.** *Biometals,* 2001, vol. 14.3-4, 331-41
- **BASSET et al.** *Carcinogenesis,* 1985, vol. 6.3, 355-59
- **REDDEL et al.** *Exp. Cell Res.,* 1985, vol. 161.2, 277-84
- **POLSON et al.** *Immunology,* 1990, vol. 71.2, 176-81
- **PATTANAPANYASAT et al.** *Br. J. Haematol.,* 1992, vol. 82.1, 13-19
- **LEDERMAN et al.** *Blood,* 1984, vol. 64.3, 748-53
- **ARREDONDO et al.** *Molecular Aspects of Medicine,* 2005, vol. 26.4, 313-27
- **TURSKI et al.** *J. Biol. Chem.,* 2009, vol. 284.4, 717-21
- **HARRIS et al.** *Nutr. Rev.,* 2004, vol. 62.2, 60-64
- **GUPTE et al.** *Cancer Treat. Rev.,* 2009, vol. 35.1, 32-46
- **RAJU.** *Curr. Opin. Immunol.,* 2008, vol. 20.4, 471-8
- **KUMPEL et al.** *Hum. Antib. Hyb.,* 1994, vol. 5.3-4, 143-51
- **KARSTEN et al.** *Nat. Med.,* 2012, vol. 18.9, 1401-6
- **GRAHAM et al.** *J. Gen. Virol.,* 1977, vol. 36, 59
- **MATHER.** *Biol. Reprod.,* 1980, vol. 23, 243
- **BAUMANN et al.** *J. Cell Biol.,* 1980, vol. 85, 1
- **MATHER et al.** *Annals N.Y. Acad. Sci.,* 1982, vol. 383, 44
- **URLAUB ; CHASIN.** *P. N. A. S.,* 1980, vol. 77, 4216
- **SIMONSEN ; LEVINSON.** *P. N. A. S.,* 1983, vol. 80, 2495-2499
- **DEROUAZI et al.** *Biochem. Biophys. Res. Commun.,* 2006, vol. 340, 1069-77
- **MATHER.** Mammalian Cell Culture. Plenum Press, 1984
- **BARNES ; SATO.** *Cell,* 1980, vol. 22, 649
- **M. BUTLER.** Mammalian Cell Biotechnology: A Practical Approach. IRL Press, 1991
- **ZECK et al.** *PLoS. One,* 2012, vol. 7.7, e40328
- **ZHANG et al.** *Cytotechnology,* 2013, vol. 65.3, 363-78
- **FU et al.** *Biotechnology Progress,* 2012, vol. 28.4, 1095-105
- **PEZZINI et al.** *J. Chromatogr. A,* 2011, vol. 1218.45, 8197-208
- **CHEN et al.** *Tissue Eng. Part C. Methods,* 2011, vol. 17.12, 1211-21
- **REUSCH et al.** *Anal. Biochem.,* 2013, vol. 432.2, 82-89
- **KAMINSKA J et al.** *Glycoconj J.,* 1998, vol. 15.8, 783-8
- **LUO et al.** *Biotechnol. Bioeng.,* 2012, vol. 109.1, 146-56
- **LI et al.** *Biotechnol. Bioeng.,* 2012, vol. 109.5, 1173-86